# EUROPEAN PATENT SPECIFICATION

(11) **EP 4 139 294 B1**
(45) Date of publication and mention of the grant of the patent: **09.04.2025**
(21) Application number: 21723350.1
(22) Date of filing: 22.04.2021
(51) Int. Cl.: C07D 401/14, A61K 31/4725, A61P 1/16, A61P 3/10, A61P 9/00, A61P 11/00, A61P 11/06, A61P 13/12, A61P 17/00, A61P 25/00, A61P 35/00

(54) **ISOQUINILINE NRF2 AGONISTS**
ISOCHINOLIN NRF2 AGONISTEN
ISOQUINOLINES COMME AGONISTES DU NRF2

(30) Priority: 22.04.2020 GB 202005863
(43) Date of publication of application: 01.03.2023
(73) Proprietor: C4x Discovery Limited, Manchester M1 3LD (GB)
(72) Inventor: LUCAS, Cathy Louise, Manchester One 53 Portland Street Manchester M1 3LD (GB); RAY, Nicholas Charles, London NW1 1AD (GB); SEWARD, Eileen Mary, Harlow Essex CM19 5TR (GB); HYND, George, Harlow Essex CM19 5TR (GB)
(74) Representative: HGF
(86) International application number: PCT/GB2021/050973
(87) International publication number: WO 2021/214470

(56) References cited:
- WO-A1-2013/067036
- WO-A1-2016/203400
- WO-A1-2018/181345
- WO-A1-2020/084300
- HONGBIN DAI ET AL: "Development of Novel Nrf2/ARE Inducers Bearing Pyrazino[2,1-a]isoquinolin Scaffold with Potent In Vitro Efficacy and Enhanced Physicochemical Properties", MOLECULES, vol. 22, no. 9, 1 September 2017 (2017-09-01), DE, pages 1541, XP055649795, ISSN: 1433-1373, DOI: 10.3390/molecules22091541
- BENJAMIN G. RICHARDSON ET AL: "Replacement of a Naphthalene Scaffold in Kelch-like ECH-Associated Protein 1 (KEAP1)/Nuclear Factor (Erythroid-derived 2)-like 2 (NRF2) Inhibitors", JOURNAL OF MEDICINAL CHEMISTRY, vol. 61, no. 17, 20 August 2018 (2018-08-20), US, pages 8029 - 8047, XP055642376, ISSN: 0022-2623, DOI: 10.1021/acs.jmedchem.8b01133
- DATABASE WPI Week 201871, Derwent World Patents Index; AN 2018-776846

## Description

The present invention relates to tetrahydroisoquinoline compounds. More specifically, the present invention relates to tetrahydroisoquinoline compounds that are Nrf2 activators. The present invention also relates to pharmaceutical compositions comprising these compounds, and to their use in the treatment of diseases or disorders associated with Nrf2 activation and/or inhibition of Keap1-Nrf2 protein-protein interactions.

### BACKGROUND OF THE INVENTION

Nuclear factor erythroid 2-related factor 2 (Nrf2) is a basic leucine zipper (bZIP) transcription factor and a member of the Cap 'n' Collar (CNC) family of transcription factors. It is a key master of the inducible cell defence system, mediating the expression of more than 100 oxidative stress-related genes that include phase I and II detoxification enzymes and antioxidant proteins. These genes all contain the antioxidant response element (ARE) in their promoter regulatory regions, which is the binding target of Nrf2. Under basal conditions the levels of Nrf2 are tightly regulated by the adaptor protein Keap1, a cytosolic actin-bound repressor protein, which binds to Nrf2 and leads to proteasomal degradation *via* the Cul3-based E3 ubiquitin ligase complex. Under conditions of oxidative stress, Keap1 is inactivated leading to an increase in the level of *de novo* synthesised Nrf2 which translocates to the nucleus, binds to AREs with a resulting up-regulation in cytoprotective gene expression.

It has been shown that Nrf2 mRNA expression in COPD subjects was significantly lower than that in control subjects and Nrf2 mRNA were negatively correlated with pack year. Nrf2 protein in COPD subjects was significantly lower than that in control subjects. CSE-induced A549 cell apoptosis was increased in a time-dependent and concentration-dependent manner, and was significantly increased by Nrf2 knockdown (Yamada, BMC *Pulmonary Medicine,* doi:10.1186/s12890-016-0189-1). Therefore, elevation of Nrf2 levels in the lungs of COPD patients should lead to a reduction in the inflammatory processes that lead to deleterious structural modifications of the lung and slow disease progression. Nrf2 may also be expected to show positive benefits in other respiratory diseases that exhibit oxidative stress components (Cho, Toxicol Appl Pharmacol, doi: 10.1016/j.taap.2009.07.024) such as acute, chronic and severe asthma (Sussan, Am J Physiol Lung Cell Mol Physiol, doi:10.1152/ajplung.00398.2014), acute lung injury/acute respiratory distress syndrome, with or without accompanying multi organ dysfunction syndrome (Yan, Free Radical Biol Med, doi:10.1016/j.freeradbiomed.2018.04.557; de la Vega *Curr Pharmacol Rep,* doi:10.1007/s40495-016-0053-2), pulmonary fibrosis, including idiopathic pulmonary fibrosis (Kikuchi, *Respir Res,* doi:10.1186/1465-9921-11-31) and cystic fibrosis (Chen, *PLoS One,* doi:10.1371/journal.pone.0003367). Indeed, the combination of cytoprotection by restoring redox and protein homeostasis, promoting resolution of inflammation, and facilitating repair in the lungs by Nrf2 activators has highlighted their potential for the treatment of COVID-19 (Cuadrado et al, Trends Pharmacol Sci, doi:10.1016/j.tips.2020.07.003).

The cardiac protective nature of Nrf2 in models of atherosclerosis, ischaemia, reperfusion, cardiac hypertrophy and heart failure has been demonstrated (Chen, Physiol Genomics, doi:10.1152/physiolgenomics.00041.2017). The Nrf2 activator Bardoxolone methyl has recently completed a Phase II study in patients with pulmonary arterial hypertension (PAH), with a Phase III study underway based on significant improvements in 6 minute walking distance. Bardoxolone reacts covalently with Keap1 but compounds activating Nrf2 *via* alternative mechanisms of Keap1 binding should also be expected to be therapeutically useful in PAH, particularly in patients that also have an underlying connective tissue disorder (CTD), such as scleroderma or lupus erythematosus. Oxidative stress is elevated in the diseased myocardium, leading to raised levels of reactive oxygen species which impact negatively on cardiac function (Bolli, Circ, doi: 10.1161/circ.76.2.3111744). Nrf2 activation has been shown to suppress myocardial oxidative stress, cardiac apoptosis, hypertrophy, fibrosis and dysfunction in mouse models of pressure overload (Wang, J Card Failure, doi:10.1016/j.cardfail.2012.06.003) and to protect against cardiac ischemic/reperfusion injury in rodent models (Zhang, J Mol Cell Cardiol, doi:10.1016/j.yjmcc.2010.05.01). Furthermore, excessive production of oxidizing agents in detriment of antioxidant defences in the cardiovascular system has also been described in metabolic diseases such as obesity, metabolic syndrome and diabetes mellitus where activation of Nrf2 has also been suggested as a promising therapeutic strategy (da Costa, Front Pharmacol., doi:10.3389/fphar.2019.00382). In addition, the Nrf2 activator sulforaphane reduces hepatic glucose production and improves glucose control in patients with type 2 diabetes (Axelsson, *Sci Transl Med.,* doi: 10.1126/scitranslmed.aah4477) and bardoxolone methyl has been shown to induce weight loss in generally obese patients in proportion to baseline BMI alongside improving glycaemic control (Chertow et al, J Diabetes Complications, doi: 10.1016/j.jdiacomp.2018.09.005). Age-associated mitochondrial dysfunction and oxidative damage are primary causes for multiple health problems including sarcopenia and cardiovascular disease and treatment of aging mice with the Nrf2 activator Sulforaphane restored Nrf2 activity, mitochondrial function, cardiac function, exercise capacity, glucose tolerance, and activation/differentiation of skeletal muscle satellite cells (Bose et al, Aging cell, doi:10.1111/acel.13261). Thus, it is expected that drugs leading to activation of Nrf2 should be useful in a number of cardiovascular and metabolic diseases including, but not limited to, atherosclerosis, hypertension, heart failure, myocardial infarction and repair, cardiac remodelling, cardiac arrhythmias, heart failure with reduced ejection fraction, diabetic cardiomyopathy, diabetic nephropathy, metabolic syndrome, obesity, diabetes mellitus (type 1 or type 2) and insulin resistance.

Subarachnoid haemorrhage (SAH) is a devastating condition with high morbidity and mortality rates due to the lack of effective therapy. Early brain injury (EBI) and cerebral vasospasm (CVS) are the two most important pathophysiological mechanisms for brain injury and poor outcomes for patients with SAH (clinicaltrials.gov/ct2/show/NCT0261474, *SFX01 After Subarachnoid Haemorrhage (SAS)).* Evidence from experimental SAH research indicates a protective role of the Nrf2/ARE pathway in EBI and CVS after SAH. Administration of sulforaphane (SFN) to rats following SAH enhances the activity of the Nrf2-ARE pathway, attenuates vasospasm in basilar arteries and suppresses the release of proinflammatory cytokines (Zhao, Brain Res., doi:10.1016/j.brainres.2016.09.035). Intracerebral haemorrhage (ICH) is the primary event in 10-15% of the 15 million strokes occurring annually worldwide. *In vitro* studies demonstrated that Nrf2 activators rapidly increased HO-1 expression in astrocytes and reduced their vulnerability to haemoglobin or hemin. Systemic treatment with small molecule Nrf2 activators increased HO-1 expression in perivascular cells, particularly astrocytes. When tested in mouse or rat ICH models, Nrf2 activators were consistently protective, improving barrier function and attenuating edema, inflammation, neuronal loss and neurological deficits (Chen-Roetling, Curr Pharm Des, doi:10.2174/1381612822666161027150616). Ischemic stroke induces reactive oxygen species, causing oxidative and inflammatory responses in ischemic brain. To date, recombinant tissue plasminogen activator is the only available therapy for the treatment of ischemic stroke. However, the treatment does not prevent oxidative stress and inflammation in the ischemic brain. D3T, a sulfur-containing dithiolethione compound, is found in cruciferous vegetables and has been reported to induce anti-oxidant genes through activation of Nrf2. D3T has been shown (Yen, J Immunol 2017, 198 (1 supplement) 206.20) to attenuate brain infarct and ameliorate neurological deficits in stroke animals. In addition, D3T reduced CNS infiltrating inflammatory immune cells including neutrophils and monocytes in the ischemic brain. Moreover, D3T-induced suppression of inflammatory cytokine production was observed in wild-type but not in Nrf2-deficient microglia. Furthermore, the protective effect of D3T on the attenuation of ischemic brain infarct was abolished in Nrf2-deficient stroke animals and in the stroke animals administered with HO-1 inhibitor. These results suggest that D3T-mediated suppression of inflammation in the ischemic brain is mediated through Nrf2/HO-1 pathway, and thus that targeting the Nrf2/HO-1 pathway may be a promising therapeutic strategy for the amelioration of neuroinflammation in ischemic stroke.

Nrf2 is believed to play a key role in some hemoglobinopathies, such as beta-thalassemia and sickle cell disease (SCD). SCD is a recessive inherited disorder caused by a single missense mutation which leads to the mutated beta-globin protein haemoglobin S (HbS). At low oxygen concentrations HbS polymerises, leading to misshapen red blood cells which are prone to rupture, releasing free heme into plasma. The resulting oxidative stress and inflammation leads to damage in multiple organs of the body. Ablation of Keap1 and the resulting constitutive activation of Nrf2 has been shown to lead to improved outcomes in SCD model mice (Zhu, Blood, doi:10.1182/blood-2017-10-810531; Keleku-Lukwete PNAS, doi:10.1073/pnas.1509158112). Nrf2 activation has been shown to slow down the progression of haemolytic anemia and organ disfunction (Ghosh, JCI Insight, doi: 10.1172/jci.insight.81090) and loss of Nrf2 function worsens the pathophysiology of SCD in transgenic SCD mice (Zhu, Blood, doi.org/10.1182/blood-2017-10-810531). Global activation of Nrf2 with the known compound D3T reduces lethality in a haem-induced acute chest syndrome model in transgenic SCD mice (Ghosh, Brit. J. Haematology doi: 10.1111/bjh.15401). In addition, Nrf2 activators have also been shown to modulate foetal haemoglobin (HbF) expression through direct binding in the gamma-globin promoter and modification of chromatin structure in the beta-globin locus. In sickle erythroid cells, Nrf2 provides unique benefits through HbF induction to inhibit haemoglobin S polymerization and protection against oxidative stress due to chronic haemolysis (Zhu et al, Exp Biol Med doi: 10.1177/1535370219825859). Thus, the development of small molecule activators of Nrf2 has the potential to ameliorate the clinical severity of sickle cell disease and other diseases where increasing HbF is beneficial such as beta-thalassemia.

The function of Nrf2 is altered in many neurodegenerative disorders, such as Huntington's disease, Parkinson's Disease, Alzheimer's disease, amyotrophic lateral sclerosis, frontotemporal dementia, multiple sclerosis and Friedreich's ataxia (Dinkova-Kostova, *FEBS,* doi:10.1111/febs.14379). Nrf2 activation mitigates multiple pathogenic processes involved in these neurodegenerative disorders through upregulation of antioxidant defences, inhibition of inflammation, improvement of mitochondrial function, and maintenance of protein homeostasis. Small molecule pharmacological activators of Nrf2 have shown protective effects in numerous animal models of neurodegenerative diseases (Joshi, Neurobiol Aging, doi:10.1016/j.neurobiolaging.2014.09.004; Alarcon-Aguilar, Neurobiol Aging, doi:10.1016/j.neurobiolaging.2014.01.143 and in cultures of human cells expressing mutant proteins. Tecfidera (dimethyl fumarate) activates Nrf2 (in addition to other mechanisms) and is approved in the US to treat relapsing-remitting multiple sclerosis. The Nrf2 activator Omaveloxolone (RTA-408) currently in Phase II trials for the treatment of the inherited neurodegenerative disorder Friedrich's ataxia, has met its primary endpoint of change in the modified Friedrich's Ataxia Rating Scale (mFARS) relative to placebo after 48 weeks of treatment. Targeting Nrf2 signalling may therefore provide a therapeutic option to delay onset, slow progression, and ameliorate symptoms of neurodegenerative disorders. Due to the role of oxidative stress and mitochondrial dysfunction in disorders of the CNS, treatment of chronic pain and schizophrenia with Nrf2 activators has also been suggested.

Rheumatoid arthritis (RA) is an autoimmune disease that causes chronic inflammation of the joints and is characterized by periods of disease flares and remissions. Multiple joints can be affected sometimes resulting in permanent joint destruction and deformity. Nrf2 has been found to be activated in the joints of arthritic mice and of RA patients. Nrf2-knockout mice have more severe cartilage injuries and more oxidative damage, with the expression of Nrf2 target genes being enhanced in Nrf2-wild-type but not in knockout mice during antibody-induced arthritis (Wruck, BMJ Annals of Rheumatic Diseases, doi:10.1136/ard.2010.132720). Additionally, in an animal model of rheumatoid arthritis, using the transfer of serum from K/BxN transgenic mice to Nrf2(-/-) mice, Nrf2 deficiency accelerated the incidence of arthritis, and animals showed a widespread disease affecting both front and hind paws (Maicas, Antioxidants & Redox Signaling, doi:10.1089/ars.2010.3835).

Ulcerative colitis (UC) and Crohn's disease (CD) are chronic relapsing-remitting forms of inflammatory bowel disease (IBD) that are caused by dysfunction of the intestinal epithelium. Damage to the intestinal epithelial cells can disrupt the barrier function of the intestinal epithelium, facilitating an aberrant immune response and inflammatory conditions. Thus, the intact intestinal epithelium is critical for the healthy gut, and cytoprotective agents that could target the intestinal epithelial cells would be beneficial for the treatment of UC and CD. Antioxidant levels and oxidative stress biomarkers are typically correlated with disease severity in IBD and a number of genome-wide association studies have linked IBD-associated single nucleotide polymorphisms (SNPs) to multiple genes involved in the response to oxidative stress, with many regulated by Nrf2 (Khor et al, Nature, doi:10.1038/nature10209). CPUY192018, a small-molecule inhibitor of the Keap1-Nrf2 protein-protein interaction (and hence Nrf2 activator) has demonstrated a cytoprotective effect in an experimental model of UC induced by dextran sodium sulphate in both NCM460 cells and mouse colon (Lu, Scientific Reports, doi:10.1038/srep26585). It has also been shown that Nrf2 knockout mice show an increased susceptibility to colitis-associated colorectal cancer (Khor, Cancer Prev Res (Phila), doi:10.1158/1940-6207).

Fumaderm, a mixture of dimethyl fumarate (DMF) and three salts of monoethyl fumarate, was licensed in Germany in 1994 for the treatment of psoriasis. The likely bioactive form of DMF, monomethyl fumarate (MMF) has been shown to increase total and nuclear Nrf2 levels in primary mouse keratinocytes and lead to enhanced mRNA expression of several Nrf2-downstream effectors such as heme oxygenase-1 and peroxiredoxin-6. (Helwa, J Pharmacol. Exp. Ther., doi:10.1124/jpet.116.239715). Other skin disorders may benefit from treatment with Nrf2 activators such as radiation-induced dermatitis/skin damage, atopic dermatitis and wound healing (Wu et al, Mol Med Rep. 2019 Aug;20(2):1761-1771).

Activation of Nrf2 has been shown to have beneficial effects in diseases of both the liver and kidney. NAFLD (Non-alcoholic fatty liver disease) is recognized as the leading cause of chronic liver disease worldwide. NAFLD represents a spectrum of diseases, some of which can progress to cirrhosis and hepatocellular carcinoma (HCC). Although all subtypes of NAFLD increase the risk for cardiovascular events and mortality, NASH (non-alcoholic steatohepatitis) is the main diagnostic subtype of NAFLD which predisposes patients to cirrhosis and liver-related complications. There are currently approved drug treatments for NAFLD and NASH. However, knockout of Nrf2 in mice profoundly predisposes to NASH stimulated by either a methionine- and choline-deficient (MCD) diet (Chowdry, Free Radic Biol Med, doi:10.1016/j.freeradbiomed.2009.11.007) or a high fat (HF) diet (Okada, J Gastroenterol, doi:10.1007/s00535-012-0659-z), and pharmacologic activation of Nrf2 has been shown to reverse NASH in mouse models (Sharma, Cell Mol Gastroenterol Hepatol, doi:10.1016/j.jcmgh.2017.11.016). Other liver diseases may benefit from treatment with Nrf2 activators such as toxin-induced liver disease, viral hepatitis and cirrhosis. Oxidative-stress molecules, such as reactive oxygen species, accumulate in the kidneys of animal models for acute kidney injury (AKI), in which Nrf2 is transiently and slightly activated. Genetic or pharmacological enhancement of Nrf2 activity in the renal tubules significantly ameliorates damage related to AKI and prevents AKI progression to chronic kidney disease (CKD) by reducing oxidative stress. However, a Phase III clinical trial of a KEAP1 inhibitor, CDDO-Me or bardoxolone-methyl, for patients with stage 4 CKD and type-2 diabetes mellitus (T2DM) was terminated due to the occurrence of cardiovascular events. Because recent basic studies have accumulated positive effects of KEAP1 inhibitors in moderate stages of CKD, Phase II trials have been restarted. The data from the ongoing projects demonstrate that a Nrf2 activator/KEAP1 inhibitor improves the glomerular filtration rate in patients with stage 3 CKD and T2DM without safety concerns (Nezu, Am J Nephrol, doi:10.1159/000475890). Inflammatory reactions and oxidative stress are implicated in the pathogenesis of focal segmental glomerulosclerosis (FSGS), a common chronic kidney disease with relatively poor prognosis and unsatisfactory treatment regimens. CXA-10 which upregulates Nrf2 pathways is currently in clinical trials for Focal Segmental Glomerulosclerosis (FIRSTx - A Study of Oral CXA-10 in Primary Focal Segmental Glomerulosclerosis (FSGS); clinicaltrials.gov/ct2/show/NCT03422510). Bardoxolone methyl has been shown in a Phil trial to lead to significant improvement in kidney function in patients with either autosomal dominant polycystic kidney disease (ADPKD), CKD associated with type 1 diabetes (T1D), IgA nephropathy (IgAN) or FSGS after 12 weeks of treatment (https://www.reatapharma.com/press-releases/reata-announces-positive-phase-2-data-for-bardoxolone-methyl-in-patients-with-focal-segmental-glomerulosclerosis-and-in-patients-from-all-four-cohorts-of-phoenix/). Alport Syndrome is the second most common inherited cause of kidney failure caused by a genetic defect in type IV collagen, a component in building the glomerular basement membrane. As bardoxolone methyl is thought to affect the underlying pathologic processes associated with mitochondrial dysfunction, inflammation and oxidative stress, it is currently being studied in these patients suggesting Nrf2 activators will be potentially useful in this disease.

Oxidative stress plays a critical role in the initiation and progression of cancer (Gorrini, Nat Rev Drug Discov., doi:10.1038/nrd4002). Due to its importance in the maintenance of redox cellular homeostasis, Nrf2 is considered a cytoprotective transcription factor and tumour suppressor. At lower homeostatic levels Nrf2 is able to eliminate ROS, carcinogens and other DNA-damaging agents, leading to the inhibition of tumour initiation and metastasis (Milkovic et al. Redox Biol. doi:10.1016/j.redox.2017.04.013). Evgen is currently evaluating SFX-01 (sulforaphane-cyclodextrin complex) in the Treatment and Evaluation of Metastatic Breast Cancer (STEM) (clinicaltrials.gov/ct2/show/NCT02970682) which includes ER+/HER- metastatic breast cancer. Bardoxolone derivatives have been shown to prevent lung cancer induced by vinyl carbamate in A/J mice (Liby, Cancer Res. doi:10.1158/0008-5472). Thus, activators of Nrf2 may have a role in the prevention of cancer.

Age related macular degeneration (AMD) is the principal cause of blindness in western countries and oxidative stress plays a major role in AMD pathogenesis and progression. It has been shown that Nrf2 activators are able to protect cells cultured to mimic the external layer of the retina from oxidative stress suggesting the potential for vision preservation in early AMD patients (Bellezza, Front Pharmacol. 2018; 9: 1280). In addition, Nrf2 activators may also be useful in other eye conditions such as Fuchs Endothelial Corneal Dystrophy and uveitis.

Recently, Nrf-2 activators have also been suggested to have benefit for the treatment of preeclampsia via suppression of oxidative stress and endothelial cell apoptosis (Jiang et al, Oxid Med Cell Longev doi:10.1155/2021/8839394).

Therefore, there is an ongoing need for agents capable of Nrf2 activation, given the role of Nrf2 in multiple indications.

WO 2013/067036 discloses bicyclic small molecule inhibitors of Keap1-Nrf2 interaction. WO 2018/181345 describes benzotriazole-linked bicyclic compounds having Nrf2-activating activity. WO 2020/084300 discloses tetrahydroisoquinoline compounds that are Nrf2 activators. Dai et al, Molecules (2017), 22, 1541 describe the development of Nrf2 activators featuring a pyrido[1,2-a]pyrazine scaffold. Richardson et al, J. Med. Chem. (2018), 61, 8029-8047 prepared compounds based on various heterocyclic scaffolds, in particular a 1,4-disubstituted isoquinoline scaffold, as inhibitors of Keap1-Nrf2 interaction.

### SUMMARY OF THE INVENTION

In one aspect, the present invention provides a compound, or a pharmaceutically acceptable salt thereof as defined in the appended claims.

In another aspect, the present invention provides a pharmaceutical composition comprising a compound of the invention as defined in the appended claims, or a pharmaceutically acceptable salt thereof, and one or more pharmaceutically acceptable excipients.

In another aspect, the present invention relates to a compound of the invention as defined in the appended claims, or a pharmaceutically acceptable salt thereof, or a pharmaceutical composition as defined herein, for use in therapy.

In another aspect, the present invention relates to a compound of the invention as defined in the appended claims, or a pharmaceutically acceptable salt thereof, or a pharmaceutical composition as defined herein, for use in the treatment of diseases or disorders mediated by Nrf2 activation.

In another aspect, the present invention relates to the use of a compound of the invention as defined in the appended claims, or a pharmaceutically acceptable salt thereof, in the manufacture of a medicament for use in the treatment of diseases or disorders mediated by Nrf2 activation.

Examples of diseases or disorders mediated by Nrf2 activation include chronic obstructive pulmonary disease, asthma, pulmonary arterial hypertension, diabetes mellitus, chronic kidney disease, ulcerative colitis, Crohn's disease, inflammatory bowel disease, Friedreich's ataxia, sickle cell disease and non-alcoholic steatohepatitis.

In another aspect, the present invention provides a compound, or a pharmaceutically acceptable salt thereof, or a pharmaceutical composition as defined herein, for use in the treatment of chronic obstructive pulmonary disease, asthma, pulmonary arterial hypertension, diabetes mellitus, chronic kidney disease, ulcerative colitis, Crohn's disease, inflammatory bowel disease, Friedreich's ataxia, sickle cell disease or non-alcoholic steatohepatitis.

In another aspect, the present invention provides the use of a compound, or a pharmaceutically acceptable salt, in the manufacture of a medicament for use in the treatment of chronic obstructive pulmonary disease, asthma, pulmonary arterial hypertension, diabetes mellitus, chronic kidney disease, ulcerative colitis, Crohn's disease, inflammatory bowel disease, Friedreich's ataxia, sickle cell disease or non-alcoholic steatohepatitis.

The present disclosure (not part of the invention) further provides a method of synthesising a compound, or a pharmaceutically acceptable salt thereof, as defined herein.

In another aspect, the present disclosure (not part of the invention) provides a compound, or a pharmaceutically acceptable salt thereof, obtainable by, or obtained by, or directly obtained by a method of synthesis as defined herein.

In another aspect, the present disclosure (not part of the invention) provides novel intermediates as defined herein which are suitable for use in any one of the synthetic methods set out herein.

Preferred, suitable, and optional features of any one particular aspect of the present invention are also preferred, suitable, and optional features of any other aspect.

### DETAILED DESCRIPTION OF THE INVENTION

### Definitions

Unless otherwise stated, the following terms used in the specification and claims have the following meanings set out below.

It is to be appreciated that references to "treating" or "treatment" include prophylaxis as well as the alleviation of established symptoms of a condition. "Treating" or "treatment" of a state, disorder or condition therefore includes: (1) preventing or delaying the appearance of clinical symptoms of the state, disorder or condition developing in a human that may be afflicted with or predisposed to the state, disorder or condition but does not yet experience or display clinical or subclinical symptoms of the state, disorder or condition, (2) inhibiting the state, disorder or condition, *i.e.,* arresting, reducing or delaying the development of the disease or a relapse thereof (in case of maintenance treatment) or at least one clinical or subclinical symptom thereof, or (3) relieving or attenuating the disease, *i.e.,* causing regression of the state, disorder or condition or at least one of its clinical or subclinical symptoms.

A "therapeutically effective amount" means the amount of a compound that, when administered to a mammal for treating a disease, is sufficient to effect such treatment for the disease. The "therapeutically effective amount" will vary depending on the compound, the disease and its severity and the age, weight, etc., of the mammal to be treated.

In this specification the term "alkyl" includes both straight and branched chain alkyl groups. References to individual alkyl groups such as "propyl" are specific for the straight chain version only and references to individual branched chain alkyl groups such as "isopropyl" are specific for the branched chain version only. For example, "(1-6C)alkyl" includes (1-4C)alkyl, (1-3C)alkyl, propyl, isopropyl and *t*-butyl. A similar convention applies to other radicals, for example "phenyl(1-6C)alkyl" includes phenyl(1-4C)alkyl, benzyl, 1-phenylethyl and 2-phenylethyl.

In this specification the term "alkylene" includes both straight and branched chain divalent alkyl groups. For example, "C₁₋₄alkylene" includes methylene (-CH₂-), ethylene (-CH₂CH₂-), propylene and butylene.

In this specification the term "alkoxy" includes both straight and branched chain alkyl groups singularly bonded to oxygen. For example, "C₁₋₄alkoxy" includes methoxy, ethoxy, isopropoxy and t-butoxy.

The term "(m-nC)" or "(m-nC) group" used alone or as a prefix, refers to any group having m to n carbon atoms.

"Cycloalkyl" means a hydrocarbon monocyclic or bicyclic ring containing carbon atoms. Examples of monocyclic cycloalkyl groups include cyclopropyl, cyclobutyl, cyclopentyl, cyclohexyl, cycloheptyl. Bicyclic rings may be fused or spiro attached; examples of bicyclic cycloalkyl groups include bicyclo[2.2.2]octane, bicyclo[2.1.1]hexane, bicyclo[1.1.1]pentane, spiro[2.4]heptane, bicyclo[4.1.0]heptane and bicyclo[2.2.1]heptane.

The term "halo" refers to fluoro, chloro, bromo and iodo.

The term "haloalkyl" is used herein to refer to an alkyl group respectively in which one or more hydrogen atoms have been replaced by halogen (e.g. fluorine) atoms. Examples of haloalkyl groups include fluoroalkyl groups such as -CHF₂, -CH₂CF₃, or perfluoroalkyl/alkoxy groups such as -CF₃, or -CF₂CF₃.

The term "heterocyclyl" means a non-aromatic saturated or partially saturated monocyclic, fused, bridged, or spiro bicyclic heterocyclic ring system(s). Monocyclic heterocyclic rings contain from about 3 to 12 (suitably from 3 to 7) ring atoms, with from 1 to 5 (suitably 1, 2 or 3) heteroatoms selected from nitrogen, oxygen or sulfur in the ring. Bicyclic heterocycles contain from 7 to 17 member atoms, suitably 7 to 12 member atoms, in the ring. Bicyclic heterocyclic(s) rings may be fused, spiro, or bridged ring systems. Examples of heterocyclic groups include cyclic ethers such as oxiranyl, oxetanyl, tetrahydrofuranyl, dioxanyl, and substituted cyclic ethers. Heterocycles containing nitrogen include, for example, azetidinyl, pyrrolidinyl, piperidinyl, piperazinyl, tetrahydrotriazinyl, tetrahydropyrazolyl, and the like. Typical sulfur containing heterocycles include tetrahydrothienyl, dihydro-1,3-dithiol, tetrahydro-2*H*-thiopyran, and hexahydrothiepine. Other heterocycles include dihydro-oxathiolyl, dihydroisoxazolyl (such as 4,5-dihydroisoxazolyl), dihydropyridinyl (such as 1,2-dihydropyridinyl or 1,6-dihydropyridinyl), tetrahydro-oxazolyl, tetrahydro-oxadiazolyl, tetrahydro-dioxazolyl, tetrahydro-oxathiazolyl, hexahydrotriazinyl, tetrahydro-oxazinyl, morpholinyl, thiomorpholinyl, tetrahydropyrimidinyl, dioxolinyl, octahydrobenzofuranyl, octahydrobenzimidazolyl, and octahydrobenzothiazolyl. For heterocycles containing sulfur, the oxidized sulfur heterocycles containing SO or SO₂ groups are also included. Examples include the sulfoxide and sulfone forms of tetrahydrothienyl and thiomorpholinyl such as tetrahydrothiene 1,1-dioxide and thiomorpholinyl 1,1-dioxide. A suitable value for a heterocyclyl group which bears 1 or 2 oxo (=O) or thioxo (=S) substituents is, for example, 2-oxopyrrolidinyl, 2-thioxopyrrolidinyl, 2-oxoimidazolidinyl, 2-thioxoimidazolidinyl, 2-oxopiperidinyl, 2,5-dioxopyrrolidinyl, 2,5-dioxoimidazolidinyl or 2,6-dioxopiperidinyl. Particular heterocyclyl groups are saturated monocyclic 3 to 7 membered heterocyclyls containing 1, 2 or 3 heteroatoms selected from nitrogen, oxygen or sulfur, for example azetidinyl, tetrahydrofuranyl, tetrahydropyranyl, pyrrolidinyl, morpholinyl, tetrahydrothienyl, tetrahydrothienyl 1,1-dioxide, thiomorpholinyl, thiomorpholinyl 1,1-dioxide, piperidinyl, homopiperidinyl, piperazinyl or homopiperazinyl. As the skilled person would appreciate, any heterocycle may be linked to another group *via* any suitable atom, such as *via* a carbon or nitrogen atom. Suitably, the term "heterocyclyl" will refer to 4, 5, 6 or 7 membered monocyclic rings as defined above.

The specification also makes use of composite terms to describe groups comprising more than one functionality. Such terms will be understood by a person skilled in the art. For example, C₁₋₄alkylene-C₃₋₇cycloalkyl refers to C₁₋₄alkylene substituted by a C₃₋₇cycloalkyl group.

The term "optionally substituted" refers to either groups, structures, or molecules that are substituted and those that are not substituted.

Where optional substituents are chosen from "one or more" groups it is to be understood that this definition includes all substituents being chosen from one of the specified groups or the substituents being chosen from two or more of the specified groups.

The phrase "compound of the invention" means those compounds which are claimed herein.

### Compounds of the Invention

The present disclosure (not part of the invention) provides a compound of Formula (I) 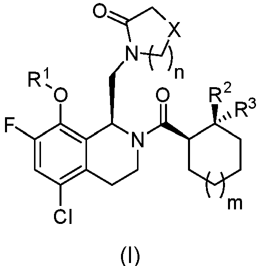 wherein:
R¹ is C₁₋₄alkylene-R⁴;
R² is selected from CO₂H and tetrazolyl;
R³ is selected from hydrogen and methyl;
X is CR⁵R⁶ or O;
R⁴ is 1,2,3-triazolyl, 1,2,4-triazolyl or pyrimidinyl, wherein said triazolyl or pyrimidinyl group is:
   optionally substituted with one or more substituents independently selected from C₁₋₄alkyl, C₁₋₃haloalkyl, C₃₋₇cycloalkyl, C₁₋₄alkylene-C₃₋₇cycloalkyl, halo, OH, C₁₋₃alkoxy, and cyano; and
   optionally fused to a cycloalkyl or heterocyclyl ring;
R⁵ is selected from hydrogen, C₁₋₄alkyl, C₃₋₇cycloalkyl and C₁₋₃haloalkyl;
R⁶ is selected from hydrogen and C₁₋₄alkyl; or
R⁵ and R⁶, together with the carbon atom to which they are attached, form a 3- or 4-membered cycloalkyl ring;
m is 0 or 1;
n is 1 or 2;
or a pharmaceutically acceptable salt thereof;
provided that the compound of formula (I) is not one of the following compounds:
   (1*S*,2*R*)-2-((*S*)-5-chloro-8-((1,5-dimethyl-1*H*-1 ,2,3-triazol-4-yl)methoxy)-7-fluoro-1-((6-oxo-5-azaspiro[2.4]heptan-5-yl)methyl)-1,2,3,4-tetrahydroisoquinoline-2-carbonyl)-1-methylcyclohexane-1-carboxylic acid;
   (1*S*,2*R*)-2-((*S*)-5-chloro-7-fluoro-1-((6-oxo-5-azaspiro[2.4]heptan-5-yl)methyl)-8-((4,5,6,7-tetrahydro-[1,2,3]triazolo[1,5-a]pyridin-3-yl)methoxy)-1,2,3,4-tetrahydroisoquinoline-2-carbonyl)-1-methylcyclohexane-1-carboxylic acid;
   (1*S*,2*R*)-2-((*S*)-5-chloro-8-((5,6-dihydro-4*H*-pyrrolo[1,2-*c*][1,2,3]triazol-3-yl)methoxy)-7-fluoro-1-((6-oxo-5-azaspiro[2.4]heptan-5-yl)methyl)-1,2,3,4-tetrahydroisoquinoline-2-carbonyl)-1-methylcyclohexane-1-carboxylic acid;
   (1*S*,2*R*)-2-((*S*)-5-chloro-8-((5-(difluoromethyl)-1-methyl-1*H*-1,2,3-triazol-4-yl)methoxy)-7-fluoro-1-((2-oxopyrrolidin-1-yl)methyl)-1,2,3,4-tetrahydroisoquinoline-2-carbonyl)-1-methylcyclohexane-1-carboxylic acid; or
   (1*S*,2*R*)-2-((*S*)-5-chloro-8-((5-(difluoromethyl)-1-methyl-1*H*-1,2,3-triazol-4-yl)methoxy)-7-fluoro-1-((6-oxo-5-azaspiro[2.4]heptan-5-yl)methyl)-1,2,3,4-tetrahydroisoquinoline-2-carbonyl)-1-methylcyclohexane-1-carboxylic acid.

Particular compounds of the disclosure (not part of the invention) include, for example, compounds of the formula (I), or pharmaceutically acceptable salts thereof, wherein, unless otherwise stated, each of R¹, R², R³, R⁴, R⁵, R⁶, X, n and m has any of the meanings defined hereinbefore or in any of paragraphs (1) to (54) hereinafter. For the avoidance of doubt, the scope of the present disclosure encompasses compounds of formula (I), or pharmaceutically acceptable salts thereof, wherein any of the substituent definitions defined herein may be combined with any of the other substituent definitions also defined herein:-
(1) R¹ is C₁₋₄alkylene-R⁴;
(2) R¹ is CH₂-R⁴;
(3) R¹ is CH₂CH₂-R⁴;
(4) R² is CO₂H;
(5) R² is tetrazolyl;
(6) R³ is hydrogen;
(7) R³ is methyl;
(8) R⁴ is 1,2,3-triazolyl optionally substituted with one or more substituents independently selected from C₁₋₄alkyl, C₁₋₃haloalkyl, C₃₋₇cycloalkyl, C₁₋₄alkylene-C₃₋₇cycloalkyl, halo, OH, C₁₋₃alkoxy, and cyano;
(9) R⁴ is 1,2,3-triazolyl optionally substituted with one or more substituents independently selected from C₁₋₄alkyl, C₁₋₃haloalkyl, C₃₋₇cycloalkyl and C₁₋₄alkylene-C₃₋₇cycloalkyl;
(10) R⁴ is 1,2,3-triazolyl optionally substituted with one or more substituents independently selected from C₁₋₄alkyl and C₁₋₃fluoroalkyl;
(11) R⁴ is 1,2,3-triazolyl substituted with one or more substituents independently selected from methyl, difluoromethyl and trifluoromethyl;
(12) R⁴ is 1,2,3-triazolyl-4-yl optionally substituted with one or more substituents independently selected from C₁₋₄alkyl, C₁₋₃haloalkyl, C₃₋₇cycloalkyl, C₁₋₄alkylene-C₃₋₇cycloalkyl, halo, OH, C₁₋₃alkoxy, and cyano;
(13) R⁴ is 1H-1,2,3-triazolyl-4-yl optionally substituted with one or more substituents independently selected from methyl, difluoromethyl and trifluoromethyl;
(14) R⁴ is 1-methyl-1H-1,2,3-triazolyl-4-yl optionally substituted with C₁₋₄alkyl, C₁₋₃haloalkyl, C₃₋₇cycloalkyl, C₁₋₄alkylene-C₃₋₇cycloalkyl, halo, OH, C₁₋₃alkoxy or cyano;
(15) R⁴ is 1-methyl-1H-1,2,3-triazolyl-4-yl optionally substituted with methyl, difluoromethyl or trifluoromethyl;
(16) R⁴ is 1,2,4-triazolyl optionally substituted with one or more substituents independently selected from C₁₋₄alkyl, C₁₋₃haloalkyl, C₃₋₇cycloalkyl, C₁₋₄alkylene-C₃₋₇cycloalkyl, halo, OH, C₁₋₃alkoxy, and cyano;
(17) R⁴ is 1,2,4-triazolyl optionally substituted with one or more substituents independently selected from C₁₋₄alkyl and C₁₋₃fluoroalkyl;
(18) R⁴ is 1,2,4-triazolyl substituted with one or more substituents independently selected from methyl, difluoromethyl and trifluoromethyl;
(19) R⁴ is 4H-1,2,4-triazol-3-yl optionally substituted with one or more substituents independently selected C₁₋₄alkyl, C₁₋₃haloalkyl, C₃₋₇cycloalkyl, C₁₋₄alkylene-C₃₋₇cycloalkyl, halo, OH, C₁₋₃alkoxy and cyano;
(20) R⁴ is 4H-1,2,4-triazol-3-yl optionally substituted with one or more substituents independently selected from methyl, difluoromethyl and trifluoromethyl;
(21) R⁴ is 4-methyl-4H-1,2,4-triazol-3-yl optionally substituted with C₁₋₄alkyl, C₁₋₃haloalkyl, C₃₋₇cycloalkyl, C₁₋₄alkylene-C₃₋₇cycloalkyl, halo, OH, C₁₋₃alkoxy or cyano;
(22) R⁴ is 4-methyl-4H-1,2,4-triazol-3-yl optionally substituted with methyl, difluoromethyl or trifluoromethyl;
(23) R⁴ is pyrimidinyl optionally substituted with one or more substituents independently selected from C₁₋₄alkyl, C₁₋₃haloalkyl, C₃₋₇cycloalkyl, C₁₋₄alkylene-C₃₋₇cycloalkyl, halo, OH, C₁₋₃alkoxy, and cyano;
(24) R⁴ is pyrimidinyl optionally substituted with one or more substituents independently selected from C₁₋₄alkyl and C₁₋₃haloalkyl;
(25) R⁴ is pyrimidinyl optionally substituted with one or more substituents independently selected from methyl, difluoromethyl and trifluoromethyl;
(26) R⁴ is pyrimidin-5-yl optionally substituted with one or more substituents independently selected from C₁₋₄alkyl, C₁₋₃haloalkyl, C₃₋₇cycloalkyl, C₁₋₄alkylene-C₃₋₇cycloalkyl, halo, OH, C₁₋₃alkoxy or cyano;
(27) R⁴ is pyrimidin-5-yl optionally substituted with methyl, difluoromethyl or trifluoromethyl;
(28) R⁴ is 1,2,3-triazolyl fused to a cycloalkyl or heterocyclyl ring;
(29) R⁴ is 1,2,3-triazol-4-yl fused to a cycloalkyl or heterocyclyl ring;
(30) R⁴ is selected from one of the following groups: 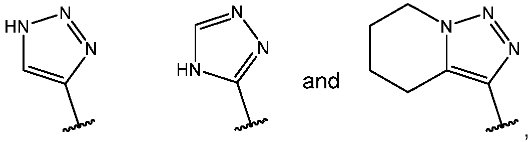 wherein said group is optionally substituted with C₁₋₄alkyl and C₁₋₃fluoroalkyl;
(31) R⁴ is selected from one of the following groups: 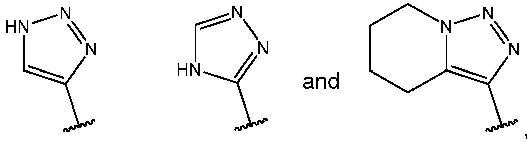 wherein said group is optionally substituted with methyl or difluoromethyl;
(32) R⁴ is selected from one of the following groups: 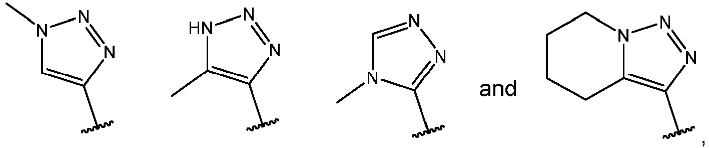 wherein said group is optionally further substituted with C₁₋₄alkyl and C₁₋₃fluoroalkyl;
(33) R⁴ is selected from one of the following groups: 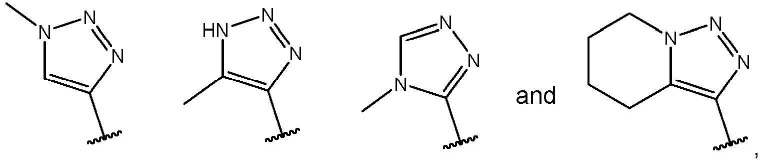 wherein said group is optionally further substituted with methyl, difluoromethyl or trifluoromethyl;
(34) R⁴ is selected from one of the following groups: 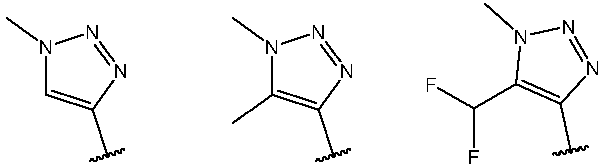 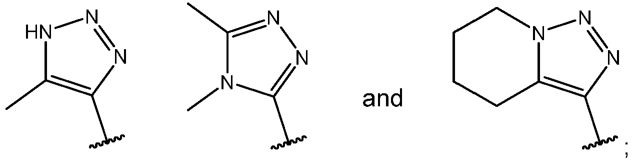
(35) R¹ is CH₂-R⁴ and R⁴ is selected from one of the following groups: 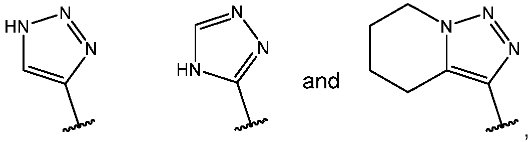 wherein said group is optionally substituted with C₁₋₄alkyl and C₁₋₃fluoroalkyl;
(36) R¹ is CH₂-R⁴ and R⁴ is selected from one of the following groups: 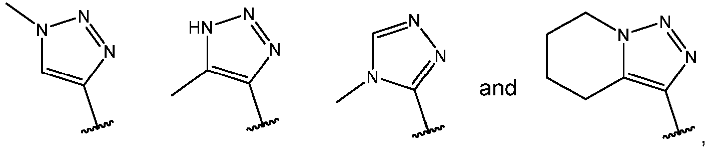 wherein said group is optionally further substituted with C₁₋₄alkyl and C₁₋₃fluoroalkyl;
(37) R¹ is CH₂-R⁴ and R⁴ is selected from one of the following groups: 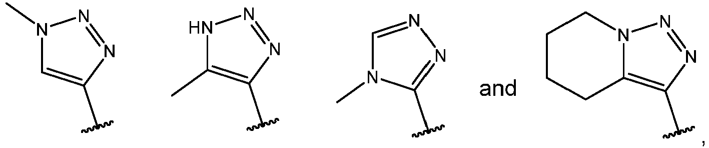 wherein said group is optionally further substituted with methyl, difluoromethyl or trifluoromethyl;
(38) R¹ is CH₂-R⁴ and R⁴ is selected from one of the following groups: 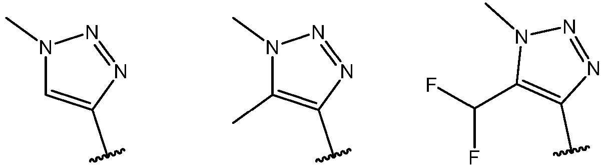 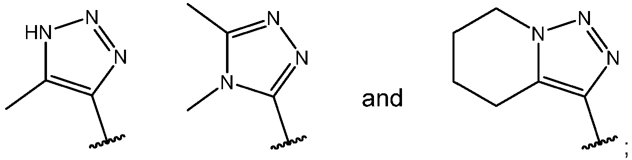
(39) R¹ is CH₂-R⁴ and R⁴ is selected from one of the following groups: 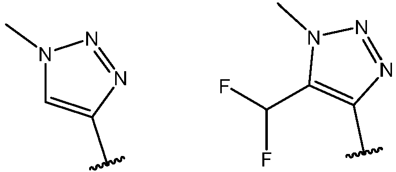 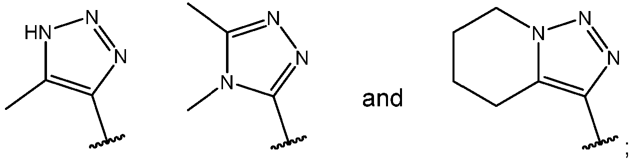
(40) X is O;
(41) X is CR⁵R⁶;
(42) R⁵ is selected from hydrogen, methyl, cyclopropyl and C₁₋₃fluoroalkyl;
(43) R⁵ is selected from hydrogen and methyl;
(44) R⁵ is methyl;
(45) R⁶ is selected from hydrogen and methyl;
(46) R⁶ is hydrogen;
(47) R⁵ is methyl and R⁶ is hydrogen;
(48) R⁵ and R⁶, together with the carbon atom to which they are attached, form a cyclopropyl ring;
(49) n is 1 and X is CR⁵R⁶;
(50) n is 1, X is CR⁵R⁶, R⁵ is methyl and R⁶ is hydrogen;
(51) n is 1, X is CR⁵R⁶ and R⁵ and R⁶, together with the carbon atom to which they are attached, form a cyclopropyl ring;
(52) n is 2 and X is O;
(53) m is 0;
(54) m is 1.

Suitably, R¹ is as defined in any one of paragraphs (1) to (3) or (35) to (39) above. In an embodiment, R¹ is as defined in paragraph (2) above. In an embodiment, R¹ is as defined in paragraph (39) above.

Suitably, R² is as defined in any one of paragraphs (4) to (5) above. In an embodiment, R² is as defined in paragraph (4) above.

Suitably, R³ is as defined in any one of paragraphs (6) to (7) above. In an embodiment, R³ is as defined in paragraph (7) above.

Suitably, R⁴ is as defined in any one of paragraphs (8) to (34) above. In an embodiment, R⁴ is as defined in paragraphs (32) to (34) above. In an embodiment, R⁴ is as defined in paragraph (34) above.

Suitably, X is as defined in any one of paragraphs (40) to (41) above. In an embodiment, X is as defined in paragraph (41) above.

Suitably, R⁵ is as defined in any one of paragraphs (42) to (44) above. In an embodiment, R⁵ is as defined in paragraph (44) above.

Suitably, R⁶ is as defined in any one of paragraphs (45) to (46) above. In an embodiment, R⁶ is as defined in paragraph (46) above.

Suitably, R⁵ and R⁶ are as defined in any one of paragraphs (47) to (48) above. In an embodiment, R⁵ and R⁶ are as defined in paragraph (48) above.

Suitably, n is as defined in any one of paragraphs (49) to (52) above. In an embodiment, n is as defined in paragraph (49) above.

Suitably, m is as defined in any one of paragraphs (53) to (54) above. In an embodiment, m is as defined in paragraph (54) above.

In a further group of compounds, the compounds have one of the structural formulae (IA) to (IK) (which are sub-formulae of Formula (I)) shown below, or a pharmaceutically acceptable salt thereof: 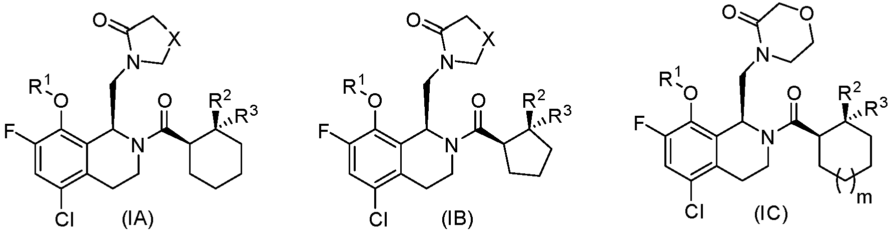 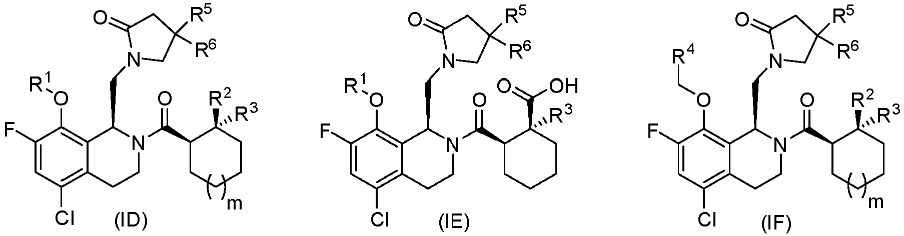 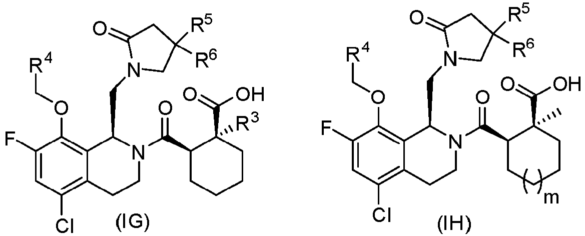 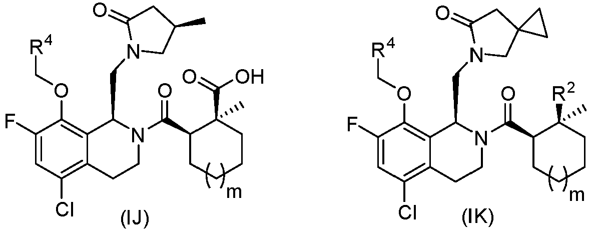 wherein R¹ to R⁶, X and m are as defined hereinbefore.

In an embodiment, the compounds have the structural formula (IA) shown above, wherein R¹ is as defined in any one of paragraphs (1) to (3) above; R² is as defined in any one of paragraphs (4) to (5) above; R³ is as defined in any one of paragraphs (6) to (7) above; and X is as defined in any one of paragraphs (40) to (41) above; provided that the compound of formula (IA) is not one of the following compounds:
(1*S*,2*R*)-2-((*S*)-5-chloro-8-((1,5-dimethyl-1*H*-1,2,3-triazol-4-yl)methoxy)-7-fluoro-1-((6-oxo-5-azaspiro[2.4]heptan-5-yl)methyl)-1,2,3,4-tetrahydroisoquinoline-2-carbonyl)-1-methylcyclohexane-1-carboxylic acid;
(1*S*,2*R*)-2-((*S*)-5-chloro-7-fluoro-1-((6-oxo-5-azaspiro[2.4]heptan-5-yl)methyl)-8-((4,5,6,7-tetrahydro-[1,2,3]triazolo[1,5-a]pyridin-3-yl)methoxy)-1,2,3,4-tetrahydroisoquinoline-2-carbonyl)-1-methylcyclohexane-1-carboxylic acid;
(1*S*,2*R*)-2-((*S*)-5-chloro-8-((5,6-dihydro-4*H*-pyrrolo[1,2-*c*][1,2,3]triazol-3-yl)methoxy)-7-fluoro-1-((6-oxo-5-azaspiro[2.4]heptan-5-yl)methyl)-1,2,3,4-tetrahydroisoquinoline-2-carbonyl)-1-methylcyclohexane-1-carboxylic acid;
(1*S*,2*R*)-2-((*S*)-5-chloro-8-((5-(difluoromethyl)-1-methyl-1*H*-1,2,3-triazol-4-yl)methoxy)-7-fluoro-1-((2-oxopyrrolidin-1-yl)methyl)-1,2,3,4-tetrahydroisoquinoline-2-carbonyl)-1-methylcyclohexane-1-carboxylic acid; or
(1*S*,2*R*)-2-((*S*)-5-chloro-8-((5-(difluoromethyl)-1-methyl-1*H*-1,2,3-triazol-4-yl)methoxy)-7-fluoro-1-((6-oxo-5-azaspiro[2.4]heptan-5-yl)methyl)-1,2,3,4-tetrahydroisoquinoline-2-carbonyl)-1-methylcyclohexane-1-carboxylic acid.

In a further group of compounds, the compounds have the structural formula (IA) shown above, wherein R¹ is as defined in paragraph (2) above; R² is as defined in paragraph (4) above; R³ is as defined in paragraph (7) above; and X is as defined paragraph (41) above.

In an embodiment, the compounds have the structural formula (IB) shown above, wherein R¹ is as defined in any one of paragraphs (1) to (3) above; R² is as defined in any one of paragraphs (4) to (5) above; R³ is as defined in any one of paragraphs (6) to (7) above; and X is as defined in any one of paragraphs (40) to (41) above.

In a further group of compounds, the compounds have the structural formula (IB) shown above, wherein R¹ is as defined in paragraph (2) above; R² is as defined in paragraph (4) above; R³ is as defined in paragraph (7) above; and X is as defined paragraph (41) above.

In an embodiment, the compounds have the structural formula (IC) shown above, wherein R¹ is as defined in any one of paragraphs (1) to (3) above; R² is as defined in any one of paragraphs (4) to (5) above; R³ is as defined in any one of paragraphs (6) to (7) above; and m is as defined in any one of paragraphs (53) to (54) above.

In a further group of compounds, the compounds have the structural formula (IC) shown above, wherein R¹ is as defined in paragraph (2) above; R² is as defined in paragraph (4) above; R³ is as defined in paragraph (7) above; and m is as defined paragraph (54) above.

In an embodiment, the compounds have the structural formula (ID) shown above, wherein R¹ is as defined in any one of paragraphs (1) to (3) above; R² is as defined in any one of paragraphs (4) to (5) above; R³ is as defined in any one of paragraphs (6) to (7) above; R⁵ is as defined in any one of paragraphs (42) to (44) above, R⁶ is as defined in any one of paragraphs (45) to (46) above; or R⁵ and R⁶ are as defined in any one of paragraphs (47) to (48) above; and m is as defined in any one of paragraphs (53) to (54) above; provided that the compound of formula (ID) is not one of the following compounds:
(1*S*,2*R*)-2-((*S*)-5-chloro-8-((1,5-dimethyl-1*H*-1,2,3-triazol-4-yl)methoxy)-7-fluoro-1-((6-oxo-5-azaspiro[2.4]heptan-5-yl)methyl)-1,2,3,4-tetrahydroisoquinoline-2-carbonyl)-1-methylcyclohexane-1-carboxylic acid;
(1*S*,2*R*)-2-((*S*)-5-chloro-7-fluoro-1-((6-oxo-5-azaspiro[2.4]heptan-5-yl)methyl)-8-((4,5,6,7-tetrahydro-[1,2,3]triazolo[1,5-a]pyridin-3-yl)methoxy)-1,2,3,4-tetrahydroisoquinoline-2-carbonyl)-1-methylcyclohexane-1-carboxylic acid;
(1*S*,2*R*)-2-((*S*)-5-chloro-8-((5,6-dihydro-4*H*-pyrrolo[1,2-*c*][1,2,3]triazol-3-yl)methoxy)-7-fluoro-1-((6-oxo-5-azaspiro[2.4]heptan-5-yl)methyl)-1,2,3,4-tetrahydroisoquinoline-2-carbonyl)-1-methylcyclohexane-1-carboxylic acid;
(1*S*,2*R*)-2-((*S*)-5-chloro-8-((5-(difluoromethyl)-1-methyl-1*H*-1,2,3-triazol-4-yl)methoxy)-7-fluoro-1-((2-oxopyrrolidin-1-yl)methyl)-1,2,3,4-tetrahydroisoquinoline-2-carbonyl)-1-methylcyclohexane-1-carboxylic acid; or
(1*S*,2*R*)-2-((*S*)-5-chloro-8-((5-(difluoromethyl)-1-methyl-1*H*-1,2,3-triazol-4-yl)methoxy)-7-fluoro-1-((6-oxo-5-azaspiro[2.4]heptan-5-yl)methyl)-1,2,3,4-tetrahydroisoquinoline-2-carbonyl)-1-methylcyclohexane-1-carboxylic acid.

In a further group of compounds, the compounds have the structural formula (ID) shown above, wherein R¹ is as defined in paragraph (2) above; R² is as defined in paragraph (4) above; R³ is as defined in paragraph (7) above; R⁵ is as defined in paragraph (44) above, R⁶ is as defined in paragraph (46) above; or R⁵ and R⁶ are as defined in paragraph (48) above; and m is as defined in paragraph (54) above.

In an embodiment, the compounds have the structural formula (IE) shown above, wherein R¹ is as defined in any one of paragraphs (1) to (3) above; R³ is as defined in any one of paragraphs (6) to (7) above; R⁵ is as defined in any one of paragraphs (42) to (44) above, R⁶ is as defined in any one of paragraphs (45) to (46) above; or R⁵ and R⁶ are as defined in any one of paragraphs (47) to (48) above; provided that the compound of formula (IE) is not one of the following compounds:
(1*S*,2*R*)-2-((*S*)-5-chloro-8-((1,5-dimethyl-1*H*-1,2,3-triazol-4-yl)methoxy)-7-fluoro-1-((6-oxo-5-azaspiro[2.4]heptan-5-yl)methyl)-1,2,3,4-tetrahydroisoquinoline-2-carbonyl)-1-methylcyclohexane-1-carboxylic acid;
(1*S*,2*R*)-2-((*S*)-5-chloro-7-fluoro-1-((6-oxo-5-azaspiro[2.4]heptan-5-yl)methyl)-8-((4,5,6,7-tetrahydro-[1,2,3]triazolo[1,5-a]pyridin-3-yl)methoxy)-1,2,3,4-tetrahydroisoquinoline-2-carbonyl)-1-methylcyclohexane-1-carboxylic acid;
(1*S*,2*R*)-2-((*S*)-5-chloro-8-((5,6-dihydro-4*H*-pyrrolo[1,2-*c*][1,2,3]triazol-3-yl)methoxy)-7-fluoro-1-((6-oxo-5-azaspiro[2.4]heptan-5-yl)methyl)-1,2,3,4-tetrahydroisoquinoline-2-carbonyl)-1-methylcyclohexane-1-carboxylic acid;
(1*S*,2*R*)-2-((*S*)-5-chloro-8-((5-(difluoromethyl)-1-methyl-1*H*-1,2,3-triazol-4-yl)methoxy)-7-fluoro-1-((2-oxopyrrolidin-1-yl)methyl)-1,2,3,4-tetrahydroisoquinoline-2-carbonyl)-1-methylcyclohexane-1-carboxylic acid; or
(1*S*,2*R*)-2-((*S*)-5-chloro-8-((5-(difluoromethyl)-1-methyl-1*H*-1,2,3-triazol-4-yl)methoxy)-7-fluoro-1-((6-oxo-5-azaspiro[2.4]heptan-5-yl)methyl)-1,2,3,4-tetrahydroisoquinoline-2-carbonyl)-1-methylcyclohexane-1-carboxylic acid.

In a further group of compounds, the compounds have the structural formula (IE) shown above, wherein R¹ is as defined in paragraph (2) above; R³ is as defined in paragraph (7) above; R⁵ is as defined in paragraph (44) above, R⁶ is as defined in paragraph (46) above; or R⁵ and R⁶ are as defined in paragraph (48) above.

In an embodiment, the compounds have the structural formula (IF) shown above, wherein R² is as defined in any one of paragraphs (4) to (5) above; R³ is as defined in any one of paragraphs (6) to (7) above; R⁴ is as defined in any one of paragraphs (8) to (34) above; R⁵ is as defined in any one of paragraphs (42) to (44) above, R⁶ is as defined in any one of paragraphs (45) to (46) above; or R⁵ and R⁶ are as defined in any one of paragraphs (47) to (48) above; and m is as defined in any one of paragraphs (53) to (54) above; provided that the compound of formula (IF) is not one of the following compounds:
(1*S*,2*R*)-2-((*S*)-5-chloro-8-((1,5-dimethyl-1*H*-1,2,3-triazol-4-yl)methoxy)-7-fluoro-1-((6-oxo-5-azaspiro[2.4]heptan-5-yl)methyl)-1,2,3,4-tetrahydroisoquinoline-2-carbonyl)-1-methylcyclohexane-1-carboxylic acid;
(1*S*,2*R*)-2-((*S*)-5-chloro-7-fluoro-1-((6-oxo-5-azaspiro[2.4]heptan-5-yl)methyl)-8-((4,5,6,7-tetrahydro-[1,2,3]triazolo[1,5-a]pyridin-3-yl)methoxy)-1,2,3,4-tetrahydroisoquinoline-2-carbonyl)-1-methylcyclohexane-1-carboxylic acid;
(1*S*,2*R*)-2-((*S*)-5-chloro-8-((5,6-dihydro-4*H*-pyrrolo[1,2-*c*][1,2,3]triazol-3-yl)methoxy)-7-fluoro-1-((6-oxo-5-azaspiro[2.4]heptan-5-yl)methyl)-1,2,3,4-tetrahydroisoquinoline-2-carbonyl)-1-methylcyclohexane-1-carboxylic acid;
(1*S*,2*R*)-2-((*S*)-5-chloro-8-((5-(difluoromethyl)-1-methyl-1*H*-1,2,3-triazol-4-yl)methoxy)-7-fluoro-1-((2-oxopyrrolidin-1-yl)methyl)-1,2,3,4-tetrahydroisoquinoline-2-carbonyl)-1-methylcyclohexane-1-carboxylic acid; or
(1*S*,2*R*)-2-((*S*)-5-chloro-8-((5-(difluoromethyl)-1-methyl-1*H*-1 ,2,3-triazol-4-yl)methoxy)-7-fluoro-1-((6-oxo-5-azaspiro[2.4]heptan-5-yl)methyl)-1,2,3,4-tetrahydroisoquinoline-2-carbonyl)-1-methylcyclohexane-1-carboxylic acid.

In a further group of compounds, the compounds have the structural formula (IF) shown above, wherein R² is as defined in paragraph (4) above; R³ is as defined in paragraph (7) above; R⁴ is as defined in paragraph (34) above; R⁵ is as defined in paragraph (44) above, R⁶ is as defined in paragraph (46) above; or R⁵ and R⁶ are as defined in paragraph (48) above; and m is as defined in paragraph (54) above.

In an embodiment, the compounds have the structural formula (IG) shown above, wherein R³ is as defined in any one of paragraphs (6) to (7) above; R⁴ is as defined in any one of paragraphs (8) to (34) above; R⁵ is as defined in any one of paragraphs (42) to (44) above, R⁶ is as defined in any one of paragraphs (45) to (46) above; or R⁵ and R⁶ are as defined in any one of paragraphs (47) to (48) above; provided that the compound of formula (IG) is not one of the following compounds:
(1*S*,2*R*)-2-((*S*)-5-chloro-8-((1,5-dimethyl-1*H*-1,2,3-triazol-4-yl)methoxy)-7-fluoro-1-((6-oxo-5-azaspiro[2.4]heptan-5-yl)methyl)-1,2,3,4-tetrahydroisoquinoline-2-carbonyl)-1-methylcyclohexane-1-carboxylic acid;
(1*S*,2*R*)-2-((*S*)-5-chloro-7-fluoro-1-((6-oxo-5-azaspiro[2.4]heptan-5-yl)methyl)-8-((4,5,6,7-tetrahydro-[1,2,3]triazolo[1,5-a]pyridin-3-yl)methoxy)-1,2,3,4-tetrahydroisoquinoline-2-carbonyl)-1-methylcyclohexane-1-carboxylic acid;
(1*S*,2*R*)-2-((*S*)-5-chloro-8-((5,6-dihydro-4*H*-pyrrolo[1,2-*c*][1,2,3]triazol-3-yl)methoxy)-7-fluoro-1-((6-oxo-5-azaspiro[2.4]heptan-5-yl)methyl)-1,2,3,4-tetrahydroisoquinoline-2-carbonyl)-1-methylcyclohexane-1-carboxylic acid;
(1*S*,2*R*)-2-((*S*)-5-chloro-8-((5-(difluoromethyl)-1-methyl-1*H*-1,2,3-triazol-4-yl)methoxy)-7-fluoro-1-((2-oxopyrrolidin-1-yl)methyl)-1,2,3,4-tetrahydroisoquinoline-2-carbonyl)-1-methylcyclohexane-1-carboxylic acid; or
(1*S*,2*R*)-2-((*S*)-5-chloro-8-((5-(difluoromethyl)-1-methyl-1*H*-1 ,2,3-triazol-4-yl)methoxy)-7-fluoro-1-((6-oxo-5-azaspiro[2.4]heptan-5-yl)methyl)-1,2,3,4-tetrahydroisoquinoline-2-carbonyl)-1-methylcyclohexane-1-carboxylic acid.

In a further group of compounds, the compounds have the structural formula (IG) shown above, wherein R³ is as defined in paragraph (7) above; R⁴ is as defined in paragraph (34) above; R⁵ is as defined in paragraph (44) above, R⁶ is as defined in paragraph (46) above; or R⁵ and R⁶ are as defined in paragraph (48) above.

In an embodiment, the compounds have the structural formula (IH) shown above, wherein R⁴ is as defined in any one of paragraphs (8) to (34) above; R⁵ is as defined in any one of paragraphs (42) to (44) above, R⁶ is as defined in any one of paragraphs (45) to (46) above; or R⁵ and R⁶ are as defined in any one of paragraphs (47) to (48) above; and m is as defined in any one of paragraphs (53) to (54) above; provided that the compound of formula (IH) is not one of the following compounds:
(1*S*,2*R*)-2-((*S*)-5-chloro-8-((1,5-dimethyl-1*H*-1,2,3-triazol-4-yl)methoxy)-7-fluoro-1-((6-oxo-5-azaspiro[2.4]heptan-5-yl)methyl)-1,2,3,4-tetrahydroisoquinoline-2-carbonyl)-1-methylcyclohexane-1-carboxylic acid;
(1*S*,2*R*)-2-((*S*)-5-chloro-7-fluoro-1-((6-oxo-5-azaspiro[2.4]heptan-5-yl)methyl)-8-((4,5,6,7-tetrahydro-[1,2,3]triazolo[1,5-a]pyridin-3-yl)methoxy)-1,2,3,4-tetrahydroisoquinoline-2-carbonyl)-1-methylcyclohexane-1-carboxylic acid;
(1*S*,2*R*)-2-((*S*)-5-chloro-8-((5,6-dihydro-4*H*-pyrrolo[1,2-*c*][1,2,3]triazol-3-yl)methoxy)-7-fluoro-1-((6-oxo-5-azaspiro[2.4]heptan-5-yl)methyl)-1,2,3,4-tetrahydroisoquinoline-2-carbonyl)-1-methylcyclohexane-1-carboxylic acid;
(1*S*,2*R*)-2-((*S*)-5-chloro-8-((5-(difluoromethyl)-1-methyl-1*H*-1,2,3-triazol-4-yl)methoxy)-7-fluoro-1-((2-oxopyrrolidin-1-yl)methyl)-1,2,3,4-tetrahydroisoquinoline-2-carbonyl)-1-methylcyclohexane-1-carboxylic acid; or
(1*S*,2*R*)-2-((*S*)-5-chloro-8-((5-(difluoromethyl)-1-methyl-1*H*-1,2,3-triazol-4-yl)methoxy)-7-fluoro-1-((6-oxo-5-azaspiro[2.4]heptan-5-yl)methyl)-1,2,3,4-tetrahydroisoquinoline-2-carbonyl)-1-methylcyclohexane-1-carboxylic acid.

In a further group of compounds, the compounds have the structural formula (IH) shown above, wherein R⁴ is as defined in paragraph (34) above; R⁵ is as defined in paragraph (44) above, R⁶ is as defined in paragraph (46) above; or R⁵ and R⁶ are as defined in paragraph (48) above; and m is as defined in paragraph (54) above.

In an embodiment, the compounds have the structural formula (IJ) shown above, wherein R⁴ is as defined in any one of paragraphs (8) to (34) above; and m is as defined in any one of paragraphs (53) to (54) above.

In a further group of compounds, the compounds have the structural formula (IJ) shown above, wherein R⁴ is as defined in any one of paragraphs (32) to (34), such as in paragraph (34) above; and m is as defined in paragraph (54) above.

In an embodiment, the compounds have the structural formula (IK) shown above, wherein R² is as defined in any one of paragraphs (4) to (5) above; R⁴ is as defined in any one of paragraphs (8) to (34) above; and m is as defined in any one of paragraphs (53) to (54) above; provided that the compound of formula (IK) is not one of the following compounds:
(1*S*,2*R*)-2-((*S*)-5-chloro-8-((1,5-dimethyl-1*H*-1,2,3-triazol-4-yl)methoxy)-7-fluoro-1-((6-oxo-5-azaspiro[2.4]heptan-5-yl)methyl)-1,2,3,4-tetrahydroisoquinoline-2-carbonyl)-1-methylcyclohexane-1-carboxylic acid;
(1*S*,2*R*)-2-((*S*)-5-chloro-7-fluoro-1-((6-oxo-5-azaspiro[2.4]heptan-5-yl)methyl)-8-((4,5,6,7-tetrahydro-[1,2,3]triazolo[1,5-a]pyridin-3-yl)methoxy)-1,2,3,4-tetrahydroisoquinoline-2-carbonyl)-1-methylcyclohexane-1-carboxylic acid;
(1*S*,2*R*)-2-((*S*)-5-chloro-8-((5,6-dihydro-4*H*-pyrrolo[1,2-*c*][1,2,3]triazol-3-yl)methoxy)-7-fluoro-1-((6-oxo-5-azaspiro[2.4]heptan-5-yl)methyl)-1,2,3,4-tetrahydroisoquinoline-2-carbonyl)-1-methylcyclohexane-1-carboxylic acid; or
(1*S*,2*R*)-2-((*S*)-5-chloro-8-((5-(difluoromethyl)-1-methyl-1*H*-1,2,3-triazol-4-yl)methoxy)-7-fluoro-1-((6-oxo-5-azaspiro[2.4]heptan-5-yl)methyl)-1,2,3,4-tetrahydroisoquinoline-2-carbonyl)-1-methylcyclohexane-1-carboxylic acid.

In a further group of compounds, the compounds have the structural formula (IK) shown above, wherein R² is as defined in paragraph (4) above; R⁴ is as defined in paragraph (34) above; and m is as defined in paragraph (54) above.

In a further group of compounds, the compounds have the structural formula (IL) (which is a sub-formula of Formula (I)) shown below, or a pharmaceutically acceptable salt thereof: 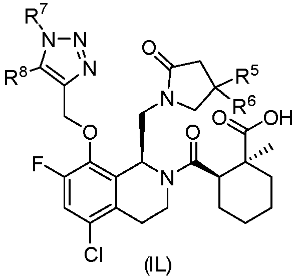 wherein R⁵ and R⁶ are as defined hereinbefore and R⁷ and R⁸ are independently selected from hydrogen, C₁₋₄alkyl, C₁₋₃haloalkyl, C₃₋₇cycloalkyl, C₁₋₄alkylene-C₃₋₇cycloalkyl, halo, OH, C₁₋₃alkoxy and cyano; or R⁷ and R⁸, taken together with the atoms to which they are attached, form a 5- or 6-membered heterocyclyl ring; provided that the compound of formula (IL) is not one of the following compounds:
(1*S*,2*R*)-2-((*S*)-5-chloro-8-((1,5-dimethyl-1*H*-1,2,3-triazol-4-yl)methoxy)-7-fluoro-1-((6-oxo-5-azaspiro[2.4]heptan-5-yl)methyl)-1,2,3,4-tetrahydroisoquinoline-2-carbonyl)-1-methylcyclohexane-1-carboxylic acid;
(1*S*,2*R*)-2-((*S*)-5-chloro-7-fluoro-1-((6-oxo-5-azaspiro[2.4]heptan-5-yl)methyl)-8-((4,5,6,7-tetrahydro-[1,2,3]triazolo[1,5-a]pyridin-3-yl)methoxy)-1,2,3,4-tetrahydroisoquinoline-2-carbonyl)-1-methylcyclohexane-1-carboxylic acid;
(1*S*,2*R*)-2-((*S*)-5-chloro-8-((5,6-dihydro-4*H*-pyrrolo[1,2-*c*][1,2,3]triazol-3-yl)methoxy)-7-fluoro-1-((6-oxo-5-azaspiro[2.4]heptan-5-yl)methyl)-1,2,3,4-tetrahydroisoquinoline-2-carbonyl)-1-methylcyclohexane-1-carboxylic acid;
(1*S*,2*R*)-2-((*S*)-5-chloro-8-((5-(difluoromethyl)-1-methyl-1*H*-1,2,3-triazol-4-yl)methoxy)-7-fluoro-1-((2-oxopyrrolidin-1-yl)methyl)-1,2,3,4-tetrahydroisoquinoline-2-carbonyl)-1-methylcyclohexane-1-carboxylic acid; or
(1*S*,2*R*)-2-((*S*)-5-chloro-8-((5-(difluoromethyl)-1-methyl-1*H*-1,2,3-triazol-4-yl)methoxy)-7-fluoro-1-((6-oxo-5-azaspiro[2.4]heptan-5-yl)methyl)-1,2,3,4-tetrahydroisoquinoline-2-carbonyl)-1-methylcyclohexane-1-carboxylic acid.

In an embodiment, the compounds have the structural formula (IL) shown above, wherein R⁵ is as defined in any one of paragraphs (42) to (44) above, R⁶ is as defined in any one of paragraphs (45) to (46) above; or R⁵ and R⁶ are as defined in any one of paragraphs (47) to (48) above; and R⁷ and R⁸ are independently selected from hydrogen, C₁₋₄alkyl, C₁₋₃haloalkyl, C₃₋₇cycloalkyl and C₁₋₂alkylene-C₃₋₇cycloalkyl; or R⁷ and R⁸, taken together with the atoms to which they are attached, form a 6-membered heterocyclyl ring.

In a further group of compounds, the compounds have the structural formula (IL) shown above, wherein R⁵ is as defined in paragraph (44) above, R⁶ is as defined in paragraph (46) above; or R⁵ and R⁶ are as defined in paragraph (48) above; and R⁷ and R⁸ are independently selected from methyl, ethyl, difluoromethyl, trifluoromethyl, cyclopropyl and CH₂-cyclopropyl; or R⁷ and R⁸, taken together with the atoms to which they are attached, form a 6-membered heterocyclyl ring.

In a further group of compounds, the compounds have the structural formula (IL) shown above, wherein R⁵ is as defined in paragraph (44) above, R⁶ is as defined in paragraph (46) above; or R⁵ and R⁶ are as defined in paragraph (48) above; R⁷ is methyl; and R⁸ is hydrogen, methyl, or difluoromethyl.

In a first aspect of the invention there is provided a compound selected from:
5-(((S)-5-chloro-8-((5-(difluoromethyl)-1-methyl-1H-1,2,3-triazol-4-yl)methoxy)-7-fluoro-2-((1R,2S)-2-methyl-2-(1H-tetrazol-5-yl)cyclohexane-1-carbonyl)-1,2,3,4-tetrahydroisoquinolin-1-yl)methyl)-5-azaspiro[2.4]heptan-6-one;
(1S,2R)-2-((S)-5-chloro-8-((5-(difluoromethyl)-1-methyl-1H-1,2,3-triazol-4-yl)methoxy)-7-fluoro-1-((3-oxomorpholino)methyl)-1,2,3,4-tetrahydroisoquinoline-2-carbonyl)-1-methylcyclohexane-1-carboxylic acid;
(1S,2R)-2-((S)-5-chloro-8-((5-(difluoromethyl)-1-methyl-1H-1,2,3-triazol-4-yl)methoxy)-7-fluoro-1-(((R)-4-methyl-2-oxopyrrolidin-1-yl)methyl)-1,2,3,4-tetrahydroisoquinoline-2-carbonyl)-1-methylcyclohexane-1-carboxylic acid;
(1S,2R)-2-((S)-5-chloro-7-fluoro-1-((6-oxo-5-azaspiro[2.4]heptan-5-yl)methyl)-8-((4,5,6,7-tetrahydro-[1,2,3]triazolo[1,5-a]pyridin-3-yl)methoxy)-1,2,3,4-tetrahydroisoquinoline-2-carbonyl)-1-methylcyclopentane-1-carboxylic acid;
(1S,2R)-2-((S)-5-chloro-8-((4,5-dimethyl-4H-1,2,4-triazol-3-yl)methoxy)-7-fluoro-1-((6-oxo-5-azaspiro[2.4]heptan-5-yl)methyl)-1,2,3,4-tetrahydroisoquinoline-2-carbonyl)-1-methylcyclohexane-1-carboxylic acid; and
(1S,2R)-2-((S)-5-chloro-7-fluoro-8-((1-methyl-1H-1,2,3-triazol-4-yl)methoxy)-1-((6-oxo-5-azaspiro[2.4]heptan-5-yl)methyl)-1,2,3,4-tetrahydroisoquinoline-2-carbonyl)-1-methylcyclohexane-1-carboxylic acid;
or a pharmaceutically acceptable salt thereof.

A suitable pharmaceutically acceptable salt of a compound of the invention is, for example, an acid-addition salt of a compound of the invention which is sufficiently basic, for example, an acid-addition salt with, for example, an inorganic or organic acid, for example hydrochloric, hydrobromic, sulfuric, phosphoric, trifluoroacetic, formic, citric or maleic acid. In addition a suitable pharmaceutically acceptable salt of a compound of the invention which is sufficiently acidic is an alkali metal salt, for example a sodium or potassium salt, an alkaline earth metal salt, for example a calcium or magnesium salt, an ammonium salt or a salt with an organic base which affords a physiologically-acceptable cation, for example a salt with methylamine, dimethylamine, trimethylamine, piperidine, morpholine or tris-(2-hydroxyethyl)amine.

Compounds that have the same molecular formula but differ in the nature or sequence of bonding of their atoms or the arrangement of their atoms in space are termed "isomers". Isomers that differ in the arrangement of their atoms in space are termed "stereoisomers". Stereoisomers that are not mirror images of one another are termed "diastereomers" and those that are non-superimposable mirror images of each other are termed "enantiomers". When a compound has an asymmetric centre, for example, it is bonded to four different groups, a pair of enantiomers is possible. An enantiomer can be characterized by the absolute configuration of its asymmetric centre and is described by the R- and S-sequencing rules of Cahn and Prelog, or by the manner in which the molecule rotates the plane of polarized light and designated as dextrorotatory or levorotatory (i.e., as (+) or (-)-isomers respectively). A chiral compound can exist as either individual enantiomer or as a mixture thereof. A mixture containing equal proportions of the enantiomers is called a "racemic mixture".

The compounds of this invention typically possess one or more asymmetric centers; such compounds can therefore be produced as individual (R)- or (S)-stereoisomers or as mixtures thereof. Unless indicated otherwise, the description or naming of a particular compound in the specification and claims is intended to include both individual enantiomers, diastereoisomers and mixtures, racemic or otherwise, thereof. The methods for the determination of stereochemistry and the separation of stereoisomers are well-known in the art (see discussion in Chapter 4 of "Advanced Organic Chemistry", 4th edition J. March, John Wiley and Sons, New York, 2001), for example by synthesis from optically active starting materials or by resolution of a racemic form. Some of the compounds of the invention may have geometric isomeric centres (E- and Z- isomers). It is to be understood that the present invention encompasses all optical, diastereoisomers and geometric isomers and mixtures thereof that possess Nrf2 activation activity.

The present invention also encompasses compounds of the invention as defined herein which comprise one or more isotopic substitutions. For example, H may be in any isotopic form, including ¹H, ²H (D) and ³H (T); C may be in any isotopic form including ¹²C, ¹³C, and ¹⁴C; and O may be in any isotopic form, including ¹⁶O and ¹⁸O; and the like.

It is also to be understood that certain compounds of the invention may exist in solvated as well as unsolvated forms such as, for example, hydrated forms. It is to be understood that the invention encompasses all such solvated forms that possess Nrf2 activation activity.

It is also to be understood that certain compounds of the invention may exhibit polymorphism, and that the invention encompasses all such forms that possess Nrf2 activation activity.

Compounds of the invention may exist in a number of different tautomeric forms and references to compounds of the invention include all such forms. For the avoidance of doubt, where a compound can exist in one of several tautomeric forms, and only one is specifically described or shown, all others are nevertheless embraced by compounds of the invention. Examples of tautomeric forms include keto-, enol-, and enolate-forms, as in, for example, the following tautomeric pairs: keto/enol (illustrated below), imine/enamine, amide/imino alcohol, amidine/amidine, nitroso/oxime, thioketone/enethiol, and nitro/aci-nitro.

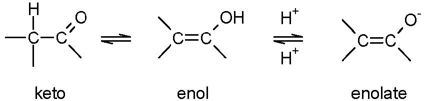

Compounds of the disclosure containing an amine function may also form *N-*oxides. A reference herein to a compound of the formula I that contains an amine function also includes the *N*-oxide. Where a compound contains several amine functions, one or more than one nitrogen atom may be oxidised to form an *N*-oxide. Particular examples of *N*-oxides are the *N*-oxides of a tertiary amine or a nitrogen atom of a nitrogen-containing heterocycle. *N*-Oxides can be formed by treatment of the corresponding amine with an oxidizing agent such as hydrogen peroxide or a per-acid (e.g. a peroxycarboxylic acid), see for example Advanced Organic Chemistry, by Jerry March, 4th Edition, Wiley Interscience, pages. More particularly, *N*-oxides can be made by the procedure of L. W. Deady (Syn. Comm. 1977, 7, 509-514) in which the amine compound is reacted with *m-*chloroperoxybenzoic acid (MCPBA), for example, in an inert solvent such as dichloromethane.

The compounds of the invention may be administered in the form of a pro-drug which is broken down in the human or animal body to release a compound of the invention. A pro-drug may be used to alter the physical properties and/or the pharmacokinetic properties of a compound of the invention. A pro-drug can be formed when the compound of the invention contains a suitable group or substituent to which a property-modifying group can be attached. Examples of pro-drugs include *in vivo* cleavable ester derivatives that may be formed at a carboxy group or a hydroxy group in a compound of the invention and *in-vivo* cleavable amide derivatives that may be formed at a carboxy group or an amino group in a compound of the invention.

Accordingly, the present invention includes those compounds of the invention when made available by organic synthesis and when made available within the human or animal body by way of cleavage of a pro-drug thereof. Accordingly, the present invention includes those compounds of the invention that are produced by organic synthetic means and also such compounds that are produced in the human or animal body by way of metabolism of a precursor compound, that is a compound of the inventionmay be a synthetically-produced compound or a metabolically-produced compound.

A suitable pharmaceutically acceptable pro-drug of a compound of the invention is one that is based on reasonable medical judgement as being suitable for administration to the human or animal body without undesirable pharmacological activities and without undue toxicity.

Various forms of pro-drug have been described, for example in the following documents:
a) Methods in Enzymology, Vol. 42, p. 309-396, edited by K. Widder, et al. (Academic Press, 1985);
b) Design of Pro-drugs, edited by H. Bundgaard, (Elsevier, 1985);
c) A Textbook of Drug Design and Development, edited by Krogsgaard-Larsen and H. Bundgaard, Chapter 5 "Design and Application of Pro-drugs", by H. Bundgaard p. 113-191 (1991);
d) H. Bundgaard, Advanced Drug Delivery Reviews, 8, 1-38 (1992);
e) H. Bundgaard, et al., Journal of Pharmaceutical Sciences, 77, 285 (1988);
f) N. Kakeya, et al., Chem. Pharm. Bull., 32, 692 (1984);
g) T. Higuchi and V. Stella, "Pro-Drugs as Novel Delivery Systems", A.C.S. Symposium Series, Volume 14; and
h) E. Roche (editor), "Bioreversible Carriers in Drug Design", Pergamon Press, 1987.

The *in vivo* effects of a compound of the inventionmay be exerted in part by one or more metabolites that are formed within the human or animal body after administration of a compound of the invention. As stated hereinbefore, the *in vivo* effects of a compound of the invention may also be exerted by way of metabolism of a precursor compound (a pro-drug).

It shall also be appreciated that compounds of the invention may also be covalently linked (at any suitable position) to other groups such as, for example, solubilising moieties (for example, PEG polymers), moieties that enable them to be bound to a solid support (such as, for example, biotin-containing moieties), and targeting ligands (such as antibodies or antibody fragments).

### Synthesis

In the description of the synthetic methods described below and in the referenced synthetic methods that are used to prepare the starting materials, it is to be understood that all proposed reaction conditions, including choice of solvent, reaction atmosphere, reaction temperature, duration of the experiment and workup procedures, can be selected by a person skilled in the art.

It is understood by one skilled in the art of organic synthesis that the functionality present on various portions of the molecule must be compatible with the reagents and reaction conditions utilised.

Necessary starting materials may be obtained by standard procedures of organic chemistry. The preparation of such starting materials is described in conjunction with the following representative process variants and within the accompanying Examples. Alternatively, necessary starting materials are obtainable by analogous procedures to those illustrated which are within the ordinary skill of an organic chemist.

It will be appreciated that during the synthesis of the compounds of the invention in the processes defined below, or during the synthesis of certain starting materials, it may be desirable to protect certain substituent groups to prevent their undesired reaction. The skilled chemist will appreciate when such protection is required, and how such protecting groups may be put in place, and later removed.

For examples of protecting groups see one of the many general texts on the subject, for example, "Protecting groups in Organic Synthesis (3rd Ed), John Wiley & Sons, NY (1999)", T. Greene & P. Wuts. Protecting groups may be removed by any convenient method described in the literature or known to the skilled chemist as appropriate for the removal of the protecting group in question, such methods being chosen so as to effect removal of the protecting group with the minimum disturbance of groups elsewhere in the molecule.

Thus, if reactants include, for example, groups such as amino, carboxy or hydroxy it may be desirable to protect the group in some of the reactions mentioned herein.

By way of example, a suitable protecting group for an amino or alkylamino group is, for example, an acyl group, for example an alkanoyl group such as acetyl, an alkoxycarbonyl group, for example a methoxycarbonyl, ethoxycarbonyl or tert-butoxycarbonyl group, an arylmethoxycarbonyl group, for example benzyloxycarbonyl, or an aroyl group, for example benzoyl. The deprotection conditions for the above protecting groups necessarily vary with the choice of protecting group. Thus, for example, an acyl group such as an alkanoyl or alkoxycarbonyl group or an aroyl group may be removed by, for example, hydrolysis with a suitable base such as an alkali metal hydroxide, for example lithium or sodium hydroxide. Alternatively, an acyl group such as a tert-butoxycarbonyl group may be removed, for example, by treatment with a suitable acid as hydrochloric, sulfuric or phosphoric acid or trifluoroacetic acid and an arylmethoxycarbonyl group such as a benzyloxycarbonyl group may be removed, for example, by hydrogenation over a catalyst such as palladium-on-carbon, or by treatment with a Lewis acid for example BF₃.OEt₂. A suitable alternative protecting group for a primary amino group is, for example, a phthaloyl group which may be removed by treatment with an alkylamine, for example dimethylaminopropylamine, or with hydrazine.

The person skilled in the art will recognise that the compounds of the invention may be prepared, in known manner, in a variety of ways. Compounds of formula (I) can be prepared by the methods given below, by the methods given in the experimental or by analogous methods. The routes described are merely illustrative of some of the methods that can be employed for the synthesis of compounds of formula (I) and the person skilled in the art will appreciate that the order of the reaction steps is not limited to those described. It will also be appreciated that the assignment of nucleophile and electrophile is not limited to that described herein and in some cases it may be appropriate for the assignment to be reversed. Different approaches to synthetic chemistry strategy are described in "Organic Synthesis: The Disconnection Approach", 2nd edition, S. Warren and P. Wyatt (2008).

### General Method A

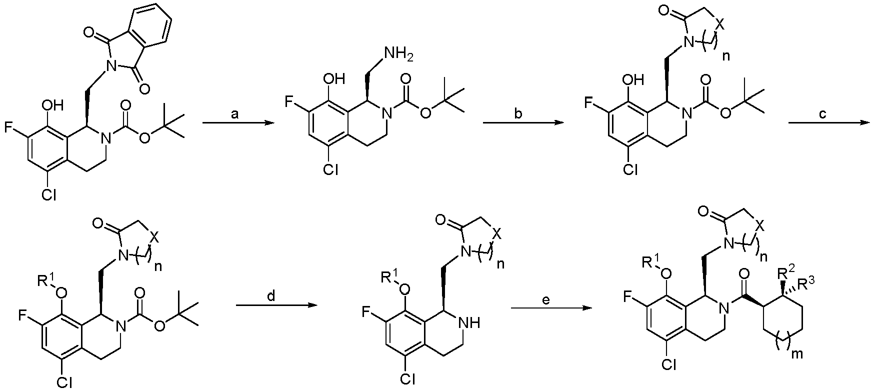

In a typical synthetic procedure the phthalimide, when used as a protecting group, is removed using typical reagents (e.g. hydrazine) and the amine is reacted with appropropriate reagents to install the desired lactam. A third step involes installation of the required ether through conventional methods such as alkylation with an alkyl halide or activated alcohol (e.g. mesylate, triflate) or Mitsunobu reaction using reagents such as DBAD or DEAD and an appropriate phosphine. The Boc protecting group is then removed typically by treatment with HCl. In the fifth step, a cycloalkyl acid group may be activated for reaction with the amine of the tetrahydroisoquinoline (THIQ) scaffold. Typical amide coupling reagents such as HATU or CDI are used to effect acid activion.

### General Method B

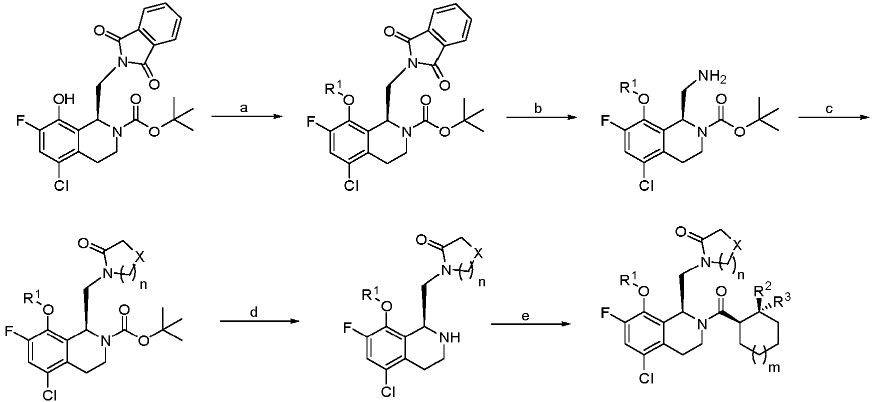

In a further typical procedure, the order of steps can be altered compared with General Method A. The required ether is installed in a first step, again through conventional methods as described in the above General Method A. The phthalimide protecting group is removed in a second step and the amine is reacted with appropropriate reagents to install the desired lactam. The Boc protecting group is then removed and cycloalkyl amide moiety may then be introduced in a fifth step.

The THIQ scaffold may be constructed according to the route outlined in Scheme 1:

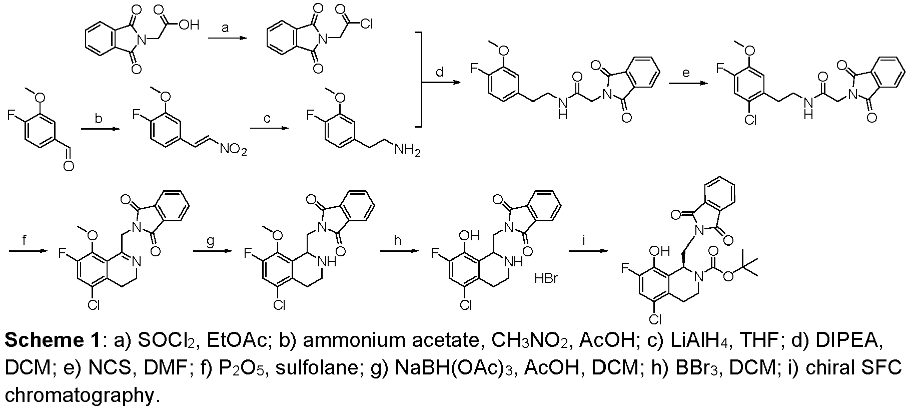

Compounds where R² is -COOH can be converted to tetrazolyl *via* reaction of the appropriate nitrile with an azide, as shown in Scheme 2.

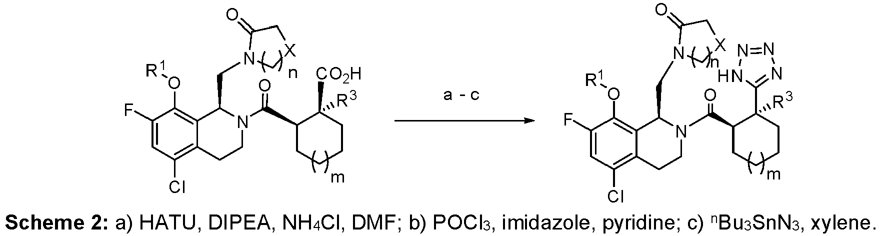

Compounds of the invention wherein the group -NR⁴R⁵ represents a pyrrolidinone may be prepared from intermediates wherein -NR⁴R⁵ represents a phthalimide group by removal of the phthalimide group with hydrazine, followed by conversion of the resulting primary amine to a pyrrolidinone by reaction with an appropriate lactone or ω-halo ester, according to the routes outlined in Scheme 3.

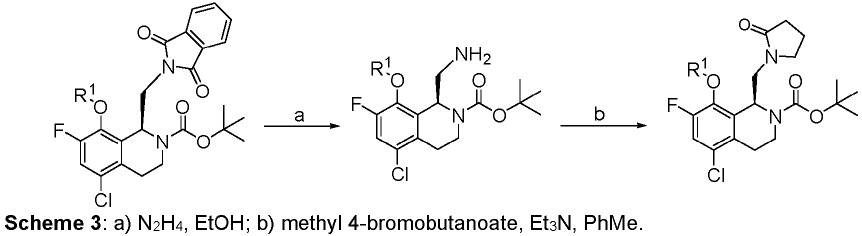

### Pharmaceutical Compositions

The compounds of the invention will normally, but not necessarily, be formulated into pharmaceutical compositions prior to administration to a patient. Therefore, according to a further aspect of the invention there is provided a pharmaceutical composition which comprises a compound of the invention as defined hereinbefore, or a pharmaceutically acceptable salt thereof, and one or more pharmaceutically acceptable excipients, diluent or carrier.

The pharmaceutical compositions of the invention may be prepared and packaged in bulk form wherein a safe and effective amount of a compound of the invention can be extracted and then given to the patient such as with powders or syrups. Alternatively, the pharmaceutical compositions of the invention may be prepared and packaged in unit dosage form wherein each physically discrete unit contains a safe and effective amount of a compound of the invention. When prepared in unit dosage form, the pharmaceutical compositions of the invention typically contain from 1 mg to 1000 mg.

The compositions of the invention may be in a form suitable for oral use (for example as tablets, capsules, caplets, pills, troches, powders, syrups, elixirs, suspensions, solutions, emulsions, sachets, and cachets), for topical use (for example as creams, ointments, lotions, solutions, pastes, sprays, foams, and gels), for transdermal administration such as *via* transdermal patches, for administration by inhalation (for example as a dry powders, aerosols, suspensions, and solutions), for administration by insufflation (for example as a finely divided powder) or for parenteral administration (for example as a sterile aqueous or oily solution for intravenous, subcutaneous, intramuscular, intraperitoneal or intramuscular dosing or as a suppository for rectal dosing).

As used herein, "pharmaceutically-acceptable excipient" means a pharmaceutically acceptable material, composition or vehicle involved in giving form or consistency to the pharmaceutical composition. Each excipient must be compatible with the other ingredients of the pharmaceutical composition when commingled such that interactions which would substantially reduce the efficacy of the compound of the invention when administered to a patient and interactions which would result in pharmaceutical compositions that are not pharmaceutically acceptable are avoided. In addition, each excipient must be of sufficiently high purity to render it pharmaceutically-acceptable.

Suitable pharmaceutically-acceptable excipients will vary depending upon the particular dosage form chosen. In addition, suitable pharmaceutically-acceptable excipients may be chosen for a particular function that they may serve in the composition. For example, certain pharmaceutically-acceptable excipients may be chosen for their ability to facilitate the production of uniform dosage forms. Certain pharmaceutically-acceptable excipients may be chosen for their ability to facilitate the production of stable dosage forms. Certain pharmaceutically-acceptable excipients may be chosen for their ability to facilitate the carrying or transporting of the compound or compounds of the invention once administered to the patient from one organ, or portion of the body, to another organ, or portion of the body. Certain pharmaceutically-acceptable excipients may be chosen for their ability to enhance patient compliance.

Suitable pharmaceutically-acceptable excipients include the following types of excipients: diluents, fillers, binders, disintegrants, lubricants, glidants, granulating agents, coating agents, wetting agents, solvents, co-solvents, suspending agents, emulsifiers, sweeteners, flavoring agents, flavor masking agents, coloring agents, anticaking agents, humectants, chelating agents, plasticizers, viscosity increasing agents, antioxidants, preservatives, stabilizers, surfactants, and buffering agents. The person skilled in the art will appreciate that certain pharmaceutically-acceptable excipients may serve more than one function and may serve alternative functions depending on how much of the excipient is present in the formulation and what other ingredients are present in the formulation.

Persons skilled in the art possess the knowledge and skill to enable them to select suitable pharmaceutically-acceptable excipients in appropriate amounts for use in the invention. In addition, there are a number of resources that are available to the skilled artisan which describe pharmaceutically-acceptable excipients and may be useful in selecting suitable pharmaceutically-acceptable excipients. Examples include Remington's Pharmaceutical Sciences (Mack Publishing Company), The Handbook of Pharmaceutical Additives (Gower Publishing Limited), and The Handbook of Pharmaceutical Excipients (the American Pharmaceutical Association and the Pharmaceutical Press).

The pharmaceutical compositions of the invention are prepared using techniques and methods known to those skilled in the art. Some of the methods commonly used in the art are described in Remington's Pharmaceutical Sciences (Mack Publishing Company).

The amount of active ingredient that is combined with one or more excipients to produce a single dosage form will necessarily vary depending upon the host treated and the particular route of administration. For example, a formulation intended for oral administration to humans will generally contain, for example, from 0.5 mg to 0.5 g of active agent (more suitably from 0.5 to 100 mg, for example from 1 to 30 mg) compounded with an appropriate and convenient amount of excipients which may vary from about 5 to about 98 percent by weight of the total composition.

The size of the dose for therapeutic or prophylactic purposes of a compound of the formula I will naturally vary according to the nature and severity of the conditions, the age and sex of the animal or patient and the route of administration, according to well-known principles of medicine.

In using a compound of the invention for therapeutic or prophylactic purposes it will generally be administered so that a daily dose in the range, for example, 0.1 mg/kg to 75 mg/kg body weight is received, given if required in divided doses. In general, lower doses will be administered when a parenteral route is employed. Thus, for example, for intravenous or intraperitoneal administration, a dose in the range, for example, 0.1 mg/kg to 30 mg/kg body weight will generally be used. Similarly, for administration by inhalation, a dose in the range, for example, 0.05 mg/kg to 25 mg/kg body weight will be used. Oral administration may also be suitable, particularly in tablet form. Typically, unit dosage forms will contain about 0.5 mg to 0.5 g of a compound of this invention.

### Routes of Administration

The compounds of the invention or pharmaceutical composition comprising the active compound may be administered to a subject by any convenient route of administration, whether systemically/peripherally or topically (i.e. at the site of desired action).

Routes of administration include, but are not limited to, oral (e.g., by ingestion); buccal; sublingual; transdermal (including, e.g., by a patch, plaster, etc.); transmucosal (including, e.g., by a patch, plaster, etc.); intranasal (e.g., by nasal spray); ocular (e.g., by eyedrops); pulmonary (e.g., by inhalation or insufflation therapy using, e.g., *via* an aerosol, e.g., through the mouth or nose); rectal (e.g., by suppository or enema); vaginal (e.g., by pessary); parenteral, for example, by injection, including subcutaneous, intradermal, intramuscular, intravenous, intraarterial, intracardiac, intrathecal, intraspinal, intracapsular, subcapsular, intraorbital, intraperitoneal, intratracheal, subcuticular, intraarticular, subarachnoid, and intrasternal; by implant of a depot or reservoir, for example, subcutaneously or intramuscularly.

In a preferred embodiment, a compound of the invention as defined herein, or a pharmaceutically acceptable salt thereof, or a pharmaceutical composition as defined herein, is administered orally or *via* inhalation.

### Therapeutic Uses and Applications

The compounds of the invention are activators of Nrf2. As a consequence, they are potentially useful therapeutic agents for the treatment of diseases or conditions mediated by Nrf2 activation.

Thus, in one aspect, the present invention relates to a compound of the invention as defined herein, or a pharmaceutically acceptable salt thereof, or a pharmaceutical composition as defined herein, for use in therapy.

In another aspect, the present invention relates to a compound of the invention as defined herein, or a pharmaceutically acceptable salt thereof, or a pharmaceutical composition as defined herein, for use in the treatment of diseases or disorders mediated by Nrf2 activation.

In another aspect, the present invention relates to the use of a compound of the invention as defined herein, or a pharmaceutically acceptable salt thereof, in the manufacture of a medicament for use in the treatment of diseases or disorders mediated by Nrf2 activation.

Examples of particular diseases or conditions that the compounds of the invention and their pharmaceutically acceptable salts may be used to treat include, but are not limited to, any one of the following: chronic obstructive pulmonary disease, acute, chronic and severe asthma, acute lung injury/acute respiratory distress syndrome with or without accompanying multi organ dysfunction syndrome, pulmonary fibrosis including idiopathic pulmonary fibrosis, cystic fibrosis, COVID-19, diabetes, atherosclerosis, hypertension, heart failure, myocardial infarction and repair, cardiac remodelling, cardiac arrhythmias, cardiac hypertrophy, heart failure with preserved ejection fraction, diabetic cardiomyopathy, sarcopenia, obesity, metabolic syndrome, diabetes mellitus, insulin resistance, pulmonary arterial hypertension, subarachnoid haemorrhage, intracerebral haemorrhage, ischemic stroke, beta-thalassemia, sickle cell disease, rheumatoid arthritis, irritable bowel disorder, ulcerative colitis, Crohn's disease, psoriasis, radiation-induced dermatitis, atopic dermatitis, non-alcoholic fatty liver disease, non-alcoholic steatohepatitis, toxin-induced liver disease, viral hepatitis and cirrhosis, chronic kidney disease, diabetic nephropathy, autosomal dominant polycystic kidney disease, CKD associated with type 1 diabetes (T1D), IgA nephropathy (IgAN), Alport Syndrome, focal segmental glomerulosclerosis, Huntington's disease, Parkinson's disease, Alzheimer's disease, amyotrophic lateral sclerosis, frontotemporal dementia, multiple sclerosis, Friedreich's ataxia, chronic pain, schizophrenia, lung cancer, breast cancer, colon cancer, age related macular degeneration (AMD), Fuchs Endothelial Corneal Dystrophy, uveitis or preeclampsia.

In particular, the compounds of the invention (including pharmaceutically acceptable salts) may be used in the treatment of chronic obstructive pulmonary disease, asthma, pulmonary arterial hypertension, diabetes mellitus, chronic kidney disease, ulcerative colitis, Crohn's disease, inflammatory bowel disease, Friedreich's ataxia, sickle cell disease or non-alcoholic steatohepatitis.

In another aspect, the present invention provides a compound, or a pharmaceutically acceptable salt thereof, or a pharmaceutical composition as defined herein, for use in the treatment of chronic obstructive pulmonary disease, acute, chronic and severe asthma, acute lung injury/acute respiratory distress syndrome with or without accompanying multi organ dysfunction syndrome, pulmonary fibrosis including idiopathic pulmonary fibrosis, cystic fibrosis, COVID-19, diabetes, atherosclerosis, hypertension, heart failure, myocardial infarction and repair, cardiac remodelling, cardiac arrhythmias, cardiac hypertrophy, heart failure with preserved ejection fraction, diabetic cardiomyopathy, sarcopenia, obesity, metabolic syndrome, diabetes mellitus, insulin resistance, pulmonary arterial hypertension, subarachnoid haemorrhage, intracerebral haemorrhage, ischemic stroke, beta-thalassemia, sickle cell disease, rheumatoid arthritis, irritable bowel disorder, ulcerative colitis, Crohn's disease, psoriasis, radiation-induced dermatitis, atopic dermatitis, non-alcoholic fatty liver disease, non-alcoholic steatohepatitis, toxin-induced liver disease, viral hepatitis and cirrhosis, chronic kidney disease, diabetic nephropathy, autosomal dominant polycystic kidney disease, CKD associated with type 1 diabetes (T1D), IgA nephropathy (IgAN), Alport Syndrome, focal segmental glomerulosclerosis, Huntington's disease, Parkinson's disease, Alzheimer's disease, amyotrophic lateral sclerosis, frontotemporal dementia, multiple sclerosis, Friedreich's ataxia, chronic pain, schizophrenia, lung cancer, breast cancer, colon cancer, age related macular degeneration (AMD), Fuchs Endothelial Corneal Dystrophy, uveitis or preeclampsia.

In another aspect, the present invention provides a compound, or a pharmaceutically acceptable salt thereof, or a pharmaceutical composition as defined herein, for use in the treatment of chronic obstructive pulmonary disease, asthma, pulmonary arterial hypertension, diabetes mellitus, chronic kidney disease, Friedreich's ataxia, sickle cell disease or non-alcoholic steatohepatitis.

In another aspect, the present invention provides the use of a compound, or a pharmaceutically acceptable salt thereof, in the manufacture of a medicament for use in the treatment of chronic obstructive pulmonary disease, acute, chronic and severe asthma, acute lung injury/acute respiratory distress syndrome with or without accompanying multi organ dysfunction syndrome, pulmonary fibrosis including idiopathic pulmonary fibrosis, cystic fibrosis, COVID-19, diabetes, atherosclerosis, hypertension, heart failure, myocardial infarction and repair, cardiac remodelling, cardiac arrhythmias, cardiac hypertrophy, heart failure with preserved ejection fraction, diabetic cardiomyopathy, sarcopenia, obesity, metabolic syndrome, diabetes mellitus, insulin resistance, pulmonary arterial hypertension, subarachnoid haemorrhage, intracerebral haemorrhage, ischemic stroke, beta-thalassemia, sickle cell disease, rheumatoid arthritis, irritable bowel disorder, ulcerative colitis, Crohn's disease, psoriasis, radiation-induced dermatitis, atopic dermatitis, non-alcoholic fatty liver disease, non-alcoholic steatohepatitis, toxin-induced liver disease, viral hepatitis and cirrhosis, chronic kidney disease, diabetic nephropathy, autosomal dominant polycystic kidney disease, CKD associated with type 1 diabetes (T1D), IgA nephropathy (IgAN), Alport Syndrome, focal segmental glomerulosclerosis, Huntington's disease, Parkinson's disease, Alzheimer's disease, amyotrophic lateral sclerosis, frontotemporal dementia, multiple sclerosis, Friedreich's ataxia, chronic pain, schizophrenia, lung cancer, breast cancer, colon cancer, age related macular degeneration (AMD), Fuchs Endothelial Corneal Dystrophy, uveitis or preeclampsia.

In another aspect, the present invention provides the use of a compound, or a pharmaceutically acceptable salt thereof, in the manufacture of a medicament for use in the treatment of chronic obstructive pulmonary disease, asthma, pulmonary arterial hypertension, diabetes mellitus, chronic kidney disease, ulcerative colitis, Crohn's disease, inflammatory bowel disease, Friedreich's ataxia, sickle cell disease or non-alcoholic steatohepatitis.

In another aspect, the present invention provides a method of activating Nrf2 *in vitro,* said method comprising administering an effective amount of a compound, or a pharmaceutically acceptable salt thereof.

### Combination Therapies

The compounds of the invention may be administered alone as a monotherapy or may administered in combination with one or more additional therapeutic agents. The selection of the one or more additional therapeutic agents will of course vary depending on the disease or condition to be treated and its severity.

It is commonplace to use combination therapies to treat certain medical conditions.

According to a particular aspect of the disclosure there is provided a combination suitable for use in the treatment of a disease or condition in which Nrf2 activation is implicated, comprising a compound of the invention as defined hereinbefore, or a pharmaceutically acceptable salt thereof, and another therapeutic agent.

According to this aspect of the disclosure there is provided a combination suitable for use in the prevention or treatment of chronic obstructive pulmonary disease, acute, chronic and severe asthma, acute lung injury/acute respiratory distress syndrome with or without accompanying multi organ dysfunction syndrome, pulmonary fibrosis including idiopathic pulmonary fibrosis, cystic fibrosis, COVID-19, diabetes, atherosclerosis, hypertension, heart failure, myocardial infarction and repair, cardiac remodelling, cardiac arrhythmias, cardiac hypertrophy, heart failure with preserved ejection fraction, diabetic cardiomyopathy, sarcopenia, obesity, metabolic syndrome, diabetes mellitus, insulin resistance, pulmonary arterial hypertension, subarachnoid haemorrhage, intracerebral haemorrhage, ischemic stroke, beta-thalassemia, sickle cell disease, rheumatoid arthritis, irritable bowel disorder, ulcerative colitis, Crohn's disease, psoriasis, radiation-induced dermatitis, atopic dermatitis, non-alcoholic fatty liver disease, non-alcoholic steatohepatitis, toxin-induced liver disease, viral hepatitis and cirrhosis, chronic kidney disease, diabetic nephropathy, autosomal dominant polycystic kidney disease, CKD associated with type 1 diabetes (T1D), IgA nephropathy (IgAN), Alport Syndrome, focal segmental glomerulosclerosis, Huntington's disease, Parkinson's disease, Alzheimer's disease, amyotrophic lateral sclerosis, frontotemporal dementia, multiple sclerosis, Friedreich's ataxia, chronic pain, schizophrenia, lung cancer, breast cancer, colon cancer, age related macular degeneration (AMD), Fuchs Endothelial Corneal Dystrophy, uveitis or preeclampsia, the combination comprising a compound of the invention as defined hereinbefore, or a pharmaceutically acceptable salt thereof, and one or more additional therapeutic agents.

In a further aspect of the disclosure there is provided a compound of the invention or a pharmaceutically acceptable salt thereof, in combination with one or more additional therapeutic agents.

Herein, where the term "combination" is used it is to be understood that this refers to simultaneous, separate or sequential administration. In one aspect of the invention "combination" refers to simultaneous administration. In another aspect of the invention "combination" refers to separate administration. In a further aspect of the invention "combination" refers to sequential administration. Where the administration is sequential or separate, the delay in administering the second component should not be such as to lose the beneficial effect of the combination.

According to a further aspect of the disclosure there is provided a pharmaceutical composition which comprises a compound of the invention, or a pharmaceutically acceptable salt thereof in combination with one or more additional therapeutic agents in association with a pharmaceutically acceptable diluent or carrier.

The one or more additional therapeutic agents may comprise a further compound of the present invention. Therefore, in an embodiment, there is provided a pharmaceutical composition which comprises two compounds of the invention, or pharmaceutically acceptable salts thereof, in association with a pharmaceutically acceptable diluent or carrier.

According to a particular aspect of the disclosure there is provided a combination suitable for use in the prevention or treatment of allergic disease, inflammatory disease or autoimmune disease (e.g. asthma or COPD); a cardiovascular or metabolic disease (e.g. diabetes); a neurodegenerative disease; a chronic kidney or liver disease; sickle cell disease; pulmonary arterial hypertension; cancer; or for aiding transplantation.

According to a particular aspect of the disclosure there is provided a combination suitable for use in the prevention or treatment of chronic obstructive pulmonary disease, asthma, pulmonary arterial hypertension, diabetes mellitus, chronic kidney disease, ulcerative colitis, Crohn's disease, inflammatory bowel disease, Friedreich's ataxia, sickle cell disease or non-alcoholic steatohepatitis.

Examples of other therapeutic agents that may be used as part of a combination therapy with a compound of the present invention (e.g. as one of two or more active agents as part of double or triple combinations) include, but are not limited to, the following:
(i) beta2-adrenoreceptor agonists (which may be a racemate or a single enantiomer) including salmeterol, salbutamol, formoterol, salmefamol, fenoterol, carmoterol, etanterol, naminterol, clenbuterol, pirbuterol, flerbuterol, reproterol, bambuterol, indacaterol, terbutaline, vilanterol, olodaterol and salts thereof;
(ii) anticholinergic agents that act as antagonists at the muscarinic receptors that include ipratropium (for example, as the bromide, CAS 22254-24-6, sold under the name Atrovent), oxitropium and tiotropium (for example, as the bromide, CAS 136310-93-5, sold under the name Spiriva), revatropate, LAS- 34273, Aclidinium, Glycopyrronium, Umeclidinium and salts thereof;
(iii) Corticosteroid anti-inflammatory agents. Examples include methyl prednisolone, prednisolone, dexamethasone, fluticasone propionate, fluticasone furoate, beclomethasone esters (for example the 17-propionate ester or the 17,21 -dipropionate ester), budesonide, flunisolide, mometasone esters (for example mometasone furoate), triamcinolone acetonide, rofleponide, ciclesonide, butixocort propionate, RPR-106541, and ST-126;
(iv) Anti-inflammatory agents including non-steroidal anti-inflammatory drugs (NSAIDs). Examples of NSAID's include sodium cromoglycate, nedocromil sodium, phosphodiesterase (PDE) inhibitors (for example, theophylline, PDE4 inhibitors or mixed PDE3/PDE4 inhibitors), leukotriene antagonists, JAK inhibitors, Pi3K inhibitors, inhibitors of leukotriene synthesis (for example montelukast), iNOS inhibitors, tryptase and elastase inhibitors, beta-2 integrin antagonists and adenosine receptor agonists or antagonists (e.g. adenosine 2a agonists), cytokine antagonists (for example chemokine antagonists, such as a CCR3 antagonist) or inhibitors of cytokine synthesis, or 5 -lipoxygenase inhibitors;
(v) Vasodilator and anti-proliferative agents (e.g. prostanoids and PDE5 inhibitors) including Epoprostenol (Flolan), Treprostinil (Remodulin), Iloprost (Ventavis), Treprostinil (Tyvaso), Bosentan (Tracleer), Ambrisentan (Letairis), Sildenafil (Revatio), Tadalafil (Adcirca);
(vi) Anti-diabetic medications including insulins, biguanides (e.g. metformin), sulfonylureas (e.g. glimepiride), meglitinides (e.g. repaglinide), thiazolidinediones (e.g., pioglitazone), dipeptidyl peptidase IV inhibitors (e.g. sitagliptin), incretin mimetics/GLP-1 analogues (e.g. liraglutide, exenatide, dulaglutide), sodium glucose co-transporter-2 (SGLT2) inhibitors (e.g. canagliflozin, dapagliflozin and empagliflozin) and α-glucosidase inhibitors (e.g. acarbose);
(vii) Hydroxyurea and other agents used to treat sickle cell disease such as L-glutamine, NCX1443, GBT440 (voxelotor), pan-Selectin antagonists (GMI-1070, rivipansel), humanized anti-P-Selectin antibody (SelG1, crinalizumab), P-selectin aptamers, sevuparin, Regadenoson, Ticagrelor, N-Acetyl-Cysteine (NAC), phosphodiesterase 9 inhibitors (e.g. PF-04447943, IMR-687, BAY 73-6691, BAY 41-2271); and
(viii) ASK1 inhibitors such as selonsertib, FXR agonists such as obeticholic acid, GS-9674, Px-102, ACC inhibitors such as GS-0976 and PPARα/δ agonists such as Elafibranor.

The combinations referred to above may conveniently be presented for use in the form of a pharmaceutical formulation and thus pharmaceutical formulations comprising a combination as defined above together with a pharmaceutically acceptable diluent or carrier represent a further aspect of the disclosure.

Such conjoint/combination treatment may be achieved by way of the simultaneous, sequential or separate dosing of the individual components of the treatment. In one embodiment, the individual compounds will be administered simultaneously in a combined pharmaceutical formulation.

Such combination therapies employ the compounds of this invention within the dosage range described herein and the other pharmaceutically active agent within approved dosage ranges and/or the dosage such as described in the relevant publication reference.

### EXAMPLES

### General Procedures:

Methods for preparing the compounds of this invention are illustrated in the following Examples. Starting materials are made according to procedures known in the art, or as illustrated herein, or are available commercially. Commercial reagents were used without further purification. Where no reaction temperature is included, the reaction was performed at ambient temperature which is typically 18-27°C.

Where compounds described in the invention are characterized by ¹H NMR spectroscopy, spectra were recorded on 500 MHz Bruker, 400 MHz Bruker, 250 MHz Bruker, 300 MHz JEOL or 400 MHz JEOL instruments. Where no temperature is included, the spectra were recorded at ambient temperature. Chemical shift values are expressed in parts per million (ppm). Where NMR spectra are complex due to the presence of interconverting isomers, approximate partial integrations of signals are reported, or characterisation for the major isomer only is reported. The following abbreviations are used for the multiplicity of the NMR signals: s=singlet, b=broad, t=triplet, q=quartet, m=multiplet, d=doublet.

### Analytical LCMS

Where compounds described in the invention are characterized by LCMS data, retention time and molecular weight are determined using the methods listed in the table below. In cases where compounds of the invention appear as slowly interconverting stereoisomers, multiple retention times are reported.

| **Method** | **Instrument** | **Column** | | **Eluents** | **Gradient** |
|---|---|---|---|---|---|
| **1** | Acquity H-Class (quaternary pump with PDA detector) and QDa Mass Spectrometer | Acquity UPLC CSH C18 (1.7 µm, 2.1 × 50 mm) | 50 °C | A: 0.1% aq. formic acid in water | 3 - 99% B from 0.0 to 1.5 min |
| | | | | B: MeCN (containing 0.1% formic acid) | |
| **2** | Acquity H-Class (quaternary pump with PDA detector) and QDa Mass Spectrometer | XBridge BEH C18 (2.5 µm, 2.1 × 50 mm) | 40 °C | A: 0.1% aq. ammonia | 3 - 95% B from 0.2 to 2.2 min, 95% B to 2.7 min |
| | | | | B: MeCN (containing 0.1% ammonia) | |
| **3** | Acquity UPLC (binary pump with PDA detector) and ZQ Mass Spectrometer | Acquity UPLC BEH C18 (1.7 µm, 2.1 × 100 mm) | 40 °C | A: 0.1% formic acid in water | 5 - 95% B from 0.4 to 6.0 min |
| | | | | B: MeCN (containing 0.1% formic acid) | |
| **4** | Acquity UPLC (binary pump with PDA detector) and ZQ Mass Spectrometer | Acquity UPLC BEH C18 (1.7µm, 2.1 × 100 mm) | 40 °C | A: 0.03% aq. ammonia | 5 - 95% from 0.4 min to 6.0 min |
| | | | | B: MeCN (containing 0.03% ammonia) | |

### Preparative Chiral SFC

Preparative chiral SFC was performed using a variant of the method outlined below.

### Method 1:

Waters Thar Prep100 preparative SFC system (P200 CO₂ pump, 2545 modifier pump, 2998 UV/VIS detector, 2767 liquid handler with Stacked Injection Module). Column: Diacel Chiralpak IA/IB/IC, YMC Amylose / Cellulose C (5 µm, 20-21.2 × 250 mm), maintained at 40°C. Conditions: supercritical fluid CO₂ and eluents chosen from MeOH, EtOH, IPA, MeCN, EtOAc, THF with modifiers chosen from Me₂NH, formic acid as specified. Gradient/isocratic as specified.

### Abbreviations:

| | |
|---|---|
| DCM | Dichloromethane |
| DEAD | Diethyl azodicarboxylate |
| DIPEA | *N,N*-Diisopropylethylamine |
| DMF | *N,N*-Dimethylformamide |
| DMSO | Dimethylsulfoxide |
| Et₂O | Diethyl ether |
| EtOAc | Ethyl acetate |
| EtOH | Ethanol |
| h | Hour(s) |
| HATU | *N*-[(Dimethylamino)-1*H*-1,2,3-triazolo-[4,5-*b*]pyridin-1-ylmethylene]-*N-*methylmethanaminium hexafluorophosphate *N*-oxide |
| HPLC | High Performance Liquid Chromatography |
| IMS | Industrial methylated spirits |
| IPA | Isopropyl alcohol |
| LCMS | Liquid Chromatography Mass Spectrometry |
| LDA | Lithium diisopropylamine |
| MeCN | Acetonitrile |
| MeOH | Methanol |
| 2-MeTHF | 2-Methyl tetrahydrofuran |
| min | Minute(s) |
| NCS | *N*-chlorosuccinimide |
| NMR | Nuclear Magnetic Resonance |
| SFC | Supercritical fluid chromatography |
| TFA | Trifluoroacetic acid |

### Synthesis of Intermediates

### Intermediate 1: (E)-1-fluoro-2-methoxy-4-(2-nitrovinyl)benzene

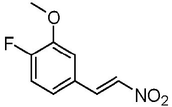

A solution of 4-fluoro-3-methoxybenzaldehyde (57 g, 370 mmol; CAS: 128495-46-5), ammonium acetate (14.25 g, 185 mmol), and nitromethane (100.14 mL, 1850 mmol) in acetic acid (150 mL) was heated at 100°C for 5 h. The reaction mixture was allowed to cool to rt overnight. The resulting solid was collected by filtration, washed with diethyl ether and the solid dried *in vacuo.* The solid was suspended in DCM (1 L) and washed with water. The organic layer was filtered to remove precipitate, dried (Na₂SO₄), filtered and concentrated *in vacuo* to give the title compound (42 g, 200 mmol, 54%). The precipitate was dissolved in 2-MeTHF, and the organic layer was washed with water, brine, dried (Na₂SO₄) and evaporated to give further product (11 g, 55.2 mmol, 15% yield) as a yellow solid. The acetic acid mother liquors were evaporated and diluted with IMS. The resulting solid was collected by filtration and washed with IMS to provide another batch of the product (1.5 g). These batches were combined to give the title compound (54.5 g, 74%) used without further purification. ¹H NMR (300 MHz, CDCl₃) δ 7.95 (d, 1H), 7.52 (d, 1H), 7.16 - 7.08 (m, 3H), 3.95 (s, 3H).

### Intermediate 2: 2-(4-fluoro-3-methoxyphenyl)ethan-1-amine

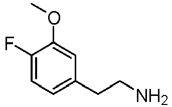

Sulfuric acid (8.92 mL, 167 mmol) was added dropwise under nitrogen to a stirred solution of lithium aluminium hydride in THF (2M; 12.7 g, 335 mmol) pre-cooled in an ice-salt bath. The mixture was stirred for 15 min until all gas evolution had subsided. A solution of Intermediate 1 (22 g, 112 mmol) in 2-MeTHF (660 mL) was added dropwise, ensuring that the temperature remained < 20°C. The cooling bath was removed and the mixture was heated under reflux for 5 min, then cooled in an ice salt bath. IPA (57 mL) was added dropwise followed by sodium hydroxide (2 M, 39 mL). MgSO₄ was added and the mixture was stirred for 30 min then filtered through Celite^{®}. The filter cake was washed with 2-MeTHF/IPA ~98:2 (~1.5 L) followed by 10% MeOH in DCM (~1.5 L). The filtrate was concentrated *in vacuo* to give the title compound (18.8 g, 99%) used without further purification. ¹H NMR (400 MHz, CDCl₃) δ 7.02 - 6.97 (m, 1H), 6.80 (dd, 1H), 6.74 - 6.68 (m, 1H), 3.89 - 3.88 (s, 3H), 2.96 (t, 2H), 2.71 (t, 2H), 1.24 - 1.18 (m, 2H).

### Intermediate 3: 2-(1,3-dioxoisoindolin-2-yl)-N-(4-fluoro-3-methoxyphenethyl)acetamide

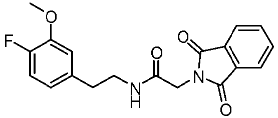

To a solution of Intermediate 2 (49.3 g, 291 mmol) and DIPEA (101.5 mL, 583 mmol) in DCM (250 mL) under nitrogen cooled in an ice-salt bath was added a solution of Intermediate 1 (65.2 g, 291 mmol) in DCM (1.25 L) dropwise. The mixture was stirred warming from 0°C to room temperature over 2h. The resulting precipitate was isolated by filtration and washed thoroughly with DCM. The solid was dried *in vacuo* to give the title compound (83 g, 80%) used without further purification. ¹H NMR (400 MHz, CDCl₃) δ 7.89 - 7.86 (m, 2H), 7.77 - 7.74 (m, 2H), 6.90 (dd, 1H), 6.78 (dd, 1H), 6.66 (ddd, 1H), 5.75 (s, 1H), 4.29 (s, 2H), 3.88 (s, 3H), 3.52 (q, 2H), 2.79 (t, 2H).

### Intermediate 4: N-(2-chloro-4-fluoro-5-methoxyphenethyl)-2-(1,3-dioxoisoindolin-2-yl)acetamide

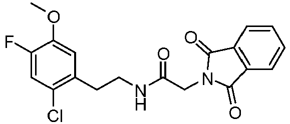

A mixture of Intermediate 3 (82.6 g, 232 mmol) and NCS (34.05 g, 255 mmol) in DMF (780 mL) was heated to 50°C and stirred for 1 h at 50°C, then cooled and concentrated *in vacuo.* To the resulting residue was added water (~2 L) and the resulting precipitate stirred for 1 h. The solid was isolated by filtration, washed with water, Et₂O, air dried and dried *in vacuo* to give the title compound (85.8 g, 94%) used without further purification. LCMS (Method 2): 1.43 min, 391.3 [M+H]⁺.

### Intermediate 5: 2-((5-chloro-7-fluoro-8-methoxy-3,4-dihydroisoquinolin-1-yl)methyl)isoindoline-1,3-dione

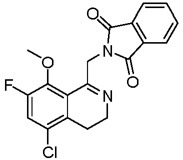

A suspension of sulfolane (794 mL, 8330 mmol) and Intermediate 4 (30.0 g, 76.8 mmol) was heated to 90°C where upon the solid dissolved. Phosphorus pentoxide (65.4 g, 461 mmol; CAS: 1314-56-3) was added in one portion and the mixture was stirred at 90-100°C for 1h. The mixture was cooled to ~30°C and poured onto stirred water (3 L). The solution was neutralised with Na₂CO₃ to pH 7.5 and the mixture was filtered. The precipitate was diluted in DCM (500 mL) and the organics washed with water, brine, dried MgSO₄ and concentrated *in vacuo* to give the title compound (27.0 g, 68.8 mmol, 90% yield). LCMS (Method 2): 1.64 min, 373.2 [M+H]⁺.

### Intermediate 6: 2-((5-chloro-7-fluoro-8-methoxy-1,2,3,4-tetrahydroisoquinolin-1-yl)methyl)isoindoline-1,3-dione

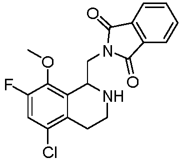

A stirred suspension of Intermediate 5 (50.3 g, 135 mmol) in DCM (600 mL) under argon was cooled in an ice bath. To this was added acetic acid (15.5 mL, 270 mmol) and sodium triacetoxyborohydride (57.2 g, 270 mmol) portion wise over 30 min. The mixture was stirred for 18 h warming to rt. To the mixture was then added further acetic acid (3.09 mL, 54.0 mmol) and sodium triacetoxyborohydride (11.4 g, 54.0 mmol) and stirring continued for 2.5 h. The mixture was diluted with DCM and neutralised with a saturated solution of sodium hydrogen carbonate. This was extracted with further DCM and the combined organics washed with brine, dried with Na₂SO₄ and concentrated *in vacuo* to give the title compound (48.8 g, 77%). LCMS (Method 1): 0.96 min, 375.0 [M+H]⁺.

### Intermediate 7: 2-((5-chloro-7-fluoro-8-hydroxy-1,2,3,4-tetrahydroisoquinolin-1-yl)methyl)isoindoline-1,3-dione hydrobromide

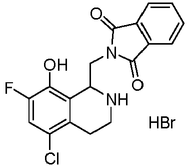

To a stirred solution of Intermediate 6 (37.0 g, 98.7 mmol) in DCM (500 mL) cooled in an ice bath under nitrogen was added boron tribromide in DCM (1 M; 395 mL, 395 mmol; CAS: 10294-33-4) dropwise and the reaction mixture was allowed to warm to rt and stirred for 72 h. The liquid was quenched by dropwise addition onto ice-water (1.0 L) and a solid precipitated. Ice water was added to the remaining residue in the reaction flask, the mixture was sonicated for 0.5 h and a solid precipitated. Both suspensions were filtered and the collected solids combined, washed with water, azeotroped with methanol and dried *in vacuo* to provide the title compound (43.0 g, 99%). LCMS (Method 1): 0.92 min, 361.0 [M+H]⁺.

### Intermediate 8: tert-butyl (S)-5-chloro-1-((1,3-dioxoisoindolin-2-yl)methyl)-7-fluoro-8-hydroxy-3,4-dihydroisoquinoline-2(1H)-carboxylate

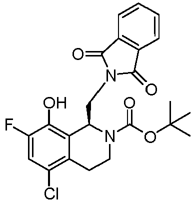

To a suspension of Intermediate 7 (43.0 g, 97.4 mmol) in DCM (900 mL) under nitrogen was added DIPEA (67.5 mL, 389 mmol) followed by di-tert-butyl dicarbonate (19.1 g, 87.6 mmol; CAS: 24424-99-5) portion wise and the resulting mixture was stirred until no more gas evolution was observed. The reaction mixture was diluted with water, extracted with DCM and the combined organics washed with water (x4), brine, dried (MgSO₄) and concentrated *in vacuo.* The residue was refluxed in MeCN (900 mL) for 2 h, cooled and the solid isolated by filtration to give a beige solid that was air-dried for 18 h to give 34 g of racemic material. The racemate was separated by SFC (Method 1: YMC Amylose-C 20x250mm, 5um 20/80 EtOH (0.1% DEA)/CO2, 100ml/min, 120bar, 40°C, DAD 230nm) to give the title compound (first eluting enantiomer; 15.9 g, 35% yield). Absolute stereochemistry confirmed by small molecule X-ray crystallography of the carboxylic acid final compound arising from enantiomer 2. LCMS (Method 2): 1.50 min, 459.2 [M-H]⁻. ¹H NMR (400 MHz; DMSO-d₆) δ 7.95 - 7.80 (m, 4H), 7.42 - 7.37 (m, 1H), 5.60 - 5.45 (m, 1H), 4.19 - 3.75 (m, 3H), 3.45 - 3.35 (m, 1H), 2.83-2.79 (m, 1H), 2.63 - 2.53 (m, 1H), 1.03-0.95 (m, 9H).

### Intermediate 9: (1R,2S)-2-[(2,4-Dimethoxyphenyl)methoxycarbonyl]cyclohexanecarboxylic acid; (1R)-1-phenylethanamine

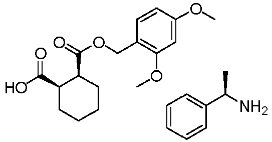

A solution of (2,4-dimethoxyphenyl)methanol (43.6 g, 259 mmol, CAS: 7314-44-5) in toluene (80 ml) was added dropwise to a suspension of *cis-1,2-*cyclohexanedicarboxylic anhydride (20.0 g, 130 mmol, CAS: 13149-00-3) and (S)-(6-methoxy-4-quinolyl)-[(2*R*,4*S*,5*R*)-5-vinylquinuclidin-2-yl]methanol (46.3 g, 143 mmol, CAS: 56-54-2) in toluene (150 ml) at -5 °C. The solution was then transferred to a fridge and allowed to stand for 4 days. The reaction mixture was warmed to rt, washed with 1M aqueous HCl (200 mL) until the washesd were pH 1, and the organic washed with water, brine (100 mL), filtered through a phase separator and concentrated *in vacuo.* The residue was diluted with toluene (100 mL) and to this was added (1R)-1-phenylethanamine (16.5 mL, 130 mmol; CAS 3886-69-9). The mixture was seeded with a few crystals of solid material from a previous batch and stirred at rt for 24 h. The resulting solid was collected, washed with toluene, triturated with Et₂O and the solid further sonicated in Et₂O, filtered and dried *in vacuo* to give the title compound (45.9 g, 80%). LCMS (Method 1): 1.44 min, 345.0 [M+Na]⁺.

### Intermediate 10: (1R,2S)-2-(((2,4-Dimethoxybenzyl)oxy)carbonyl)cyclohexane-1-carboxylic acid

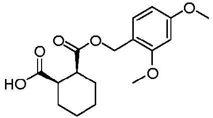

Intermediate 9 (45.9 g, 103 mmol) was partitioned between citric acid (10% aqueous; 300 mL) and EtOAc (300 mL). The aqueous layer was extracted with EtOAc and the combined organics washed with water, brine, dried over Na₂SO₄, filtered and concentrated *in vacuo* and dried *in vacuo* to give the title compound (33.7 g, assumed quantitative), used without further purification. ¹H NMR (400 MHz, CDCl₃) δ 7.22-7.20 (m, 1H), 6.46-6.43 (m, 2H), 5.15-5.04 (q, 2H), 3.80 (s, 3H), 3.79 (s, 3H), 2.89-2.81 (m, 2H), 2.08-2.00 (m, 2H), 1.81-1.73 (m, 2H), 1.58-1.38 (m, 4H).

### Intermediate 11: (1R,2S)-2-(((2,4-Dimethoxybenzyl)oxy)carbonyl)-2-methylcyclohexane-1-carboxylic acid

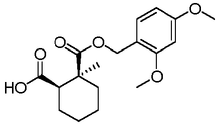

To a stirred solution of Intermediate 10 (20.4 g, 63.1 mmol) in anhydrous THF (150 mL) cooled to -25 °C under argon was added LDA (2M in THF/hexanes; 158 mL, 158 mmol) dropwise. The mixture was stirred at -25 °C for 30 min then iodomethane (11.8 mL, 189 mmol) was added dropwise, and the reaction mixture allowed to warm to rt over 4 h. The reaction was quenched with saturated aqueous NH₄Cl solution, extracted with EtOAc and the combined organics washed with 10% aqueous citric acid solution, the aqueous layer was further extracted with EtOAc and the combined organics were washed with brine, dried over Na₂SO₄, filtered and concentrated *in vacuo* to give the title compound (23.6 g, assumed quantitative). LCMS (Method 1): 1.60 min, 359.1 [M+Na]⁺.

### Intermediate 12: 2-(3H-[1,2,3]triazolo[4,5-b]pyridin-3-yl) 1-(2,4-dimethoxybenzyl) (1S,2R)-1-methylcyclohexane-1,2-dicarboxylate

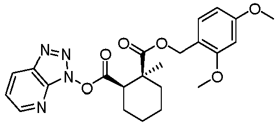

To a stirred solution of Intermediate 11 (21.2 g, 63.1 mmol) in DMF (112 mL) at rt under argon was added HATU (31.2 g, 82.1 mmol; CAS: 148893-10-1) and the reaction mixture was stirred for 5 min. To the mixture was added DIPEA (12.1 mL, 69.5 mmol) and the mixture was stirred at rt for 18 h. The mixture was diluted with water, extracted with EtOAc and the combined organics were washed with water, brine, dried over Na₂SO₄, filtered and concentrated *in vacuo.* Purification on the Teledyne ISCO CombiFlash^{®} Rf+ (330 g silica column Puriflash HC, 0 - 50% EtOAc in cyclohexane) gave the title compound (25.0 g, 87%). LCMS (Method 1): 1.70 min, 477.2 [M+Na]⁺.

### Intermediate 13: (1R,2S)-2-[(2,4-Dimethoxyphenyl)methoxycarbonyl]cyclopentanecarboxylic acid; (1R)-1-phenylethanamine

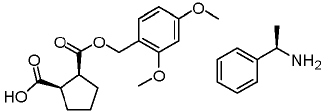

To a stirred suspension of (3a*R*,6a*S*)-4,5,6,6a-tetrahydro-3a*H*-cyclopenta-[c]furan-1,3-dione (14.8 g, 106 mmol, CAS: 35878-28-5) and (S)-(6-methoxy-4-quinolyl)-[(2*R*,4*S*,5*R*)-5-vinylquinuclidin-2-yl]methanol (37.7 g, 117 mmol, CAS: 56-54-2) in toluene (100 mL) was added (2,4-dimethoxyphenyl)methanol (35.5 g, 211 mmol) in toluene (70 mL) dropwise at -5 °C. The solution was then transferred to a fridge and allowed to stand for 4 days. The solution was warmed to rt, washed with 1M aqueous HCl (400 mL) until pH 1 and the organic layer was washed with water, brine and filtered through a phase separator and concentrated *in vacuo.* The residue was diluted with toluene (90 mL) and to this was added (1*R*)-1-phenylethanamine (13.4 mL, 106 mmol; CAS 3886-69-9) and the reaction mixture stirred at rt for 24 h. The resulting solid was collected, washed with toluene, triturated with Et₂O and the solid further sonicated in Et₂O, filtered and dried *in vacuo* to give the title compound (34.0 g, 75%). LCMS (Method 1): 1.34 min, 331.0 [M+Na]⁺.

### Intermediate 14: (1R,2S)-2-(((2,4-Dimethoxybenzyl)oxy)carbonyl)cyclopentane-1-carboxylic acid

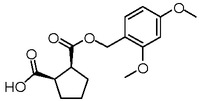

Intermediate 13 (34.0 g, 79.1 mmol) was partitioned between 10% aqueous citric acid solution (300 mL) and EtOAc (300 mL). The aqueous was further extracted with EtOAc and the combined organics washed with water, brine, dried over Na₂SO₄, filtered and concentrated *in vacuo* to provide the title compound (24.9 g, assumed quantitative), used without further purification. LCMS (Method 1): 1.33 min, 331.0 [M+Na]⁺.

### Intermediate 15: (1R,2S)-2-(((2,4-Dimethoxybenzyl)oxy)carbonyl)-2-methylcyclopentane-1-carboxylic acid

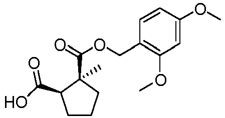

To a stirred solution of diisopropylamine (0.57 mL, 4.05 mmol; CAS: 108-18-9) in anhydrous THF (3.6 mL) at -78 °C under argon was added n-butyllithium (2.5 M in THF; 1.6 mL, 4.05 mmol) and the reaction mixture stirred at -78 °C for 15 mins. To this was added Intermediate 14 (500 mg, 1.62 mmol) in anhydrous THF (3.6 mL) dropwise and the reaction mixture stirred at -78 °C for 15 mins. lodomethane (0.3 mL, 4.86 mmol) was then added and the reaction mixture stirred at -78 °C for 15 mins then warmed to 0 °C over 1 h. The reaction mixture was diluted with saturated aqueous ammonium chloride solution (50 mL) and 10% citric acid solution and the mixture extracted with EtOAc. The combined organics were washed with brine, dried over Na₂SO₄, filtered and concentrated *in vacuo* to give the title compound (561 mg, assumed quantitative), used without further purification. LCMS (Method 1): 1.47 min, 345.0 [M+Na]⁺.

### Intermediate 16: 2-(3H-[1,2,3]triazolo[4,5-b]pyridin-3-yl) 1-(2,4-dimethoxybenzyl) (1S,2R)-1-methylcyclopentane-1,2-dicarboxylate

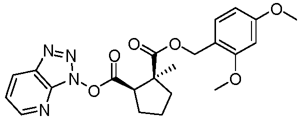

To a stirred solution of Intermediate 15 (561 mg, 1.74 mmol) in DMF (1.5 mL) was added HATU (728 g, 1.91 mmol) and the resulting mixture was stirred at rt under argon for 5 min. DIPEA (0.33 mL, 1.91 mmol) was added and the resulting mixture was stirred at rt for 16 h. The reaction mixture was concentrated *in vacuo* and purified by flash column chromatography on the Teledyne ISCO CombiFlash^{®} Rf+ (40 g silica column Puriflash HC, 0 - 50% EtOAc in isohexane) to provide the title compound (307 mg, 40%). LCMS (Method 1): 1.62 min, 463.1 [M+Na]⁺.

### Synthesis of Examples

### Example 1: 5-(((S)-5-chloro-8-((5-(difluoromethyl)-1-methyl-1H-1,2,3-triazol-4-yl)methoxy)-7-fluoro-2-((1R,2S)-2-methyl-2-(1H-tetrazol-5-yl)cyclohexane-1-carbonyl)-1,2,3,4-tetrahydroisoquinolin-1-yl)methyl)-5-azaspiro[2.4]heptan-6-one

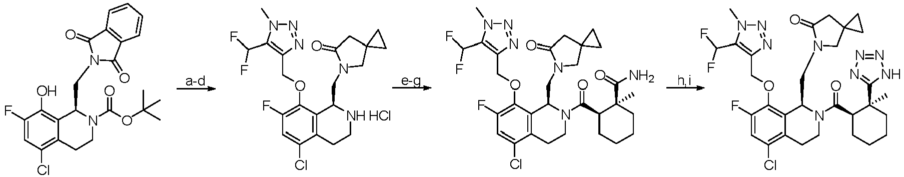

### Step a.

To a stirred solution of Intermediate 8 (3.65 g, 7.92 mmol) in DMF (14 mL) at rt under nitrogen was added 4-(chloromethyl)-5-(difluoromethyl)-1-methyl-triazole (1.87 g, 10.3 mmol; CAS: 2138555-23-2) and cesium carbonate (7.74 g, 23.8 mmol) and the reaction mixture was stirred at rt for 18 h. To this was added water and the mixture extracted with EtOAc. The combined organics were washed with water, brine, dried over Na₂SO₄, filtered and concentrated *in vacuo.* The residue was purified by flash column chromatography on the Teledyne ISCO CombiFlash^{®} Rf+ (120 g silica column Puriflash HC, 100% DCM followed by 0 - 30% EtOAc in DCM) to give tert-butyl (S)-5-chloro-8-((5-(difluoromethyl)-1-methyl-1*H*-1,2,3-triazol-4-yl)methoxy)-1-((1,3-dioxoisoindolin-2-yl)methyl)-7-fluoro-3,4-dihydroisoquinoline-2(1*H*)-carboxylate (4.07 g, 85%). LCMS (Method 1): 1.78 min, 628.1 [M+Na]⁺.

### Step b.

To a stirred solution of the above intermediate (4.07 g, 6.72 mmol) in EtOH (34 mL) was added hydrazine monohydrate (1.26 mL, 16.8 mmol; CAS: 7803-57-8) and the resulting mixture was heated at 75 °C for 2 h. The reaction mixture was allowed to cool to rt, diluted with cold MeCN, filtered and the filtrate concentrated *in vacuo* and dried *in vacuo* to give *tert*-butyl (S)-1-(aminomethyl)-5-chloro-8-((5-(difluoromethyl)-1-methyl-1*H-*1,2,3-triazol-4-yl)methoxy)-7-fluoro-3,4-dihydroisoquinoline-2(1*H*)-carboxylate (3.04 g, 95%). LCMS (Method 1): 1.12 min, 476.2 [M+H]⁺.

### Step c.

To a stirred solution of the above intermediate (3.04 g, 6.39 mmol) in toluene (26 mL) was added methyl 2-[1-(bromomethyl)cyclopropyl]acetate (1.46 g, 7.03 mmol; CAS: 855473-50-6) and triethylamine (1.34 mL, 9.58 mmol) was heated under reflux for 16 h. The reaction mixture was allowed to cool to rt, concentrated *in vacuo* and the residue was partitioned between DCM and brine. The organic phase was separated and the aqueous was further extracted with DCM (x 2). The combined organics were washed with brine, dried over Na₂SO₄ and concentrated *in vacuo.* The residue was purified by flash column chromatography on the Teledyne ISCO CombiFlash^{®} Rf+ (80 g silica column Puriflash HC, 0 - 100% EtOAc in cyclohexane) to give tert-butyl (S)-5-chloro-8-((5-(difluoromethyl)-1-methyl-1H-1 ,2,3-triazol-4-yl)methoxy)-7 -fluoro-1-((6-oxo-5-azaspiro[2.4]heptan-5-yl)methyl)-3,4-dihydroisoquinoline-2(1H)-carboxylate (3.06 g, 84%). LCMS (Method 1): 1.66 min, 592.2 [M+Na]⁺.

### Step d.

A solution of the above intermediate (3.06 g, 5.37 mmol) in HCl in dioxane (4M; 26.8 mL) was stirred at rt for 1 h. The reaction mixture was concentrated *in vacuo,* azeotroped with chloroform and dried *in vacuo* to give (S)-5-((5-chloro-8-((5-(difluoromethyl)-1-methyl-1*H*-1,2,3-triazol-4-yl)methoxy)-7-fluoro-1,2,3,4-tetrahydroisoquinolin-1-yl)methyl)-5-azaspiro[2.4]heptan-6-one hydrochloride (2.93 g, quantitative). LCMS (Method 1): 0.98 min, 470.1 [M+H]⁺.

### Step e.

To a stirred solution of the above intermediate (6.93 g, 13.7 mmol) and Intermediate 12 (9.33 g, 20.5 mmol) in DMF (16 mL) was added DIPEA (4.74 mL, 27.4 mmol) and the resulting mixture was stirred at rt under argon for 5 days. To a stirred solution of the above intermediate (2.72 g, 5.37 mmol) and Intermediate 12 (3.66 g, 8.05 mmol) in DMF (6.3 mL) was added DIPEA (1.86 mL, 10.74 mmol) and the resulting mixture was stirred at rt under argon for 4 days. The two reaction mixtures were combined, diluted with EtOAc and saturated sodium bicarbonate solution and the mixture extracted with EtOAc. The combined organics were washed with water, brine, dried over Na₂SO₄, filtered and concentrated *in vacuo.* The crude product was purified by flash column chromatography on the Teledyne ISCO CombiFlash^{®} Rf+ (300 g silica column Puriflash HC, 0 - 100% EtOAc in cyclohexane) to give 2,4-dimethoxybenzyl (1*S*,2*R*)-2-((*S*)-5-chloro-8-((5-(difluoromethyl)-1-methyl-1*H*-1,2,3-triazol-4-yl)methoxy)-7-fluoro-1-((6-oxo-5-azaspiro[2.4]heptan-5-yl)methyl)-1,2,3,4-tetrahydroisoquinoline-2-carbonyl)-1-methylcyclohexane-1-carboxylate (4.1 g, 27%). LCMS (Method 2): 3.39 min, 788.5 [M+H]⁺. Impure product-containing fractions were combined, concentrated *in vacuo* and further purified by flash column chromatography on the Teledyne ISCO CombiFlash^{®} Rf+ (200 g silica column Puriflash HC, 0 - 100% EtOAc in cyclohexane) to provide another batch of product (7.06 g, 8.96 mmol). LCMS (Method 2): 3.40 min, 788.6 [M+H]⁺. Both batches were combined to give 2,4-dimethoxybenzyl (1*S*,2*R*)-2-((*S*)-5-chloro-8-((5-(difluoromethyl)-1-methyl-1*H*-1 ,2,3-triazol-4-yl)methoxy)-7-fluoro-1-((6-oxo-5-azaspiro-[2.4]heptan-5-yl)methyl)-1,2,3,4-tetrahydroisoquinoline-2-carbonyl)-1-methylcyclohexane-1-carboxylate (11.16 g, 14.158 mmol, 55%) used without further purification.

### Step f.

To a stirred solution of the above intermediate (11.16 g, 14.16 mmol) in DCM (68 mL) was added triethylsilane (2.26 mL, 14.16 mmol), followed by TFA (1.09 mL, 14.16 mmol) and the resulting mixture was stirred at rt for 2 h. Further TFA (0.28 mL, 3.54 mmol) was added and the mixture stirred for 0.5 h. The reaction mixture was concentrated *in vacuo* and the residue dried *in vacuo* for 18 h. The residue was taken up in DCM, filtered and the filtrate purified by flash column chromatography on the Teledyne ISCO CombiFlash^{®} Rf+ (300 g silica column Puriflash HC, 0 - 100% EtOAc in cyclohexane), followed by flash column chromatography on the Teledyne ISCO CombiFlash^{®} Rf+ (120 g silica column Puriflash HC, 0 - 50% methyl acetate in DCM) and then by flash column chromatography on the Teledyne ISCO CombiFlash^{®} Rf+ (300 g silica column Puriflash HC, 1 - 10% MeOH in DCM) to give (1S,2R)-2-((S)-5-chloro-8-((5-(difluoromethyl)-1-methyl-1*H*-1,2,3-triazol-4-yl)methoxy)-7-fluoro-1-((6-oxo-5-azaspiro[2.4]heptan-5-yl)methyl)-1,2,3,4-tetrahydroisoquinoline-2-carbonyl)-1-methylcyclohexane-1-carboxylic acid as a solid (4.4 g). Impure product-containing fractions were combined, concentrated *in vacuo* and further purified by flash column chromatography to provide additional product (1.26 g). Both batches were combined to give (1S,2R)-2-((S)-5-chloro-8-((5-(difluoromethyl)-1-methyl-1*H*-1,2,3-triazol-4-yl)methoxy)-7-fluoro-1-((6-oxo-5-azaspiro[2.4]heptan-5-yl)methyl)-1,2,3,4-tetrahydroisoquinoline-2-carbonyl)-1-methylcyclohexane-1-carboxylic acid (5.66 g, 63%). LCMS (Method 4): 4.68 min, 638.1 [M+H]⁺. ¹H NMR (400 MHz, CDCl₃) δ 7.20 (d, 1H), 6.82 (t, 1H), 5.48 (dd, 1H), 5.40 (d, 1H), 5.23 (d, 1H), 4.16 (s, 3H), 4.06 (dd, 1H), 4.00-3.89 (m, 1H), 3.83 (m, 1H), 3.24 (d, 1H), 3.13 (d, 1H), 3.08 (dd, 1H), 2.99 (m, 1H), 2.67 (m, 1H), 2.57 (dd, 1H), 2.47-2.36 (m, 2H), 2.07 (d, 1H), 1.85-1.72 (m, 2H), 1.70-1.43 (m, 3H), 1.41-1.23 (m, 1H), 1.12 (s, 3H), 1.03 (m, 1H), 0.69-0.50 (m, 4H).

### Step g.

To a stirred solution of the above intermediate (315 mg, 0.49 mmol), HATU (206 mg, 0.54 mmol; CAS: 148893-10-1) and ammonium chloride (29 mg, 0.54 mmol) in DMF (1.6 mL) was added DIPEA (0.17 mL, 0.99 mmol) and the reaction mixture was stirred at rt for 18 h. The reaction mixture was diluted saturated sodium bicarbonate solution and extracted with EtOAc. The combined organics were washed with water and brine, dried over Na₂SO₄, filtered and concentrated *in vacuo.* The crude product was purified by flash column chromatography on the Teledyne ISCO CombiFlash^{®} Rf+ (25 g silica column Puriflash HC, 0 - 5% MeOH in DCM) to give (1*S*,2*R*)-2-((*S*)-5-chloro-8-((5-(difluoromethyl)-1-methyl-1*H*-1,2,3-triazol-4-yl)methoxy)-7-fluoro-1-((6-oxo-5-azaspiro[2.4]heptan-5-yl)methyl)-1,2,3,4-tetrahydroisoquinoline-2-carbonyl)-1-methylcyclohexane-1-carboxamide (270 mg, 86%). LCMS (Method 2): 2.53 min, 637.4 [M+H]⁺.

### Step h.

To a stirred solution of the above intermediate (240 mg, 0.38 mmol) in DCM (2.5 mL) was added trifluoroacetic anhydride (0.16 mL, 1.13 mmol) and the resulting mixture was stirred at rt under nitrogen for 3 h. The reaction mixture was concentrated *in vacuo* and the crude product purified by flash column chromatography on the Teledyne ISCO CombiFlash^{®} Rf+ (25 g silica column Puriflash HC, 15 um, 0 - 5% MeOH in DCM) to give (1*S*,2*R*)-2-((*S*)-5-chloro-8-((5-(difluoromethyl)-1-methyl-1*H*-1,2,3-triazol-4-yl)methoxy)-7-fluoro-1-((6-oxo-5-azaspiro[2.4]heptan-5-yl)methyl)-1,2,3,4-tetrahydroisoquinoline-2-carbonyl)-1-methylcyclohexane-1-carbonitrile (197 mg, 84%). LCMS (Method 1): 1.56 min, 619.3 [M+H]⁺.

### Step i.

To a stirred solution of the above intermediate (145 mg, 0.23 mmol) and azidotributyltin(IV) (0.41 mL, 1.5 mmol; CAS: 17846-68-3) were added together in α,α,α-trifluorotoluene (4.0 mL) and the resulting mixture was heated at 180°C for 19 h under microwave irradiation. In a separate tube, to a stirred solution of the above intermediate (25 mg, 0.04 mmol) and azidotributyltin(IV) (0.09 mL, 0.32 mmol; CAS: 17846-68-3) were added together in α,α,α-trifluorotoluene (0.8 mL) and the resulting mixture was heated at 180°C for 19 h under microwave irradiation. The reaction mixtures were combined, concentrated *in vacuo* and purified by flash column chromatography on the Teledyne ISCO CombiFlash^{®} Rf+ (40 g silica column Puriflash HC, 0 - 5% MeOH in DCM), followed by flash column chromatography on the Teledyne ISCO CombiFlash^{®} Rf+ (12 g silica column Puriflash HC, 15 um, 0 - 100% EtOAc in cyclohexane). Product containing fractions were combined and concentrated *in vacuo,* the residue was dissolved in MeCN/water (1:1) and freeze dried to provide the title compound (51 mg, 33%). LCMS (Method 3): 4.54 min, 662.2 [M+H]⁺. ¹H NMR (400 MHz, DMSO-d₆) δ 15.53 (bs, 1H), 7.53 (d, 1H), 7.18 (t, 1H), 5.40-5.28 (m, 2H), 5.20 (m, 1H), 4.09-3.96 (m, 4H), 3.90 (dd, 1H), 3.56 (m, 1H), 3.18 (m, 1H), 2.92 (d, 1H), 2.88-2.78 (m, 2H), 2.75 (m, 1H), 2.68 (d, 1H), 2.30-2.10 (m, 2H), 2.02 (d, 1H), 1.93-1.76 (m, 2H), 1.75-1.61 (m, 2H), 1.61-1.37 (m, 3H), 1.30 (s, 3H), 0.73-0.24 (m, 4H).

### Example 2: (1S,2R)-2-((S)-5-chloro-8-((5-(difluoromethyl)-1-methyl-1H-1,2,3-triazol-4-yl)methoxy)-7-fluoro-1-((3-oxomorpholino)methyl)-1,2,3,4-tetrahydroisoquinoline-2-carbonyl)-1-methylcyclohexane-1-carboxylic acid

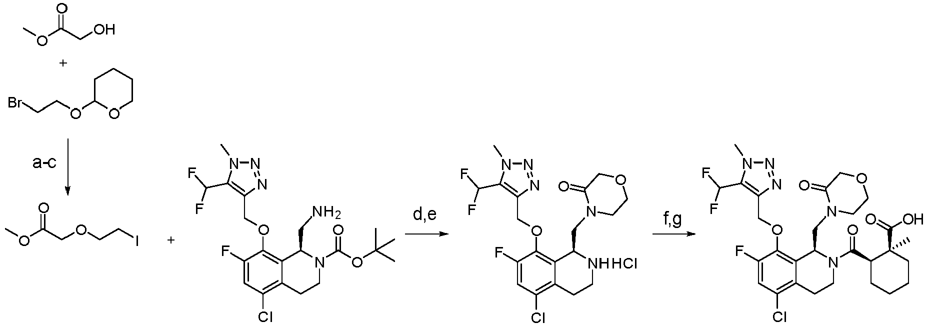

### Step a.

To a suspension of sodium hydride (60% dispersion in mineral oil; 1.33 g, 33.3 mmol; CAS: 7646-69-7) in DMF (10 mL) cooled to 0°C was added methyl 2-hydroxyacetate (2.14 mL, 27.8 mmol; CAS: 96-35-5) dropwise and the resulting mixture was stirred at rt under argon for 0.5 h. To this was added 2-(2-bromoethoxy)tetrahydropyran (4.61 mL, 30.5 mmol; CAS: 17739-45-6) and the resulting mixture was allowed to warm to rt and stirred for 4 h. To this was added water, the mixture was extracted with EtOAc and the combined organics were washed with water, brine, dried over Na₂SO₄, filtered and concentrated *in vacuo.* The crude product was purified by flash column chromatography on the Teledyne ISCO CombiFlash^{®} Rf+ (120 g silica column Puriflash HC/Biotage SNAP, 0 - 50% EtOAc in cyclohexane) to give methyl 2-(2-((tetrahydro-2H-pyran-2-yl)oxy)ethoxy)acetate (2.14 g, 35%). ¹H NMR (400 MHz; CDCl₃) δ 4.64 (dd, 1H), 4.20 - 4.19 (m, 2H), 3.93 - 3.83 (m, 2H), 3.78 - 3.75 (m, 5H), 3.68 - 3.62 (m, 1H), 3.54 - 3.48 (m, 1H), 1.90 - 1.50 (m, 6H).

### Step b.

To a stirred solution of the above intermediate (2.14 g, 9.81 mmol) in MeOH (20 mL) was added pyridinium p-toluenesulfonate (49 mg, 0.20 mmol; CAS: 24057-28-1) and the resulting mixture was heated under reflux for 2 h. The reaction mixture was allowed to cool to rt, concentrated *in vacuo* and purification by flash column chromatography on the Teledyne ISCO CombiFlash^{®} Rf+ (40 g silica column Puriflash HC, 0 - 100% EtOAc in cyclohexane) gave methyl 2-(2-hydroxyethoxy)acetate (1.12 g, 85%). ¹H NMR (400 MHz; CDCl₃) δ 4.16 (s, 2H), 3.78 - 3.74 (m, 5H), 3.71 - 3.68 (m, 2H), 2.71 (t, 1H).

### Step c.

To a stirred solution of the above intermediate (1.12 g, 8.4 mmol), imidazole (0.85 g, 12.5 mmol) and triphenylphosphine (2.85 g, 10.9 mmol) in Et₂O (4.2 mL) and MeCN (2.1 mL) cooled in an ice bath was added iodine (2.97 g, 11.7 mmol) and the resulting mixture was stirred at 0°C for 2 h. The reaction mixture was diluted with EtOAc, filtered and the filtrate washed with aqueous sodium sulfite solution and brine, dried over Na₂SO₄ and concentrated *in vacuo.* The crude product was purified by flash column chromatography on the Teledyne ISCO CombiFlash^{®} Rf+ (80 g silica column Puriflash HC, 0 - 50% EtOAc in cyclohexane) to give methyl 2-(2-iodoethoxy)acetate (1.39 g, 68%). ¹H NMR (400 MHz; CDCl₃) δ 4.16 (s, 2H), 3.83 (t, 2H), 3.77 (s, 3H), 3.30 (t, 2H).

### Step d.

To a stirred solution of *tert*-butyl (S)-1-(aminomethyl)-5-chloro-8-((5-(difluoromethyl)-1-methyl-1*H*-1 ,2, 3-triazol-4-yl)methoxy)-7 -fluoro-3,4-dihydroisoquinoline-2(1*H*)-carboxylate (290 mg, 0.61 mmol; Example 1, step b) in toluene (2.5 mL) was added the above intermediate (193 mg, 0.79 mmol) and triethylamine (0.13 mL, 0.910 mmol) and the reaction mixture was heated at 120°C for 18 h. The reaction mixture was cooled to rt and concentrated *in vacuo.* The residue was diluted with brine and extracted with DCM. The combined organics were washed with brine, dried over Na₂SO₄, concentrated and purified by flash column chromatography on the Teledyne ISCO CombiFlash^{®} Rf+ (25 g silica column Puriflash HC, 0 - 100% EtOAc in cyclohexane) to give *tert*-butyl (*S*)-5-chloro-8-((5-(difluoromethyl)-1-methyl-1*H*-1,2,3-triazol-4-yl)methoxy)-7-fluoro-1-((3-oxomorpholino)methyl)-3,4-dihydroisoquinoline-2(1H)-carboxylate (229 mg, 67%). LCMS (Method 1): 1.56 min, 582.2 [M+Na]⁺.

### Step e.

A solution of hydrochloric acid (4 M in dioxane; 2.04 mL, 8.18 mmol) was added to the above intermediate (229 mg, 0.410 mmol) and the resulting reaction mixture stirred at rt for 1h. The mixture was concentrated *in vacuo* and azeotroped with toluene to give (S)-4-((5-chloro-8-((5-(difluoromethyl)-1-methyl-1H-1,2,3-triazol-4-yl)methoxy)-7-fluoro-1,2,3,4-tetrahydroisoquinolin-1-yl)methyl)morpholin-3-one hydrochloride (219 mg, quantitative). LCMS (Method 1): 0.85 min, 460.1 [M+H]⁺.

### Step f.

To a stirred solution the above intermediate (203 mg, 0.41 mmol) and Intermediate 12 (279 mg, 0.61 mmol) in DMF (1 mL) was added DIPEA (0.14 mL, 0.82 mmol) and the reaction mixture was stirred at rt under argon for 6 days. The reaction mixture was diluted with saturated sodium bicarbonate solution, extracted with EtOAc and the combined organics were washed with water and brine, dried over Na₂SO₄, filtered and concentrated *in vacuo.* The crude product was purified by flash column chromatography on the Teledyne ISCO CombiFlash^{®} Rf+ (25 g silica column Puriflash HC, 0 - 100% EtOAc in cyclohexane) to provide 2,4-dimethoxybenzyl (1*S*,2*R*)-2-((*S*)-5-chloro-8-((5-(difluoromethyl)-1-methyl-1*H*-1,2,3-triazol-4-yl)methoxy)-7-fluoro-1-((3-oxomorpholino)methyl)-1,2,3,4-tetrahydroisoquinoline-2-carbonyl)-1-methylcyclohexane-1-carboxylate (173 mg, 54%). LCMS (Method 1): 1.70 min, 778.3 [M+H]⁺.

### Step g.

To a stirred solution of the above intermediate (173 mg, 0.22 mmol) in DCM (2 mL) was added triethylsilane (0.04 mL, 0.22 mmol), followed by TFA (0.02 mL, 0.22 mmol) and the resulting mixture was stirred at rt for 1h. The reaction mixture was concentrated *in vacuo* and the residue purified by flash column chromatography on the Teledyne ISCO CombiFlash^{®} Rf+ (12 g silica column Puriflash HC, 15 um, 0 - 5% MeOH in DCM). Fractions containing the desired product were combined and concentrated *in vacuo* and the residue was taken up in MeCN/water (1:1) and freeze dried to provide the title compound (94.3 mg, 67%). LCMS (Method 3): 4.45 min, 628.0 [M+H]⁺. ¹H NMR (400 MHz, DMSO-d₆) δ 11.88 (bs, 1H), 7.54 (d, 1H), 7.34 (t, 1H), 5.52 (dd, 1H), 5.44 (d, 1H), 5.25 (d, 1H), 4.31 (dd, 1H), 4.14 (s, 3H), 3.98 (d, 1H), 3.90 (m, 1H), 3.84-3.70 (m, 2H), 3.69-3.50 (m, 2H), 3.26 (m, 1H), 2.98-2.86 (m, 2H), 2.84-2.83 (m, 2H), 2.62 (m, 1H), 2.19 (m, 1H), 1.68-1.49 (m, 3H), 1.47-1.19 (m, 4H), 1.10 (s, 3H).

### Example 3: (1S,2R)-2-((S)-5-chloro-8-((5-(difluoromethyl)-1-methyl-1H-1,2,3-triazol-4-ylmethoxy)-7-fluoro-1-(((R)-4-methyl-2-oxopyrrolidin-1-yl)methyl)-1,2,3,4-tetrahydroisoquinoline-2-carbonyl)-1-methylcyclohexane-1-carboxylic acid

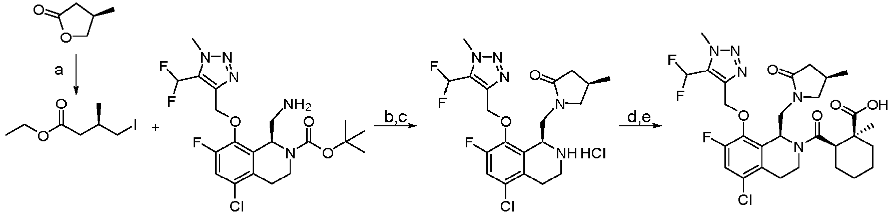

### Step a.

To a stirred solution of (R)-4-methyldihydrofuran-2(3*H*)-one (13.4 g, 134 mmol, CAS: 65284-00-6) in EtOH (250 mL) at -20 °C was added trimethylsilyl iodide (38.1 mL, 268 mmol) dropwise. The solution was stirred at -20 °C for 30 min. Triethyl orthoformate (22.3 mL, 134 mmol) was then added and the reaction stirred at reflux for 18 h. The reaction mixture was cooled to rt and concentrated *in vacuo.* The crude was product purified by flash column chromatography (silica, 5% EtOAc in heptane) to give ethyl (*R*)-4-iodo-3-methylbutanoate (24.3 g, 71%). ¹H NMR (300 MHz, CDCl₃) δ 4.15 (q, 2H), 3.32-3.23 (m, 2H), 2.46 (dd, 1H), 2.24 (dd, 1H), 2.07-1.99 (m, 1H), 1.30-1.25 (t, 3H), 1.06 (d, 3H).

### Step b.

To a stirred solution of *tert*-butyl (S)-1-(aminomethyl)-5-chloro-8-((5-(difluoromethyl)-1-methyl-1*H*-1 ,2, 3-triazol-4-yl)methoxy)-7 -fluoro-3,4-dihydroisoquinoline-2(1*H*)-carboxylate (145 mg, 0.30 mmol; Example 1, step b) in toluene (1.3 mL) was added the above intermediate (101 mg, 0.40 mmol) and triethylamine (0.06 mL, 0.46 mmol) and the reaction mixture was heated at 120°C for 18 h. The reaction mixture was allowed to cool to rt and concentrated *in vacuo.* The residue was diluted with brine, extracted with DCM and the combined organic phases were washed with brine, dried over Na₂SO₄ and concentrated *in vacuo.* The crude product was purified by flash column chromatography on the Teledyne ISCO CombiFlash^{®} Rf+ (25 g silica column Puriflash HC, 0 - 100% EtOAc in cyclohexane) to give *tert*-butyl (*S*)-5-chloro-8-((5-(difluoromethyl)-1-methyl-1H-1,2,3-triazol-4-yl)methoxy)-7-fluoro-1-(((*R*)-4-methyl-2-oxopyrrolidin-1-yl)methyl)-3,4-dihydroisoquinoline-2(1*H*)-carboxylate (89 mg, 52%). LCMS (Method 1): 1.63 min, 580.2 [M+Na]⁺.

### Step c.

Hydrochloric acid (4 M in Dioxane; 0.8 mL, 3.2 mmol) was added to the above intermediate (89 mg, 0.16 mmol) and the resulting reaction mixture left to stir at rt for 1 h. The reaction mixture was concentrated *in vacuo* and azeotroped with toluene to give *(R)-1-(((S)-5-chloro-8-((5-(difluoromethyl)-1-methyl-1H-1,2,3-triazol-4-yl)methoxy)-7-*fluoro-1,2,3,4-tetrahydroisoquinolin-1-yl)methyl)-4-methylpyrrolidin-2-one hydrochloride (83 mg, quantitative). LCMS (Method 1): 0.93 min, 458.1 [M+H]⁺.

### Step d.

To a stirred solution of the above intermediate (79 mg, 0.16 mmol) in DMF (0.5 mL) was added Intermediate 12 (109 mg, 0.24 mmol) and DIPEA (0.06 mL, 0.32 mmol) and the resulting mixture stirred at rt under argon for 6 days. The reaction mixture was diluted with saturated sodium bicarbonate solution, extracted with EtOAc and the combined organics were washed with water and brine, dried over Na₂SO₄, filtered and concentrated *in vacuo.* Purification by flash column chromatography on the Teledyne ISCO CombiFlash^{®} Rf+ (12 g silica column Puriflash HC, 0 - 100% EtOAc in cyclohexane) gave 2,4-dimethoxybenzyl (1*S*,2*R*)-2-((*S*)-5-chloro-8-((5-(difluoromethyl)-1-methyl-1*H*-1,2,3-triazol-4-yl)methoxy)-7-fluoro-1-(((*R*)-4-methyl-2-oxopyrrolidin-1-yl)methyl)-1,2,3,4-tetrahydroisoquinoline-2-carbonyl)-1-methylcyclohexane-1-carboxylate (44 mg, 36%). LCMS (Method 1): 1.75 min, 776.3 [M+H]⁺.

### Step e.

To a stirred solution of the above intermediate (44 mg, 0.060 mmol) in DCM (0.5 mL) was added triethylsilane (0.01 mL, 0.06 mmol), followed by TFA (0.004 mL, 0.06 mmol) and the reaction mixture was stirred at rt for 1 h and concentrated *in vacuo.* Purification by flash column chromatography on the Teledyne ISCO CombiFlash^{®} Rf+ (12 g silica column Puriflash HC, 15 um, 0 - 5% MeOH in DCM). Fractions containing the desired product were combined and concentrated *in vacuo.* The residue was taken up in acetonitrile/water (1:1) and freeze dried to provide the title compound (26 mg, 71%). LCMS (Method 3): 4.61 min, 626.0 [M+H]⁺. ¹H NMR (400 MHz, DMSO-d₆) δ 11.97 (bs, 1H), 7.52 (d, 1H), 7.35 (t, 1H), 5.48-5.38 (m, 2H), 5.21 (d, 1H), 4.14 (s, 3H), 3.98-3.80 (m, 2H), 3.56 (m, 1H), 3.02 (m, 1H), 2.95-2.71 (m, 4H), 2.70-2.57 (m, 1H), 2.37-2.22 (m, 2H), 2.16 (m, 1H), 1.73-1.50 (m, 4H), 1.48-1.15 (m, 4H), 1.08 (s, 3H), 0.98 (d, 3H).

### Example 4: (1S,2R)-2-((S)-5-chloro-7-fluoro-1-((6-oxo-5-azaspiro[2.4]heptan-5-yl)methyl)-8-((4,5,6,7-tetrahydro-[1,2,3]triazolo[1,5-a]pyridin-3-yl)methoxy)-1,2,3,4-tetrahydroisoquinoline-2-carbonyl)-1-methylcyclopentane-1-carboxylic acid

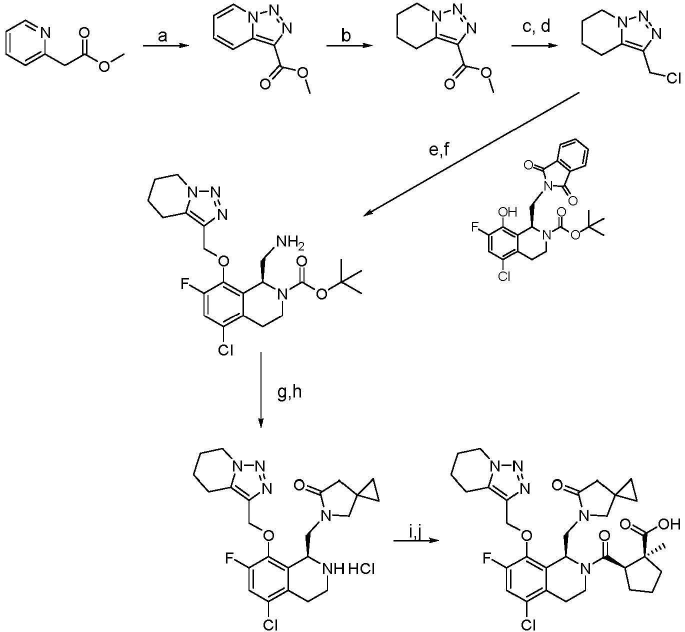

### Step a.

To a stirred solution of methyl 2-(pyridin-2-yl)acetate (5.0 g, 33.1 mmol, CAS: 1658-42-0) in MeCN (83 mL) was added 4-acetamidobenzenesulfonyl azide (7.95 g, 33.1 mmol, CAS: 2158-14-7) and the reaction mixture was cooled to 0°C under argon. To this was added DBU (3.97 mL, 33.1 mmol) dropwise over 10 min. The reaction mixture was allowed to warm to rt and stirred 3 h. To this was added of saturated ammonium chloride solution and the mixture extracted with EtOAc. The combined organics were washed with brine, dried over Na₂SO₄, filtered and concentrated *in vacuo.* Purification by flash column chromatography on the Interchim 4125 (120 g silica column Puriflash HP, 0 - 15% EtOAc in DCM) gave methyl [1,2,3]triazolo[1,5-a]pyridine-3-carboxylate (5.48 g, 89%). ¹H NMR (400 MHz, CDCl₃) δ 8.86 - 8.83 (m, 1H), 8.31 - 8.28 (m, 1H), 7.56 (ddd, 1H), 7.19 - 7.15 (m, 1H), 4.06 (s, 3H).

### Step b.

To a stirred solution of the above intermediate (2.0 g, 11.3 mmol) in EtOH (455 mL) was added Pd/C (10%; 1.20 g, 11.3 mmol) and the reaction mixture was degassed and backfilled with hydrogen (x 3). The reaction mixture was stirred under a hydrogen atmosphere at atmospheric pressure for 3h. The mixture was filtered through Celite^{®} and concentrated *in vacuo* to provide methyl 4,5,6,7-tetrahydro-[1,2,3]triazolo[1,5-a]pyridine-3-carboxylate (1.98 g, 99%). ¹H NMR (400 MHz, CDCl₃) δ: 4.41 (t, 2H), 3.94 (s, 3H), 3.11 (t, 2H), 2.13 - 2.06 (m, 2H), 1.98 - 1.91 (m, 2H).

### Step c.

To a stirred suspension of the above intermediate (1.96 g, 11.1 mmol) in THF (88 mL) was added lithium borohydride (2M in THF; 10.0 mL, 19.9 mmol) dropwise over 10 min. The reaction was stirred at rt under argon for 20h. The reaction mixture was cooled to 0°C and diluted with 10% citric acid (33 mL) then extracted with DCM. The combined organics were washed with water and concentrated *in vacuo.* The crude product was purified by flash column chromatography on the Interchim Puriflash^{®} 4100 (80 g silica column Puriflash HP, 0 - 30% EtOAc in DCM) to give (4,5,6,7-tetrahydro-[1,2,3]triazolo[1,5-a]pyridin-3-yl)methanol (620 mg, 35%). ¹H NMR (400 MHz, CDCl₃) δ 4.76 (s, 2H), 4.38 (t, 2H), 2.92 - 2.88 (m, 3H), 2.17 - 2.10 (m, 2H), 2.05 - 1.95 (m, 2H).

### Step d.

Thionyl chloride (0.59 mL, 8.1 mmol) was added to the above intermediate (620 mg, 4.1 mmol). Chloroform (6.5 mL) was added and the mixture stirred at rt for 16 h. The reaction mixture was concentrated *in vacuo,* azeotroped with toluene (x 2) and concentrated *in vacuo* to provide 3-(chloromethyl)-4,5,6,7-tetrahydrotriazolo[1,5-a]pyridine (702 mg, assumed quantitative) used without further purification. LCMS (Method 1): 0.90 min, 171.9 [M+H]⁺.

### Step e.

To a stirred solution of Intermediate 8 (434 mg, 0.94 mmol) in DMF (3 mL) was added the above intermediate (210 mg, 1.22 mmol) and cesium carbonate (920 mg, 2.82 mmol) and the reaction mixture was stirred under nitrogen at rt for 18 h. The reaction mixture was diluted with water, extracted with EtOAc and the combined organics were washed with water, brine, dried over Na₂SO₄, filtered and concentrated *in vacuo.* Purification by flash column chromatography on the Teledyne ISCO CombiFlash^{®} Rf+ (40 g silica column Puriflash HC, 0 - 100% EtOAc in DCM) gave *tert*-butyl (S)-5-chloro-1-((1,3-dioxoisoindolin-2-yl)methyl)-7-fluoro-8-((4,5,6,7-tetrahydro-[1,2,3]triazolo[1,5-*a*]pyridin-3-yl)methoxy)-3,4-dihydroisoquinoline-2(1*H*)-carboxylate (425 mg, 76%). LCMS (Method 1): 1.76 min, 596.2 [M+H]⁺.

### Step f.

To a stirred solution of the above intermediate (425 mg, 0.71 mmol) in EtOH (3.6 mL) was added hydrazine monohydrate (0.13 mL, 1.78 mmol; CAS: 7803-57-8) and the reaction mixture was heated at 75°C for 1.5 h. The reaction mixture was allowed to cool to rt, diluted with cold MeCN, filtered and the filtrate concentrated *in vacuo* to give *tert-*butyl (S)-1-(aminomethyl)-5-chloro-7-fluoro-8-((4,5,6,7-tetrahydro-[1,2,3]triazolo[1,5-a]pyridin-3-yl)methoxy)-3,4-dihydroisoquinoline-2(1*H*)-carboxylate (298 mg, 85%). LCMS (Method 1): 1.07 min, 466.2 [M+H]⁺.

### Step g.

To a stirred solution of the above intermediate (298 mg, 0.64 mmol) in toluene (3 mL) was added methyl 2-[1-(bromomethyl)cyclopropyl]acetate (146 mg, 0.70 mmol; CAS: 855473-50-6) and triethylamine (0.13 mL, 0.96 mmol) and the mixture was heated under reflux for 16 h. The reaction mixture was allowed to cool to rt and concentrated *in vacuo.* The residue was diluted with brine, extracted with DCM and the combined organics were washed with brine, dried over Na₂SO₄ and concentrated *in vacuo.* Purification by flash column chromatography on the Teledyne ISCO CombiFlash^{®} Rf+ (40 g silica column Puriflash HC, 0 - 90% EtOAc in DCM) gave tert-butyl (S)-5-chloro-7-fluoro-1-((6-oxo-5-azaspiro[2.4]heptan-5-yl)methyl)-8-((4,5,6,7-tetrahydro-[1,2,3]triazolo[1,5-a]pyridin-3-yl)methoxy)-3,4-dihydroisoquinoline-2(1H)-carboxylate (321 mg, 90%). LCMS (Method 1): 1.66 min, 582.3 [M+Na]⁺.

### Step h.

Hydrochloric acid (4M in dioxane; 2.9 mL, 11.5 mmol) was added to the above intermediate (321 mg, 0.57 mmol) and the resulting reaction mixture left to stir at rt for 1 h. The reaction mixture was concentrated *in vacuo* and azeotroped with toluene to give (S)-5-((5-chloro-7-fluoro-8-((4,5,6,7-tetrahydro-[1,2,3]triazolo[1,5-a]pyridin-3-yl)methoxy)-1,2,3,4-tetrahydroisoquinolin-1-yl)methyl)-5-azaspiro[2.4]heptan-6-one hydrochloride (294 mg, 98%). LCMS (Method 1): 1.01 min, 460.2 [M+H]⁺.

### Step i.

To a stirred solution of the above intermediate (135 mg, 0.27 mmol) and Intermediate 16 (204 mg, 0.47 mmol) in DMF (0.4 mL) was added DIPEA (0.1 mL, 0.60 mmol) and the reaction mixture was stirred at rt under argon for 18 h. The reaction mixture was diluted with saturated sodium bicarbonate solution, extracted with EtOAc and the combined organics were washed with water and brine, dried over Na₂SO₄, filtered and concentrated *in vacuo.* Purification by flash column chromatography on the Teledyne ISCO CombiFlash^{®} Rf+ (40 g silica column Puriflash HC, 0 - 100% EtOAc in DCM) gave 2,4-dimethoxybenzyl (1*S*,2*R*)-2-((*S*)-5-chloro-7-fluoro-1-((6-oxo-5-azaspiro[2.4]heptan-5-yl)methyl)-8-((4,5,6,7-tetrahydro-[1,2,3]triazolo[1,5-a]pyridin-3-yl)methoxy)-1,2,3,4-tetrahydroisoquinoline-2-carbonyl)-1-methylcyclopentane-1-carboxylate (154 mg, 74%). LCMS (Method 1): 1.68 min, 764.4 [M+H]⁺.

### Step j.

To a stirred solution of the above intermediate (154 mg, 0.20 mmol) in DCM (2.0 mL) at 0°C was added triethylsilane (0.14 mL, 0.89 mmol), followed by TFA (0.03 mL, 0.45 mmol) and the resulting mixture was stirred at rt for 1h 15. Further triethylsilane (0.04 mL, 0.22 mmol) and TFA (0.02 mL, 0.22 mmol) were added and stirring continued for an additional 45 mins at rt. The reaction mixture was concentrated *in vacuo* and the residue azeotroped with toluene (x 2). Purification by flash column chromatography on the Interchim Puriflash^{®} 4125 (40g 15 µm silica column Puriflash HP, 0 - 5% MeOH in DCM) gave the title compound (57 mg, 43%). LCMS (Method 3): 4.33 min, 614.1 [M+H]⁺. ¹H NMR (400 MHz, CDCl₃) δ 7.11 (d, 1H), 5.60 (dd, 1H), 5.23 (m, 1H), 5.15 (m, 1H), 4.42 (m, 1H), 4.31 (m, 1H), 4.01 (dd, 1H), 3.84 (m, 2H), 3.35 (d, 1H), 3.13-3.01 (m, 2H), 2.94 (m, 1H), 2.87-2.65 (m, 5H), 2.55 (m, 1H), 2.34 (d, 1H), 2.21-2.00 (m, 4H), 1.98-1.65 (m, 4H), 1.44 (m, 1H), 1.28 (s, 3H), 0.66-0.50 (m, 4H).

### Example 5: (1S,2R)-2-((S)-5-chloro-8-((4,5-dimethyl-4H-1,2,4-triazol-3-yl)methoxy)-7-fluoro-1-((6-oxo-5-azaspiro[2.4]heptan-5-yl)methyl)-1,2,3,4-tetrahydroisoquinoline-2-carbonyl)-1-methylcyclohexane-1-carboxylic acid

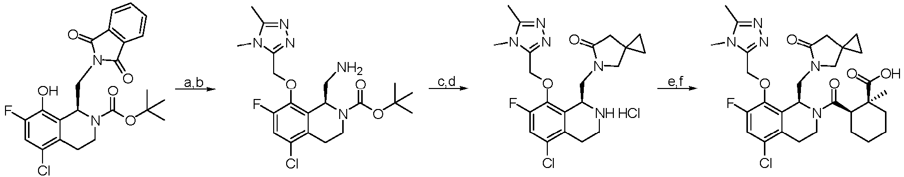

### Step a.

To a stirred solution of Intermediate 8 (300 mg, 0.65 mmol) in DMF (1.2 mL) was added 3-(chloromethyl)-4,5-dimethyl-1,2,4-triazole (123 mg, 0.85 mmol; CAS: 881845-16-5) and cesium carbonate (636 mg, 1.95 mmol) and the reaction mixture was stirred under nitrogen at rt for 4 h. Further 3-(chloromethyl)-4,5-dimethyl-1,2,4-triazole (19 mg, 0.13 mmol) and cesium carbonate (106 mg, 0.33 mmol) were added and the resulting mixture stirred for a further 1 h. The reaction mixture was diluted with water, extracted with EtOAc and the combined organics were washed with water, brine, dried over Na₂SO₄, filtered and concentrated *in vacuo.* Purification by flash column chromatography on the Teledyne ISCO CombiFlash^{®} Rf+ (25 g silica column Puriflash HC, 0 - 5% MeOH in DCM) gave *tert-*butyl (S)-5-chloro-8-((4,5-dimethyl-4*H*-1,2,4-triazol-3-yl)methoxy)-1-((1,3-dioxoisoindolin-2-yl)methyl)-7-fluoro-3,4-dihydroisoquinoline-2(1*H*)-carboxylate (331 mg, 89%). LCMS (Method 1): 1.47 min, 570.2 [M+H]⁺.

### Step b.

To a stirred solution of the above intermediate (331 mg, 0.58 mmol) in EtOH (3 mL) was added hydrazine monohydrate (0.11 mL, 1.45 mmol; CAS: 7803-57-8) and the resulting mixture was heated at 75°C for 1 h. The reaction mixture was allowed to cool to rt, diluted with cold MeCN, filtered and the filtrate concentrated *in vacuo* to give tert-butyl (S)-1-(aminomethyl)-5-chloro-8-((4,5-dimethyl-4*H*-1,2,4-triazol-3-yl)methoxy)-7-fluoro-3,4-dihydroisoquinoline-2(1*H*)-carboxylate (229 mg, 90%). LCMS (Method 1): 1.00 min, 440.1 [M+H]⁺.

### Step c.

To a stirred solution of the above intermediate (229 mg, 0.52 mmol) in toluene (2.1 mL) was added methyl 2-[1-(bromomethyl)cyclopropyl]acetate (119 mg, 0.57 mmol; CAS: 855473-50-6) and triethylamine (0.11 mL, 0.780 mmol) and the reaction mixture was heated under reflux for 16 h. The reaction mixture was allowed to cool to rt and concentrated *in vacuo.* The residue was diluted with brine, extracted with DCM the combined organics were washed with brine, dried over Na₂SO₄ and concentrated *in vacuo.* Purification by flash column chromatography on the Teledyne ISCO CombiFlash^{®} Rf+ (25 g silica column Puriflash HC, 0 - 5% MeOH in DCM) gave *tert*-butyl (*S*)-5-chloro-8-((4,5-dimethyl-4*H*-1,2,4-triazol-3-yl)methoxy)-7-fluoro-1-((6-oxo-5-azaspiro[2.4]heptan-5-yl)methyl)-3,4-dihydroisoquinoline-2(1*H*)-carboxylate (95 mg, 34%). LCMS (Method 1): 1.36 min, 534.2 [M+H]⁺.

### Step d.

Hydrochloric acid (4 M in dioxane; 0.89 mL, 3.56 mmol) was added to the above intermediate (95 mg, 0.18 mmol) and the resulting reaction mixture left to stir at rt for 2 h. The reaction mixture was concentrated *in vacuo* and azeotroped with toluene to give (S)-5-((5-chloro-8-((4,5-dimethyl-4*H*-1,2,4-triazol-3-yl)methoxy)-7-fluoro-1,2,3,4-tetrahydroisoquinolin-1-yl)methyl)-5-azaspiro[2.4]heptan-6-one hydrochloride (110 mg, assumed quantitative). LCMS (Method 2): 1.90 min, 434.3 [M+H]⁺.

### Step e.

To a stirred solution of the above intermediate (84 mg, 0.18 mmol) and Intermediate 12 (121 mg, 0.27 mmol) in DMF (0.5 mL) was added DIPEA (0.06 mL, 0.36 mmol) and the resulting mixture was stirred at rt under argon for 3 days. The reaction mixture was diluted with saturated sodium bicarbonate solution, extracted with EtOAc and the combined organics washed with water and brine, dried over Na₂SO₄, filtered and concentrated *in vacuo.* The crude product was purified by flash column chromatography on the Teledyne ISCO CombiFlash^{®} Rf+ (25 g silica column Puriflash HC, 0 - 5% MeOH in DCM) to give 2,4-dimethoxybenzyl (1*S*,2*R*)-2-((*S*)-5-chloro-8-((4,5-dimethyl-4H-1,2,4-triazol-3-yl)methoxy)-7-fluoro-1-((6-oxo-5-azaspiro[2.4]heptan-5-yl)methyl)-1,2,3,4-tetrahydroisoquinoline-2-carbonyl)-1-methylcyclohexane-1-carboxylate (72 mg, 54%). LCMS (Method 1): 1.51 min, 752.3 [M+H]⁺.

### Step f.

To a stirred solution of the above intermediate (72 mg, 0.10 mmol) in DCM (0.9 mL) was added triethylsilane (0.02 mL, 0.10 mmol), followed by TFA (0.01 mL, 0.10 mmol) and the reaction mixture stirred at rt for 1 h. The reaction mixture was concentrated *in vacuo* and the residue purified by flash column chromatography on the Teledyne ISCO CombiFlash^{®} Rf+ (25 g silica column Puriflash HC, 15 um, 0 - 5% MeOH in DCM). Fractions containing the desired product were combined and concentrated *in vacuo.* The residue was taken up in MeCN/water (1:1) and freeze dried to provide the title compound (24 mg, 41%). LCMS (Method 3): 3.78 min, 602.0 [M+H]⁺. ¹H NMR (400 MHz, DMSO-d₆) δ 11.84 (bs, 1H), 7.55 (d, 1H), 5.61 (dd, 1H), 5.38-5.26 (m, 2H), 4.03-3.88 (m, 2H), 3.57 (m, 1H), 3.51 (s, 3H), 3.06 (d, 1H), 3.00 (m, 1H), 2.87 (dd, 1H), 2.80 (dd, 1H), 2.72-2.59 (m, 2H), 2.40-2.24 (m, 4H), 2.21 (d, 1H), 2.07 (d, 1H), 1.72-1.48 (m, 3H), 1.47-1.17 (m, 4H), 1.11 (s, 3H), 0.63-0.43 (m, 4H).

### Example 6: (1S,2R)-2-((S)-5-chloro-7-fluoro-8-((1-methyl-1H-1,2,3-triazol-4-yl)methoxy)-1-((6-oxo-5-azaspiro[2.4]heptan-5-yl)methyl)-1,2,3,4-tetrahydroisoquinoline-2-carbonyl)-1-methylcyclohexane-1-carboxylic acid

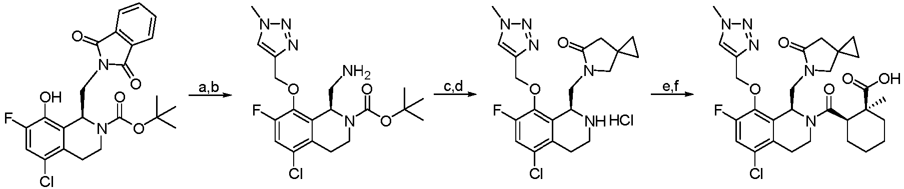

### Step a.

To a stirred solution of Intermediate 8 (300 mg, 0.65 mmol) in DMF (1.2 mL) was added 4-(chloromethyl)-1-methyl-triazole (111 mg, 0.85 mmol; CAS: 269726-46-7) and cesium carbonate (636 mg, 1.95 mmol) and the reaction mixture was stirred under nitrogen at rt for 18 h. The reaction mixture was diluted with water, extracted with EtOAc and the combined organics washed with water, brine, dried over Na₂SO₄, filtered and concentrated *in vacuo.* The crude product was purified by flash column chromatography on the Teledyne ISCO CombiFlash^{®} Rf+ (25 g silica column Puriflash HC, 0 - 100% EtOAc in cyclohexane) to provide *tert*-butyl (S)-5-chloro-1-((1,3-dioxoisoindolin-2-yl)methyl)-7-fluoro-8-((1-methyl-1*H*-1,2,3-triazol-4-yl)methoxy)-3,4-dihydroisoquinoline-2(1*H*)-carboxylate (332 mg, 92%). LCMS (Method 1): 1.68 min, 578.2 [M+Na]⁺.

### Step b.

To a stirred solution of the above intermediate (332 mg, 0.60 mmol) in ethanol (3 mL) was added hydrazine monohydrate (0.11 mL, 1.49 mmol; CAS: 7803-57-8) and the resulting mixture was heated at 75°C for 1 h. The reaction mixture was allowed to cool to rt, diluted with cold MeCN, filtered and the filtrate concentrated *in vacuo* to give *tert-butyl* (S)-1-(aminomethyl)-5-chloro-7-fluoro-8-((1-methyl-1*H*-1,2,3-triazol-4-yl)methoxy)-3,4-dihydroisoquinoline-2(1*H*)-carboxylate (264 mg, assumed quantitative). LCMS (Method 1): 1.05 min, 426.1 [M+H]⁺.

### Step c.

To a stirred solution of the above intermediate (254 mg, 0.60 mmol) in toluene (2.4 mL) was added methyl 2-[1-(bromomethyl)cyclopropyl]acetate (136 mg, 0.66 mmol; CAS: 855473-50-6) and triethylamine (0.12 mL, 0.89 mmol) and the reaction mixture was heated under reflux for 16 h. The reaction mixture was allowed to cool to rt and concentrated *in vacuo.* The residue was diluted with brine, extracted with DCM and the combined organics washed with brine, dried over Na₂SO₄ and concentrated *in vacuo.* The crude product was purified by flash column chromatography on the Teledyne ISCO CombiFlash^{®} Rf+ (25 g silica column Puriflash HC, 0 - 100% EtOAc in cyclohexane) to give *tert-butyl (S)-5-chloro-7-fluoro-8-((1-methyl-1H-1,2,3-triazol-4-yl)methoxy)-1-((6-oxo-*5-azaspiro[2.4]heptan-5-yl)methyl)-3,4-dihydroisoquinoline-2(1H)-carboxylate (199 mg, 64%). LCMS (Method 1): 1.54 min, 542.2 [M+Na]⁺.

### Step d.

Hydrochloric acid (4M in dioxane; 1.9 mL, 7.65 mmol) was added to the above intermediate (199 mg, 0.38 mmol) and the resulting reaction mixture stirred at rt for 2 h. The reaction mixture was concentrated *in vacuo* and azeotroped with toluene to give (*S*)-5-((5-chloro-7-fluoro-8-((1-methyl-1*H*-1,2,3-triazol-4-yl)methoxy)-1,2,3,4-tetrahydroisoquinolin-1-yl)methyl)-5-azaspiro[2.4]heptan-6-one hydrochloride (217 mg, assumed quantitative). LCMS (Method 1): 0.90 min, 420.1 [M+H]⁺.

### Step e.

To a stirred solution of the above intermediate (175 mg, 0.38 mmol) and Intermediate 12 (261 mg, 0.58 mmol) in DMF (0.5 mL) was added DIPEA (0.13 mL, 0.77 mmol) and the reaction mixture was stirred at rt under argon for 3 days. The reaction mixture was diluted with saturated sodium bicarbonate solution, extracted with EtOAc and the combined organics were washed with water and brine, dried over Na₂SO₄, filtered and concentrated *in vacuo.* The crude product was purified by flash column chromatography on the Teledyne ISCO CombiFlash^{®} Rf+ (25 g silica column Puriflash HC, 0 - 100% EtOAc in cyclohexane) to give 2,4-dimethoxybenzyl (1S,2R)-2-((S)-5-chloro-7-fluoro-8-((1-methyl-1*H*-1,2,3-triazol-4-yl)methoxy)-1-((6-oxo-5-azaspiro[2.4]heptan-5-yl)methyl)-1,2,3,4-tetrahydroisoquinoline-2-carbonyl)-1-methylcyclohexane-1-carboxylate (177 mg, 63%). LCMS (Method 1): 1.66 min, 738.3 [M+H]⁺.

### Step f.

To a stirred solution of the above intermediate (177 mg, 0.24 mmol) in DCM (2.2 mL) was added triethylsilane (0.04 mL, 0.24 mmol), followed by TFA (0.02 mL, 0.24 mmol) and the reaction mixture was stirred at rt for 1 h. The reaction mixture was concentrated *in vacuo* and purified by flash column chromatography on the Teledyne ISCO CombiFlash^{®} Rf+ (25 g silica column Puriflash HC, 15 um, 0 - 5% MeOH in DCM). Fractions containing the desired product were combined and concentrated *in vacuo* to give the title compound (94 mg, 67%). 44 mg of product was dissolved in MeCN/water (1:1) and freeze dried to provide the title compound (37 mg, 25%). LCMS (Method 3): 4.26 min, 588.0 [M+H]⁺. ¹H NMR (400 MHz, DMSO-d₆) δ 11.91 (bs, 1H), 8.18 (s, 1H), 7.50 (d, 1H), 5.68 (dd, 1H), 5.30 (m, 1H), 5.22 (m, 1H), 4.03 (s, 3H), 4.00-3.88 (m, 2H), 3.57 (m, 1H), 3.28 (d, 1H), 3.05-2.95 (m, 2H), 2.84-2.71 (m, 2H), 2.64 (m, 1H), 2.35-2.16 (m, 2H), 2.10 (d, 1H), 1.73-1.48 (m, 3H), 1.47-1.19 (m, 4H), 1.10 (s, 3H), 0.65-0.44 (m, 4H).

### Reference Example 7: (1S,2R)-2-((S)-5-chloro-7-fluoro-8-((5-methyl-1H-1,2,3-triazol-4-yl)methoxy)-1-((6-oxo-5-azaspiro[2.4]heptan-5-yl)methyl)-1,2,3,4-tetrahydroisoquinoline-2-carbonyl)-1-methylcyclohexane-1-carboxylic acid

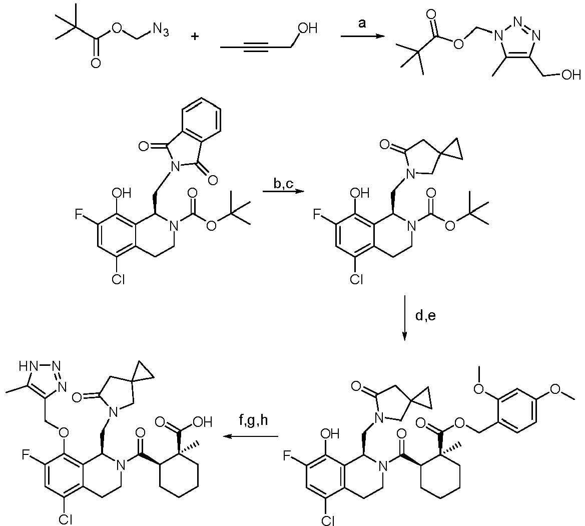

### Step a.

A mixture of azidomethyl 2,2-dimethylpropanoate (2.36 g, 15 mmol; CAS: 872700-68-0), and 2-butyn-1-ol (1.05 g, 15 mmol; CAS: 764-01-2) were heated in a sealed vial under argon at 110°C for 12 h. The mixture was cooled to rt, and purified by flash column chromatography (silica, 0 - 80% EtOAc in cyclohexane) to give (4-(hydroxymethyl)-5-methyl-1H-1,2,3-triazol-1-yl)methyl pivalate (1.5 g, 44%). LCMS (Method 1): 1.05 min, 228.1 [M+H]⁺.

### Step b.

To a stirred suspension of Intermediate 8 (5 g, 10.9 mmol) in EtOH (100 mL) was added hydrazine monohydrate (2.03 mL, 27.12 mmol; CAS: 7803-57-8) and the resulting mixture was heated at 85°C for 1 h. The reaction mixture was allowed to cool to rt, diluted with cold MeCN, filtered and the filtrate concentrated *in vacuo* to give tert-butyl (S)-1-(aminomethyl)-5-chloro-7-fluoro-8-hydroxy-3,4-dihydroisoquinoline-2(1H)-carboxylate (3.7 g, 95%). LCMS (Method 2): 2.17 min, 331.2 [M+H]⁺

### Step c.

To a stirred solution of the above intermediate (3.4 g, 10.8 mmol) in toluene (75 mL) was added methyl 1-(bromomethyl)cyclopropaneacetate (2.6 g, 12.3 mmol; CAS: 855473-50-6 ) and triethylamine (2.6 mL,18.5 mmol) in toluene (75 mL) and heated under reflux for 16 h. The reaction mixture was cooled to rt, concentrated *in vacuo* and the residue was partitioned between EtOAc and water. The organic phase was separated, and the aqueous layer was further extracted with DCM. The combined organics were dried (Na₂SO₄), filtered and concentrated *in vacuo.* The crude product was purified by flash column chromatography (silica, 0 - 50% EtOAc in DCM) to give tert-butyl (S)-5-chloro-7-fluoro-8-hydroxy-1-((6-oxo-5-azaspiro[2.4]heptan-5-yl)methyl)-3,4-dihydroisoquinoline-2(1H)-carboxylate (2.6 g, 60%). LCMS (Method 1) 1.61 min, 447.1 [M+Na]⁺

### Step d.

To a suspension of the above intermediate (1 g, 2.4 mmol) in 1,4-dioxane (2 mL) was added HCl in dioxane (4 M; 5.6 mL, 23.5 mmol) and the mixture was stirred at rt for 2 h. The mixture was filtered, and the solids azeoptroped with MeOH, then MeOH/toluene to give (*S*)-5-((5-chloro-7-fluoro-8-hydroxy-1,2,3,4-tetrahydroisoquinolin-1-yl)methyl)-5-azaspiro[2.4]heptan-6-one hydrochloride (0.85 g, quantitative). LCMS (Method 1): 0.83 min, 324.9 [M+H]⁺.

### Step e.

To a stirred solution of the above intermediate (0.85 g, 2.35 mmol) and Intermediate 12 (1.6 g, 3.53 mmol) in DMF (6 mL) was added DIPEA (1 mL, 5.88 mmol) and the resulting mixture was stirred at rt for 24 h. Additional portions of Intermediate 12 (1 g, 2.2 mmol) and DIPEA (1 mL, 5.88 mmol) were added and the mixture was stirred at rt for 3 days. The reaction mixture was diluted with EtOAc and saturated citric acid solution and the mixture extracted with EtOAc. The combined organics were washed with water and brine, dried over Na₂SO₄, filtered and concentrated *in vacuo.* The crude product was purified by flash column chromatography on the Biotage Isolera Four^{™} (40 g silica column Puriflash HC, 0 - 100% EtOAc in cyclohexane) to give 2,4-dimethoxybenzyl (1*S*,2*R*)-2-((*S*)-5-chloro-7-fluoro-8-hydroxy-1-((6-oxo-5-azaspiro[2.4]heptan-5-yl)methyl)-1,2,3,4-tetrahydroisoquinoline-2-carbonyl)-1-methylcyclohexane-1-carboxylate (0.59 g, 39%). LCMS (Method 1): 1.68 min, 643.2 [M+H]⁺

### Step f.

To a solution of the above intermediate (0.58 g, 0.9 mmol), (4-(hydroxymethyl)-5-methyl-1H-1,2,3-triazol-1-yl)methyl pivalate (0.23 g, 0.99 mmol, Example 7, Step a) and triphenylphosphine (0.31 g, 1.17 mmol) in THF (5 mL) was added DEAD (0.21 mL, 1.35 mmol) dropwise. The mixture was stirred for at rt 1 h then concentrated *in vacuo.* The crude product was purified by flash column chromatography on the Biotage Isolera Four^{™} (40 g silica column Puriflash HC 50 µM, 0 - 50% EtOAc in cyclohexane) then further purified by flash column chromatography on the Biotage Isolera Four^{™} (40 g silica column Puriflash HC 50 µM, 0 - 40% EtOAc in cyclohexane) to give 2,4-dimethoxybenzyl (1*S*,2*R*)-2-((S)-5-chloro-7-fluoro-8-((5-methyl-1-((pivaloyloxy)methyl)-1*H*-1,2,3-triazol-4-yl)methoxy)-1-((6-oxo-5-azaspiro[2.4]heptan-5-yl)methyl)-1,2,3,4-tetrahydroisoquinoline-2-carbonyl)-1-methylcyclohexane-1-carboxylate (0.56 g, 73%). LCMS (Method 1) 1.93 min, 852.3 [M+H]⁺.

### Step g.

To a solution of the above intermediate (0.55 g, 0.65 mmol) in DCM (3.6 mL) was added triethylsilane (0.52 mL, 3.23 mmol), followed by TFA (0.2 mL, 2.6 mmol) and the resulting mixture was stirred at rt for 1 h. The mixture was diluted with water and DCM, and the phases separated. The organics were washed with water, the filtered through a phase separator and concentrated *in vacuo.* The crude product was purified by flash column chromatography on the Biotage Isolera Four^{™} (40 g silica column Puriflash HC 50 µM, 0 - 5% MeOH in DCM) to give (1*S*,2*R*)-2-((*S*)-5-chloro-7-fluoro-8-((5-methyl-1-((pivaloyloxy)methyl)-1*H*-1,2,3-triazol-4-yl)methoxy)-1-((6-oxo-5-azaspiro[2.4]heptan-5-yl)methyl)-1,2,3,4-tetrahydroisoquinoline-2-carbonyl)-1-methylcyclohexane-1-carboxylic acid (0.35 g, 77%). LCMS (Method 1): 1.70 min, 702.3 [M+H]⁺.

### Step h.

To a solution of the above intermediate (0.35 g, 0.49 mmol) in THF (4mL) was added a solution of lithium hydroxide monohydrate (82.4 mg, 1.97 mmol) in water (0.5 mL) and the resulting solution was stirred at rt for 1 h. Ammonia (aqueous 30-33 wt%; 0.5 mL) was added and the mixture was to stirred at rt for 18 h. The solution was diluted with EtOAc and water then acidified to pH 5 by dropwise addition of HCl (1 M ). The mixture was extracted with EtOAc. The combined organics were washed with brine, dried over Na₂SO₄ and concentrated *in vacuo.* The crude product was purified by flash column chromatography on the Biotage Isolera Four^{™} (40 g silica column Puriflash HC 15 µm, 5 -80% EtOAc in cyclohexane) to give the title compound (60 mg, 21%). LCMS (Method 3) 4.27 min, 588.1 [M+H]⁺. ¹H NMR (400 MHz, DMSO-d₆) δ 14.78 (bs, 1H), 11.92 (bs, 1H), 7.52 (d, 1H), 5.79 (dd,1H), 5.28 (dd, 1H), 5.18 (dd, 1H), 4.03-3.90 (m, 2H), 3.58 (m, 1H), 3.18 (d, 1H), 3.01 (m, 1H), 2.90 (dd, 1H), 2.80 (dd, 1H), 2.67 (m, 1H), 2.55 (d, 1H), 2.36-2.23 (m, 4H), 2.19 (d, 1H), 2.09 (d, 1H), 1.72-1.47 (m, 3H), 1.46-1.15 (m, 4H), 1.11 (s, 3H), 0.63-0.42 (m, 4H).

### Biological Assays

### KEAP1 Kelch fluorescence polarization (FP) assay method

Inhibition of the Kelch domain-NRF2 interaction was determined using a fluorescence polarization-based competition assay in a black 384-well microplate. Compounds were tested at a starting concentration of 10 µM serially diluted 1:3 to generate a 12-point dose response curve on the Biomek FX robot. Each well contained 2 nM FITC-labelled NRF2 peptide (FITC-LDEETGEFL-NH2) and 25nM human KEAP1 (N-term, residues 321-609) enzyme in a final volume of 20 µL of assay buffer (50 mM Tris-HCl pH 8.0, 100 mM NaCl, 5 mM MgCl₂, 0.005% Tween-20, 0.005% BSA, 0.5% DMSO) in the presence of varying concentrations of test compound. Unlabelled peptide (LDEETGEFL-NH2) at 50 µM (negative control) and 0.5% DMSO (positive control) was used to determine the assay window.

After 1 h at room temperature, fluorescence polarization (excitation 470 nm/emission 530 nm) was measured using an Envision plate reader. IC₅₀ values were determined by fitting the data to a four parameter logistic fit using XLfit or XE Runner within ActivityBase. The assay limit is such that compounds below 10 nM cannot be differentiated. IC₅₀ values for the Example compounds are shown in Table 1 (NT = not tested).

**Table 1**

| **Example No.** | **IC₅₀ (nM)** |
|---|---|
| **1** | 4.8 |
| **2** | 5.4 |
| **3** | 4.4 |
| **4** | 5.0 |
| **5** | 4.9 |
| **6** | 5.0 |
| 7 | NT |

### Beas2B NQO1 mRNA cell based assay

The up regulation of the NRF2 mediated gene NAD(P)H:quinone acceptor oxidoreductase 1 (NQO1) was measured using the following assay method: BEAS-2B cells (ATCC CRL-9609) were plated in 96-well clear plates at 20,000 cells/well in 75µL of cell culture media and incubated overnight (37 °C, 5% CO₂). On day 2, 25µL of compound or controls were added to the cells for 24 h. On day 3, the medium was aspirated from the plate and the Cells-to-CT^{™} 1-Step TaqMan^{®} Kit (Ambion A25603) was used to perform expression analysis directly from cultured cells without RNA purification according to the manufacturer's instructions.

Briefly, cells were washed with ice cold PBS and 22.5 µL of roomtemperature DNase/Lysis solution was added to the cells and incubated at room temperature for 5 minutes. To stop the reaction, 2.25 µL of stop solution was added to the cell lysate. The samples were diluted 1:5 using nuclease free water and 2.5 µL transferred into the PCR plate. Real-time PCR was performed using the C-1000 Thermal Cycler (Bio-Rad) using human beta actin as the internal control. The cDNA was amplified with a specific primer for NQO1 using the 1-step RT-PCR master mix (Ambion Cells-to-CT^{™} 1-Step TaqMan^{®} Kit A25603). The primers/probes sets that were used for amplification of cDNA were obtained from TaqMan Gene Expression Assays (Applied Biosystems). The comparative CT (ΔΔCT) relative quantification method was used to calculate the relative mRNA level of the target gene NQO1 as described in the Applied Biosystems Chemistry Guide. The data are expressed as an increase in target gene mRNA compared to vehicle (0.1% DMSO) control and the EC₅₀ values were determined by fitting the data to a four parameter logistic fit using XLfit or XE Runner within ActivityBase. EC₅₀ values for the Example compounds are shown in Table 2 (NT = not tested).

**Table 2**

| **Example No.** | **EC₅₀ (nM)** |
|---|---|
| **1** | 6.8 |
| **2** | 1.3 |
| **3** | 2.1 |
| **4** | 0.3 |
| **5** | 0.5 |
| **6** | 1.0 |
| **7** | NT |

### PK/PD Method

Male Wistar Han rats (Charles River labs) were administered the test item orally or intravenously at the designated dose concentration. The intravenous dose was administered as a slow bolus *via* the tail vein. The oral formulation was administered by gastric gavage into the stomach. Actual dose times were recorded.

At the designated time points, 2 x 0.25 mL blood samples were collected into K₂EDTA blood tubes via the tail vein. The collected blood samples were then either centrifuged for plasma or decanted into the 1.5 mL PCR RNAlater tubes containing 650 µL of RNAlater.

Immediately following collection, blood samples were placed on wet ice. As soon as practically possible the 0.25 mL blood samples in K₂EDTA were centrifuged (+4°C, 1500 g, 10 min) and the resulting plasma stored in appropriately labelled polypropylene tubes in a freezer set to maintain a temperature of -80°C until determination of plasma pharmacokinetics. The additional 0.25 mL blood samples in the 1.5 mL PCR RNAlater tubes containing 650 µL of RNAlater were stored in a refrigerator at 4°C until determination of blood pharmacodynamics.

Following the last sample collection each animal was sacrificed by anesthetic overdose by IP injection of Pentobarbitone Na as soon as practically possible and death was confirmed by cervical dislocation.

The lungs from each animal were removed and divided into 4 equal pieces immediately after extraction. The first two sections of lung (labelled left and right) were placed in 5 mL RNAlater tissue protect tube containing 5 mL RNAlater stabilisation reagent and stored at 4°C to allow RNA stabilization (PD analysis). The remaining two sections (labelled left and right) were weighed and snap frozen by immersion in liquid nitrogen in polypropylene tubes (PK analysis).

The liver was also collected from each animal. Six representative pieces (of a similar size to the lung pieces) from different areas (no more than 0.5 cm thickness) were collected. Four pieces (no more than 0.5 cm thickness) were placed in two separate (two pieces per tube) 5 mL RNAlater tissue protect tubes containing 5 mL RNAlater stabilisation reagent (tissue sections were completely submerged into the RNA later solution) and stored at 4°C (PD analysis). The remaining two pieces were weighed and snap frozen by immersion in liquid nitrogen in separate polypropylene tubes (a maximum of 0.5 g per tube, PK analysis).

In some studies, the heart, spleen and brain were also collected from each animal. These tissues were sectioned into four equal sized pieces and two pieces placed in a single 5 mL RNA later tissue protect tube containing 5 mL RNAlater stabilisation reagent and stored at 4°C. The remaining two pieces were weighed and placed individually into polypropylene tubes and snap frozen by immersion in liquid nitrogen.

Study sample tubes containing RNAlater RNA Stabilisation Reagent were stored at ca +4 °C to allow RNA stabilisation reagent to perfuse the tissue. Sections snap frozen were stored in a freezer set to maintain a temperature of -80°C.

PK study samples were quantified using a method based on protein precipitation and LC-MS/MS analysis. Prior to analysis defrosted tissue samples were weighed and homogenised following the addition of HPLC grade water using an Omni-Prep Bead Ruptor (Omni Inc., Kennesaw, GA) at 4°C. Plasma and tissue homogenate samples were extracted using protein precipitation with acetonitrile acidified with 0.1% formic acid containing internal standard(s). Samples were mixed and centrifuged at 4000 rpm at 4°C for 30 minutes to remove precipitated proteins, and the supernatant diluted appropriately with HPLC grade water in a 96-well plate. Representative aliquots of plasma and tissue homogenates were assayed for test item by LC-MS/MS using a Waters Xevo TQ-S (Waters, Elstree, UK) against matrix matched calibration curves and quality control standards. The standards were prepared by spiking aliquots of control plasma and tissue homogenate with the test item and extracted as described for the experimental samples.

RNA extraction and real-time PCR analysis for NQO1 gene expression in in-vivo PD studies was performed as detailed below.

Total RNA was isolated using RNeasy plus mini RNA isolation kit (Qiagen) or Mouse RiboPure^{™}-Blood RNA Isolation Kit (ThermoFisher Scientific) according to the manufacturer's instructions and quantified using an Agilent RNA 6000 Nano instrument. Real-time PCR was performed using the C-1000 Thermal Cycler (Bio-Rad) using rat beta actin as the internal control. The cDNA was amplified with specific primer for NQO1/Nqo1 using universal master mix (Applied Biosystems). The primers/probes sets used for amplification of cDNA were obtained from TaqMan Gene Expression Assays (Applied Biosystems). The comparative CT (ΔΔCT) relative quantification method is used to calculate the relative mRNA level of the target gene NQO1 as described in the Applied Biosystems Chemistry Guide. The data is expressed as an increase in target gene mRNA compared to vehicle control treated for each tissue type.

## Claims

1. A compound which is selected from:
5-(((S)-5-chloro-8-((5-(difluoromethyl)-1-methyl-1H-1,2,3-triazol-4-yl)methoxy)-7-fluoro-2-((1R,2S)-2-methyl-2-(1H-tetrazol-5-yl)cyclohexane-1-carbonyl)-1,2,3,4-tetrahydroisoquinolin-1-yl)methyl)-5-azaspiro[2.4]heptan-6-one;
(1S,2R)-2-((S)-5-chloro-8-((5-(difluoromethyl)-1-methyl-1H-1,2,3-triazol-4-yl)methoxy)-7-fluoro-1-((3-oxomorpholino)methyl)-1,2,3,4-tetrahydroisoquinoline-2-carbonyl)-1-methylcyclohexane-1-carboxylic acid;
(1S,2R)-2-((S)-5-chloro-8-((5-(difluoromethyl)-1-methyl-1H-1,2,3-triazol-4-yl)methoxy)-7-fluoro-1-(((R)-4-methyl-2-oxopyrrolidin-1-yl)methyl)-1,2,3,4-tetrahydroisoquinoline-2-carbonyl)-1-methylcyclohexane-1-carboxylic acid;
(1S,2R)-2-((S)-5-chloro-7-fluoro-1-((6-oxo-5-azaspiro[2.4]heptan-5-yl)methyl)-8-((4,5,6,7-tetrahydro-[1,2,3]triazolo[1,5-a]pyridin-3-yl)methoxy)-1,2,3,4-tetrahydroisoquinoline-2-carbonyl)-1-methylcyclopentane-1-carboxylic acid;
(1S,2R)-2-((S)-5-chloro-8-((4,5-dimethyl-4H-1,2,4-triazol-3-yl)methoxy)-7-fluoro-1-((6-oxo-5-azaspiro[2.4]heptan-5-yl)methyl)-1,2,3,4-tetrahydroisoquinoline-2-carbonyl)-1-methylcyclohexane-1-carboxylic acid; and
(1S,2R)-2-((S)-5-chloro-7-fluoro-8-((1-methyl-1H-1,2,3-triazol-4-yl)methoxy)-1-((6-oxo-5-azaspiro[2.4]heptan-5-yl)methyl)-1,2,3,4-tetrahydroisoquinoline-2-carbonyl)-1-methylcyclohexane-1-carboxylic acid;
or a pharmaceutically acceptable salt thereof.

2. A compound according to claim 1, which is: 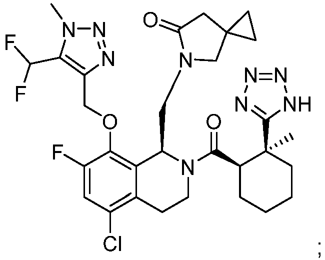 or a pharmaceutically acceptable salt thereof.

3. A compound according to claim 1, which is: 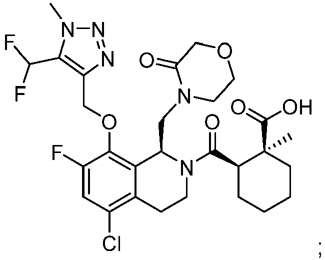 or a pharmaceutically acceptable salt thereof.

4. A compound according to claim 1, which is: 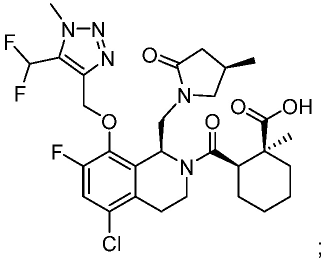 or a pharmaceutically acceptable salt thereof.

5. A compound according to claim 1, which is: 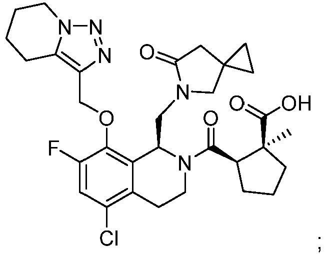 or a pharmaceutically acceptable salt thereof.

6. A compound according to claim 1, which is: 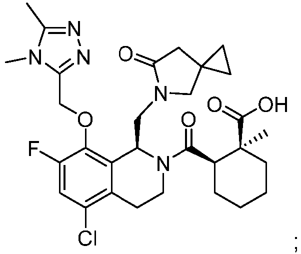 or a pharmaceutically acceptable salt thereof.

7. A compound according to claim 1, which is: 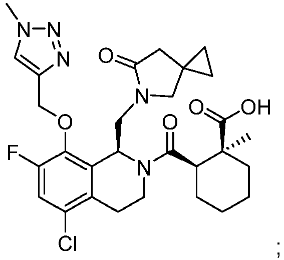 or a pharmaceutically acceptable salt thereof.

8. A pharmaceutical composition comprising a compound according to any one of claims 1 to 7, or a pharmaceutically acceptable salt thereof, and one or more pharmaceutically acceptable excipients.

9. A compound according to any one of claims 1 to 7, or a pharmaceutically acceptable salt thereof, or a pharmaceutical composition according to claim 8, for use in therapy.

10. A compound according to any one of claims 1 to 7, or a pharmaceutically acceptable salt thereof, or a pharmaceutical composition according to claim 8, for use in the treatment of diseases or disorders mediated by Nrf2 activation.

11. A compound according to any one of claims 1 to 7, or a pharmaceutically acceptable salt thereof, or a pharmaceutical composition according to claim 8, for use in the treatment of chronic obstructive pulmonary disease, acute, chronic and severe asthma, acute lung injury/acute respiratory distress syndrome with or without accompanying multi organ dysfunction syndrome, pulmonary fibrosis including idiopathic pulmonary fibrosis, cystic fibrosis, COVID-19, diabetes, atherosclerosis, hypertension, heart failure, myocardial infarction and repair, cardiac remodelling, cardiac arrhythmias, cardiac hypertrophy, heart failure with preserved ejection fraction, diabetic cardiomyopathy, sarcopenia, obesity, metabolic syndrome, diabetes mellitus, insulin resistance, pulmonary arterial hypertension, subarachnoid haemorrhage, intracerebral haemorrhage, ischemic stroke, beta-thalassemia, sickle cell disease, rheumatoid arthritis, irritable bowel disorder, ulcerative colitis, Crohn's disease, psoriasis, radiation-induced dermatitis, atopic dermatitis, non-alcoholic fatty liver disease, non-alcoholic steatohepatitis, toxin-induced liver disease, viral hepatitis and cirrhosis, chronic kidney disease, diabetic nephropathy, autosomal dominant polycystic kidney disease, CKD associated with type 1 diabetes (T1D), IgA nephropathy (IgAN), Alport Syndrome, focal segmental glomerulosclerosis, Huntington's disease, Parkinson's disease, Alzheimer's disease, amyotrophic lateral sclerosis, frontotemporal dementia, multiple sclerosis, Friedreich's ataxia, chronic pain, schizophrenia, lung cancer, breast cancer, colon cancer, age related macular degeneration (AMD), Fuchs Endothelial Corneal Dystrophy, or uveitis.

## Patentansprüche

1. Verbindung, die ausgewählt ist aus:
5-(((S)-5-Chlor-8-((5-(difluormethyl)-1-methyl-1H-1,2,3-triazol-4-yl)methoxy)-7-fluor-2-((1R,2S)-2-methyl-2-(1H-tetrazol-5-yl)cyclohexan-1-carbonyl)-1,2,3,4-tetrahydroisochinolin-1-yl)methyl)-5-azaspiro[2.4]heptan-6-on;
(1S,2R)-2-((S)-5-Chlor-8-((5-(difluormethyl)-1-methyl-1H-1,2,3-triazol-4-yl)methoxy)-7-fluor-1-((3-oxomorpholino)methyl)-1,2,3,4-tetrahydroisochinolin-2-carbonyl)-1-methylcyclohexan-1-carbonsäure;
(1S,2R)-2-((S)-5-Chlor-8-((5-(difluormethyl)-1-methyl-1H-1,2,3-triazol-4-yl)methoxy)-7-fluor-1-(((R)-4-methyl-2-oxopyrrolidin-1-yl)methyl)-1,2,3,4-tetrahydroisochinolin-2-carbonyl)-1-methylcyclohexan-1-carbonsäure;
(1 S,2R)-2-((S)-5-Chlor-7-fluor-1-((6-oxo-5-azaspiro[2.4]heptan-5-yl)methyl)-8-((4,5,6,7-tetrahydro-[1,2,3]triazolo[1,5-a]pyridin-3-yl)methoxy)-1,2,3,4-tetrahydroisochinolin-2-carbonyl)-1-methylcyclopentan-1-carbonsäure;
(1S,2R)-2-((S)-5-Chlor-8-((4,5-dimethyl-4H-1,2,4-triazol-3-yl)methoxy)-7-fluor-1-((6-oxo-5-azaspiro[2.4]heptan-5-yl)methyl)-1,2,3,4-tetrahydroisochinolin-2-carbonyl)-1-methylcyclohexan-1-carbonsäure; und
(1S,2R)-2-((S)-5-Chlor-7-fluor-8-((1-methyl-1H-1,2,3-triazol-4-yl)methoxy)-1-((6-oxo-5-azaspiro[2.4]heptan-5-yl)methyl)-1,2,3,4-tetrahydroisochinolin-2-carbonyl)-1-methylcyclohexan-1-carbonsäure;
oder einem pharmazeutisch unbedenklichen Salz davon.

2. Verbindung nach Anspruch 1, die wie folgt ist: 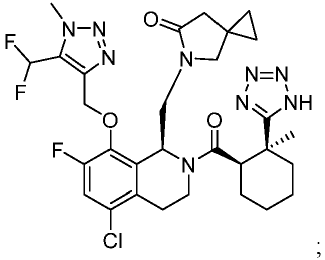 oder ein pharmazeutisch unbedenkliches Salz davon.

3. Verbindung nach Anspruch 1, die wie folgt ist: 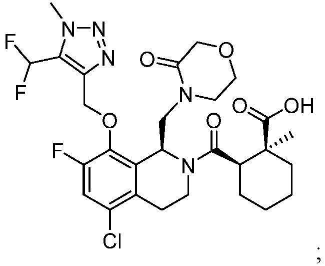 oder ein pharmazeutisch unbedenkliches Salz davon.

4. Verbindung nach Anspruch 1, die wie folgt ist: 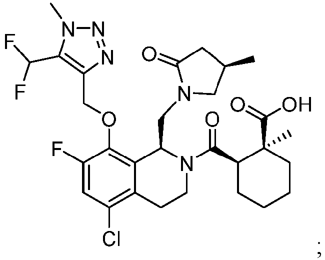 oder ein pharmazeutisch unbedenkliches Salz davon.

5. Verbindung nach Anspruch 1, die wie folgt ist: 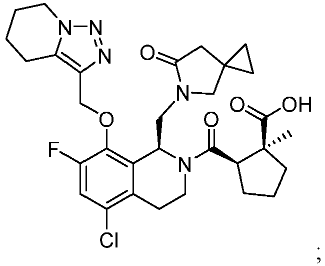 oder ein pharmazeutisch unbedenkliches Salz davon.

6. Verbindung nach Anspruch 1, die wie folgt ist: 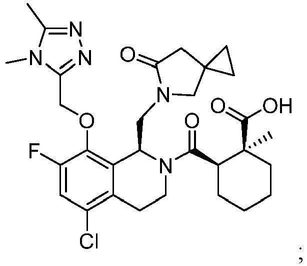 oder ein pharmazeutisch unbedenkliches Salz davon.

7. Verbindung nach Anspruch 1, die wie folgt ist: 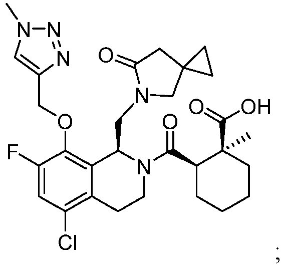 oder ein pharmazeutisch unbedenkliches Salz davon.

8. Pharmazeutische Zusammensetzung, umfassend eine Verbindung nach einem der Ansprüche 1 bis 7 oder ein pharmazeutisch unbedenkliches Salz davon und einen oder mehrere pharmazeutisch unbedenkliche Hilfsstoffe.

9. Verbindung nach einem der Ansprüche 1 bis 7 oder pharmazeutisch unbedenkliches Salz davon, oder pharmazeutische Zusammensetzung nach Anspruch 8 zur Verwendung in Therapie.

10. Verbindung nach einem der Ansprüche 1 bis 7 oder pharmazeutisch unbedenkliches Salz davon, oder pharmazeutische Zusammensetzung nach Anspruch 8 zur Verwendung bei der Behandlung von Erkrankungen oder Störungen vermittelt durch Nrf2-Aktivierung.

11. Verbindung nach einem der Ansprüche 1 bis 7 oder pharmazeutisch unbedenkliches Salz davon oder pharmazeutische Zusammensetzung nach Anspruch 8 zur Verwendung bei der Behandlung von chronisch obstruktiver pulmonaler Erkrankung, akutem, chronischem und schwerem Asthma, akuter Lungenverletzung/akutem Atemnotsyndrom mit oder ohne begleitendem Multiorganfehlfunktionssyndrom, pulmonaler Fibrose beinhaltend idiopathische pulmonale Fibrose, zystische Fibrose, COVID-19, Diabetes, Atherosklerose, Bluthochdruck, Herzinsuffizienz, Myokardinfarkt und -reparatur, kardialem Remodelling, Herzrhythmusstörungen, Herzhypertrophie, Herzinsuffizienz mit konservierter Ejektionsfraktion, diabetischer Kardiomyopathie, Sarkopenie, Adipositas, metabolischem Syndrom, Diabetes mellitus, Insulinresistenz, pulmonaler arterieller Hypertonie, Subarachnoidalblutung, intrazerebraler Blutung, ischämischem Schlaganfall, Betathalassämie, Sichelzellerkrankung, rheumatoider Arthritis, Reizdarmstörung, Colitis ulcerosa, Crohn-Erkrankung, Psoriasis, strahleninduzierter Dermatitis, atopischer Dermatitis, nichtalkoholischer Fettlebererkrankung, nichtalkoholischer Steatohepatitis, toxininduzierter Lebererkrankung, Virushepatitis und - zirrhose, chronischer Nierenerkrankung, diabetischer Nephropathie, autosomal-dominanter polyzystischer Nierenerkrankung, CKD assoziiert mit Typ-1-Diabetes (T1D), IgA-Nephropathie (IgAN), Alport-Syndrom, fokaler segmentaler Glomerulosklerose, Huntington-Erkrankung, Parkinson-Erkrankung, Alzheimer-Erkrankung, amyotropher Lateralsklerose, frontotemporaler Demenz, multipler Sklerose, Friedreich-Ataxie, chronischen Schmerzen, Schizophrenie, Lungenkrebs, Brustkrebs, Darmkrebs, altersbedingter Makuladegeneration (AMD), Fuchs-Endothelhornhautdystrophie oder Uveitis.

## Revendications

1. Composé qui est choisi parmi :
la 5-(((S)-5-chloro-8-((5-(difluorométhyl)-1-méthyl-1H-1,2,3-triazol-4-yl)méthoxy)-7-fluoro-2-((1R,2S)-2-méthyl-2-(1H-tétrazol-5-yl)cyclohexane-1-carbonyl)-1,2,3,4-tétrahydroisoquinoléin-1-yl)méthyl)-5-azaspiro[2.4]heptan-6-one ;
l'acide (1S,2R)-2-((S)-5-chloro-8-((5-(difluorométhyl)-1-méthyl-1H-1,2,3-triazol-4-yl)méthoxy)-7-fluoro-1-((3-oxomorpholino)méthyl)-1,2,3,4-tétrahydroisoquinoléine-2-carbonyl)-1-méthylcyclohexane-1-carboxylique ;
l'acide (1S,2R)-2-((S)-5-chloro-8-((5-(difluorométhyl)-1-méthyl-1H-1,2,3-triazol-4-yl)méthoxy)-7-fluoro-1-(((R)-4-méthyl-2-oxopyrrolidin-1-yl)méthyl)-1,2,3,4-tétrahydroisoquinoléine-2-carbonyl)-1-méthylcyclohexane-1-carboxylique ;
l'acide (1S,2R)-2-((S)-5-chloro-7-fluoro-1-((6-oxo-5-azaspiro[2.4]heptan-5-yl)méthyl)-8-((4,5,6,7-tétrahydro-[1,2,3]triazolo[1,5-a]pyridin-3-yl)méthoxy)-1,2,3,4-tétrahydroisoquinoléine-2-carbonyl)-1-méthylcyclopentane-1-carboxylique ;
l'acide (1S,2R)-2-((S)-5-chloro-8-((4,5-diméthyl-4H-1,2,4-triazol-3-yl)méthoxy)-7-fluoro-1-((6-oxo-5-azaspiro[2.4]heptan-5-yl)méthyl)-1,2,3,4-tétrahydroisoquinoléine-2-carbonyl)-1-méthylcyclohexane-1-carboxylique ; et
l'acide (1S,2R)-2-((S)-5-chloro-7-fluoro-8-((1-méthyl-1H-1,2,3-triazol-4-yl)méthoxy)-1-((6-oxo-5-azaspiro[2.4]heptan-5-yl)méthyl)-1,2,3,4-tétrahydroisoquinoléine-2-carbonyl)-1-méthylcyclohexane-1-carboxylique ;
ou un sel pharmaceutiquement acceptable de celui-ci.

2. Composé selon la revendication 1, qui est : 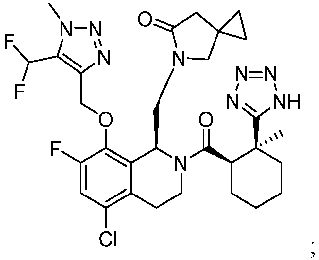 ou un sel pharmaceutiquement acceptable de celui-ci.

3. Composé selon la revendication 1, qui est : 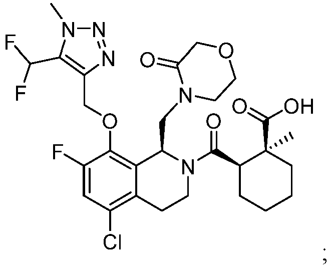 ou un sel pharmaceutiquement acceptable de celui-ci.

4. Composé selon la revendication 1, qui est : 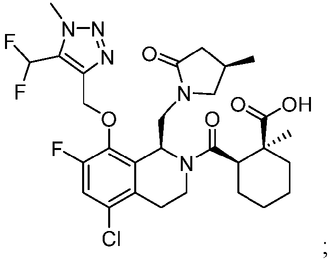 ou un sel pharmaceutiquement acceptable de celui-ci.

5. Composé selon la revendication 1, qui est : 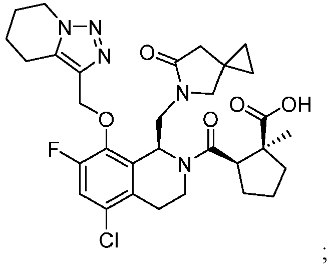 ou un sel pharmaceutiquement acceptable de celui-ci.

6. Composé selon la revendication 1, qui est : 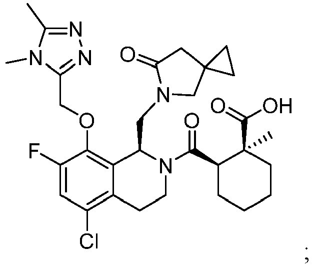 ou un sel pharmaceutiquement acceptable de celui-ci.

7. Composé selon la revendication 1, qui est : 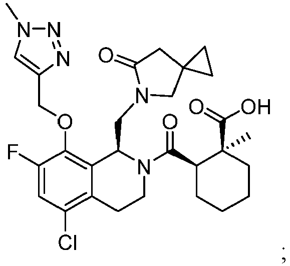 ou un sel pharmaceutiquement acceptable de celui-ci.

8. Composition pharmaceutique comprenant un composé selon l'une quelconque des revendications 1 à 7, ou un sel pharmaceutiquement acceptable de celui-ci et un ou plusieurs excipients pharmaceutiquement acceptables.

9. Composé selon l'une quelconque des revendications 1 à 7, ou sel pharmaceutiquement acceptable de celui-ci ou composition pharmaceutique selon la revendication 8, destiné(e) à être utilisé(e) en thérapie.

10. Composé selon l'une quelconque des revendications 1 à 7, ou sel pharmaceutiquement acceptable de celui-ci, ou composition pharmaceutique selon la revendication 8, destiné(e) à être utilisé(e) dans le traitement de maladies ou de troubles médiés par l'activation de Nrf2.

11. Composé selon l'une quelconque des revendications 1 à 7, ou sel pharmaceutiquement acceptable de celui-ci, ou composition pharmaceutique selon la revendication 8, destiné(e) à être utilisé(e) dans le traitement de la bronchopneumopathie chronique obstructive, de l'asthme aigu, chronique et sévère, de la lésion pulmonaire aiguë/du syndrome de détresse respiratoire aiguë avec ou sans syndrome de défaillance multiviscéral, de la fibrose pulmonaire, y compris la fibrose pulmonaire idiopathique, de la mucoviscidose, le COVID-19, du diabète, de l'athérosclérose, de l'hypertension, de l'insuffisance cardiaque, de l'infarctus et de la réparation du myocarde, du remodelage cardiaque, des arythmies cardiaques, de l'hypertrophie cardiaque, de l'insuffisance cardiaque avec fraction d'éjection préservée, de la cardiomyopathie diabétique, de la sarcopénie, de l'obésité, du syndrome métabolique, du diabète sucré, de la résistance à l'insuline, de l'hypertension artérielle pulmonaire, de l'hémorragie sous-arachnoïdienne, de l'hémorragie intracérébrale, de l'accident vasculaire cérébral ischémique, de la bêta-thalassémie, de la drépanocytose, de la polyarthrite rhumatoïde, du trouble du côlon irritable, de la rectocolite hémorragique, de la maladie de Crohn, du psoriasis, de la dermatite radio-induite, de la dermatite atopique, de la stéatose hépatique non-alcoolique, de la stéatohépatite non alcoolique, de la maladie hépatique induite par les toxines, de l'hépatite virale et de la cirrhose, de la maladie rénale chronique, de la néphropathie diabétique, de la polykystose rénale autosomique dominante, de la MRC associée au diabète de type 1 (DT1), de la néphropathie IgA (IgAN), du syndrome d'Alport, de la glomérulosclérose segmentaire focale, de la maladie de Huntington, de la maladie de Parkinson, de la maladie d'Alzheimer, de la sclérose latérale amyotrophique, de la démence frontotemporale, de la sclérose en plaques, de l'ataxie de Friedreich, de la douleur chronique, de la schizophrénie, du cancer du poumon, du cancer du sein, du cancer du côlon, de la dégénérescence maculaire liée à l'âge (DMLA), de la dystrophie cornéenne endothéliale de Fuchs ou de l'uvéite.
